# EUROPEAN PATENT APPLICATION

(11) **EP 2 133 360 A2**
(43) Date of publication of application: **16.12.2009**
(21) Application number: 09011943.9
(22) Date of filing: 14.11.2000
(51) Int. Cl.: C07K 14/415, C12N 15/82, A01H 5/00

(54) **Environmental stress tolerance genes**

(30) Priority: 17.11.1999 US 166228 P; 17.04.2000 US 197899 P; 22.08.2000 US 227439 P
(62) Divisional of application: 08006255.7
(71) Applicant: Mendel Biotechnology, Inc., Hayward, CA 94545-3720 (US)
(72) Inventor: Pineda, Omaira, Vero Beach, FL 32960 (US); Yu, Guo-Liang, Berkeley, CA 94705 (US); Creelman, Robert, Castro Valley, CA 94546 (US); Riechmann, Jose Luis, Pasadena, CA 91101 (US); Heard, Jacqueline, Stonington, CT 06378-1722 (US); Ratcliffe, Oliver, Oakland, CA 94606 (US); Reuber, Lynne, San Mateo, CA 94402 (US); Keddie, James, San Mateo, CA 94402 (US)
(74) Representative: Brasnett, Adrian Hugh

(57) **Abstract**

Recombinant polynucleotides and methods for modifying the phenotype of a plant are provided, and plants having such modified phenotypes. In particular, the phenotype that is being modified is tolerance to heat.

SEQUENCE LISTING

<110>  Mendel Biotechnology, Inc.

<120>  Environmental Stress Tolerance Genes

<130>  AHB/FP6652176

<140>
<141>  2000-11-14

<150>  08006255.7
<151>  2000-11-14

<150>  00980418.8
<151>  2000-11-14

<150>  60/166,228
<151>  1999-11-17

<150>  60/197,899
<151>  2000-04-17

<150>  60/227,439
<151>  2000-08-22

<160>  54

<170>  PatentIn version 3.0

<210>  1

<211>  913

<212>  DNA

<213>  Arabidopsis thaliana

<220>

<221>  CDS

<222>  (81)..(761)

<223>  G22

<400>  1
agaaaacatc tctcactctc taaaatacac actctcatca aaaaccttct cttcggttca      60

gaagcattca agaatccatt atg agc tca tct gat tcc gtt aat aac ggc gtt     113
                      Met Ser Ser Ser Asp Ser Val Asn Asn Gly Val
                      1               5                   10

aac tca cgg atg tac ttc cgt aac ccg agt ttc agc aac gtt atc tta       161
Asn Ser Arg Met Tyr Phe Arg Asn Pro Ser Phe Ser Asn Val Ile Leu
            15                  20                  25

aac gat aac tgg agc gac ttg ccg tta agt gtc gac gat tct caa gac       209
Asn Asp Asn Trp Ser Asp Leu Pro Leu Ser Val Asp Asp Ser Gln Asp

        30                  35                  40

atg gct att tac aac act ctc cgt gat gcc gtt agc tcc ggc tgg aca 257
Met Ala Ile Tyr Asn Thr Leu Arg Asp Ala Val Ser Ser Gly Trp Thr
    45                  50                  55

ccc tcc gtt cct ccc gtt acc tct ccg gcg gag gaa aat aag cct ccg 305
Pro Ser Val Pro Pro Val Thr Ser Pro Ala Glu Glu Asn Lys Pro Pro
60                  65                  70                  75

gcg acg aag gcg agt ggc tca cac gcg ccg agg cag aag ggg atg cag 353
Ala Thr Lys Ala Ser Gly Ser His Ala Pro Arg Gln Lys Gly Met Gln
                80                  85                  90

tac aga gga gtg agg agg agg ccg tgg ggg aaa ttc gcg gcg gag att 401
Tyr Arg Gly Val Arg Arg Arg Pro Trp Gly Lys Phe Ala Ala Glu Ile
            95                  100                 105

agg gat ccg aag aag aac gga gct agg gtt tgg ctc ggg act tac gag 449
Arg Asp Pro Lys Lys Asn Gly Ala Arg Val Trp Leu Gly Thr Tyr Glu
        110                 115                 120

acg ccg gag gac gcg gcg gtg gcg tac gac cga gcg gcg ttt cag ctc 497
Thr Pro Glu Asp Ala Ala Val Ala Tyr Asp Arg Ala Ala Phe Gln Leu
    125                 130                 135

aga gga tcg aaa gct aag ctg aat ttt ccg cat ttg att ggt tct tgt 545
Arg Gly Ser Lys Ala Lys Leu Asn Phe Pro His Leu Ile Gly Ser Cys
140                 145                 150                 155

aag tat gag ccg gtt agg att agg cct cgc cgt cgc tcg ccg gaa ccg 593
Lys Tyr Glu Pro Val Arg Ile Arg Pro Arg Arg Arg Ser Pro Glu Pro
                160                 165                 170

tca gtc tcc gat cag tta acg tcg gag cag aag agg gaa agc cac gtg 641
Ser Val Ser Asp Gln Leu Thr Ser Glu Gln Lys Arg Glu Ser His Val
            175                 180                 185

gat gac ggc gag tct agt ttg gtt gta ccg gag ttg gat ttc acg gtg 689
Asp Asp Gly Glu Ser Ser Leu Val Val Pro Glu Leu Asp Phe Thr Val
        190                 195                 200

gat cag ttt tac ttc gat ggt agt tta tta atg gac caa tca gaa tgt 737
Asp Gln Phe Tyr Phe Asp Gly Ser Leu Leu Met Asp Gln Ser Glu Cys
    205                 210                 215

tct tat tct gat aat cgg ata taa ttagttttaa gattaagcaa aatttgtcca 791
Ser Tyr Ser Asp Asn Arg Ile
220                 225

acgagttttg ctgtatgaaa tatctatcga tgactcaaca ggttttgatc atgatcatat 851

gtaatgtgat ggaaattaaa tattgacgtt tgtttttttg ttgtaaaaaa aaaaaaaaaa 911

aa 913

<210> 2

<211> 226

<212> PRT

<213> Arabidopsis thaliana

<400> 2

Met Ser Ser Ser Asp Ser Val Asn Asn Gly Val Asn Ser Arg Met Tyr
1               5                   10                  15

Phe Arg Asn Pro Ser Phe Ser Asn Val Ile Leu Asn Asp Asn Trp Ser
            20                  25                  30

Asp Leu Pro Leu Ser Val Asp Asp Ser Gln Asp Met Ala Ile Tyr Asn
        35                  40                  45

Thr Leu Arg Asp Ala Val Ser Ser Gly Trp Thr Pro Ser Val Pro Pro
    50                  55                  60

Val Thr Ser Pro Ala Glu Glu Asn Lys Pro Pro Ala Thr Lys Ala Ser
65                  70                  75                  80

Gly Ser His Ala Pro Arg Gln Lys Gly Met Gln Tyr Arg Gly Val Arg
                85                  90                  95

Arg Arg Pro Trp Gly Lys Phe Ala Ala Glu Ile Arg Asp Pro Lys Lys
            100                 105                 110

Asn Gly Ala Arg Val Trp Leu Gly Thr Tyr Glu Thr Pro Glu Asp Ala
        115                 120                 125

Ala Val Ala Tyr Asp Arg Ala Ala Phe Gln Leu Arg Gly Ser Lys Ala
    130                 135                 140

Lys Leu Asn Phe Pro His Leu Ile Gly Ser Cys Lys Tyr Glu Pro Val
145                 150                 155                 160

Arg Ile Arg Pro Arg Arg Arg Ser Pro Glu Pro Ser Val Ser Asp Gln
                165                 170                 175

Leu Thr Ser Glu Gln Lys Arg Glu Ser His Val Asp Asp Gly Glu Ser
            180                 185                 190

Ser Leu Val Val Pro Glu Leu Asp Phe Thr Val Asp Gln Phe Tyr Phe
        195                 200                 205

Asp Gly Ser Leu Leu Met Asp Gln Ser Glu Cys Ser Tyr Ser Asp Asn
    210                 215                 220

Arg Ile
225

<210>  3

<211>  1195

<212>  DNA

<213>  Arabidopsis thaliana

<220>

<221> CDS

<222> (50)..(1096)

<223> G188

<400> 3
ctctcaccaa cataatcaaa gaagctttcc tcacgaattc aagatcgcc atg tcc tcc 58
Met Ser Ser
1

gag gat tgg gat ctc ttc gcc gtc gtc aga agc tgc agc tct tct gtt 106
Glu Asp Trp Asp Leu Phe Ala Val Val Arg Ser Cys Ser Ser Ser Val
    5                   10                  15

tcc acc acc aat tct tgt gct ggt cat gaa gac gac ata gga aac tgt 154
Ser Thr Thr Asn Ser Cys Ala Gly His Glu Asp Asp Ile Gly Asn Cys
20                  25                  30                  35

aaa caa caa caa gat cct cct cct cct cct ctg ttt caa gct tct tct 202
Lys Gln Gln Gln Asp Pro Pro Pro Pro Pro Leu Phe Gln Ala Ser Ser
                40                  45                  50

tct tgc aac gag tta caa gat tct tgc aaa cca ttt tta ccc gtt act 250
Ser Cys Asn Glu Leu Gln Asp Ser Cys Lys Pro Phe Leu Pro Val Thr
            55                  60                  65

act act act act act act tgg tct cct cct cct cta ctt cct cct cct 298
Thr Thr Thr Thr Thr Thr Trp Ser Pro Pro Pro Leu Leu Pro Pro Pro
        70                  75                  80

aaa gcc tca tca cca tct ccc aat atc tta cta aaa caa gaa caa gta 346
Lys Ala Ser Ser Pro Ser Pro Asn Ile Leu Leu Lys Gln Glu Gln Val
    85                  90                  95

ctt ctc gaa tca caa gat caa aaa cct cct ctt agt gtt agg gtt ttc 394
Leu Leu Glu Ser Gln Asp Gln Lys Pro Pro Leu Ser Val Arg Val Phe
100                 105                 110                 115

cca cca tcc act tct tct tct gtc ttt gtt ttt aga ggt caa cgc gac 442
Pro Pro Ser Thr Ser Ser Ser Val Phe Val Phe Arg Gly Gln Arg Asp
                120                 125                 130

cag ctt ctt caa caa caa tcc caa cct ccc ctt cga tct aga aaa aga 490
Gln Leu Leu Gln Gln Gln Ser Gln Pro Pro Leu Arg Ser Arg Lys Arg
            135                 140                 145

aag aat cag caa aaa aga acc ata tgt cat gta acg caa gag aat ctt 538
Lys Asn Gln Gln Lys Arg Thr Ile Cys His Val Thr Gln Glu Asn Leu
        150                 155                 160

tct tct gat ttg tgg gct tgg cgt aaa tac ggt caa aaa ccc atc aaa 586
Ser Ser Asp Leu Trp Ala Trp Arg Lys Tyr Gly Gln Lys Pro Ile Lys
    165                 170                 175

ggc tct cct tat cca agg aat tat tac aga tgt agt agc tca aaa gga 634
Gly Ser Pro Tyr Pro Arg Asn Tyr Tyr Arg Cys Ser Ser Ser Lys Gly
180                 185                 190                 195

tgt tta gca cga aaa caa gtt gaa aga agt aat tta gat cct aat atc 682

Cys Leu Ala Arg Lys Gln Val Glu Arg Ser Asn Leu Asp Pro Asn Ile
                200                 205                 210

ttc atc gtt act tac acc gga gaa cac act cat cca cgt cct act cac      730
Phe Ile Val Thr Tyr Thr Gly Glu His Thr His Pro Arg Pro Thr His
            215                 220                 225

cgg aac tct ctc gcc gga agt act cgt aac aaa tct cag ccc gtt aac      778
Arg Asn Ser Leu Ala Gly Ser Thr Arg Asn Lys Ser Gln Pro Val Asn
        230                 235                 240

ccg gtt cct aaa ccg gac aca tct cct tta tcg gat aca gta aaa gaa      826
Pro Val Pro Lys Pro Asp Thr Ser Pro Leu Ser Asp Thr Val Lys Glu
    245                 250                 255

gag att cat ctt tct ccg acg aca ccg ttg aaa gga aac gat gac gtt      874
Glu Ile His Leu Ser Pro Thr Thr Pro Leu Lys Gly Asn Asp Asp Val
260                 265                 270                 275

caa gaa acg aat gga gat gaa gat atg gtt ggt caa gaa gtc aac atg      922
Gln Glu Thr Asn Gly Asp Glu Asp Met Val Gly Gln Glu Val Asn Met
                280                 285                 290

gaa gag gaa gag gag gaa gaa gaa gtg gaa gaa gat gat gaa gaa gaa      970
Glu Glu Glu Glu Glu Glu Glu Glu Val Glu Glu Asp Asp Glu Glu Glu
            295                 300                 305

gaa gat gat gat gac gtg gat gat ctt ttg ata cca aat tta gcg gtg      1018
Glu Asp Asp Asp Asp Val Asp Asp Leu Leu Ile Pro Asn Leu Ala Val
        310                 315                 320

aga gat cga gat gat ttg ttc ttc gct gga agt ttt cca tct tgg tcc      1066
Arg Asp Arg Asp Asp Leu Phe Phe Ala Gly Ser Phe Pro Ser Trp Ser
    325                 330                 335

gcc gga tcc gcc ggt gac ggt ggt gga tga tgaaaacgaa taaaatctca      1116
Ala Gly Ser Ala Gly Asp Gly Gly Gly
340                 345

atttacaatt tacaaaaaga aaaaagtcag tttttaatta ttatttttgt ttgttaaaac      1176

ttgacattta ttgtgttat                                                   1195

<210>  4

<211>  348

<212>  PRT

<213>  Arabidopsis thaliana

<400>  4

Met Ser Ser Glu Asp Trp Asp Leu Phe Ala Val Val Arg Ser Cys Ser
1               5                   10                  15

Ser Ser Val Ser Thr Thr Asn Ser Cys Ala Gly His Glu Asp Asp Ile
            20                  25                  30

Gly Asn Cys Lys Gln Gln Gln Asp Pro Pro Pro Pro Pro Leu Phe Gln
        35                  40                  45

Ala Ser Ser Ser Cys Asn Glu Leu Gln Asp Ser Cys Lys Pro Phe Leu
    50                  55                  60

Pro Val Thr Thr Thr Thr Thr Thr Thr Trp Ser Pro Pro Pro Leu Leu
65                  70                  75                  80

Pro Pro Pro Lys Ala Ser Ser Pro Ser Pro Asn Ile Leu Leu Lys Gln
                85                  90                  95

Glu Gln Val Leu Leu Glu Ser Gln Asp Gln Lys Pro Pro Leu Ser Val
            100                 105                 110

Arg Val Phe Pro Pro Ser Thr Ser Ser Ser Val Phe Val Phe Arg Gly
        115                 120                 125

Gln Arg Asp Gln Leu Leu Gln Gln Gln Ser Gln Pro Pro Leu Arg Ser
    130                 135                 140

Arg Lys Arg Lys Asn Gln Gln Lys Arg Thr Ile Cys His Val Thr Gln
145                 150                 155                 160

Glu Asn Leu Ser Ser Asp Leu Trp Ala Trp Arg Lys Tyr Gly Gln Lys
                165                 170                 175

Pro Ile Lys Gly Ser Pro Tyr Pro Arg Asn Tyr Tyr Arg Cys Ser Ser
            180                 185                 190

Ser Lys Gly Cys Leu Ala Arg Lys Gln Val Glu Arg Ser Asn Leu Asp
        195                 200                 205

Pro Asn Ile Phe Ile Val Thr Tyr Thr Gly Glu His Thr His Pro Arg
    210                 215                 220

Pro Thr His Arg Asn Ser Leu Ala Gly Ser Thr Arg Asn Lys Ser Gln
225                 230                 235                 240

Pro Val Asn Pro Val Pro Lys Pro Asp Thr Ser Pro Leu Ser Asp Thr
                245                 250                 255

Val Lys Glu Glu Ile His Leu Ser Pro Thr Thr Pro Leu Lys Gly Asn
            260                 265                 270

Asp Asp Val Gln Glu Thr Asn Gly Asp Glu Asp Met Val Gly Gln Glu
        275                 280                 285

Val Asn Met Glu Glu Glu Glu Glu Glu Glu Glu Val Glu Glu Asp Asp
    290                 295                 300

Glu Glu Glu Glu Asp Asp Asp Asp Val Asp Asp Leu Leu Ile Pro Asn
305                 310                 315                 320

Leu Ala Val Arg Asp Arg Asp Asp Leu Phe Phe Ala Gly Ser Phe Pro
                325                 330                 335

Ser Trp Ser Ala Gly Ser Ala Gly Asp Gly Gly Gly
            340                 345

<210>  5

<211>  584

<212>  DNA

<213>  Arabidopsis thaliana

<220>

<221>  CDS

<222>  (157)..(441)

<223>  G225

<400>  5
   ctctctctct cactcttttc ttttccgaga acccaacaaa aaaaaagcta ctattaatcc      60
   ttcccctcgt gaggaaatca tttcttcttg tttctcgaga tttattctct ttctctctct     120
   ctttctctgt gtgtttcgtg tcttcagatt agttcg atg ttt cgt tca gac aag       174
                                           Met Phe Arg Ser Asp Lys
                                           1               5
   gcg gaa aaa atg gat aaa cga cga cgg aga cag agc aaa gcc aag gct       222
   Ala Glu Lys Met Asp Lys Arg Arg Arg Arg Gln Ser Lys Ala Lys Ala
               10                  15                  20
   tct tgt tcc gaa gag gtg agt agt atc gaa tgg gaa gct gtg aag atg       270
   Ser Cys Ser Glu Glu Val Ser Ser Ile Glu Trp Glu Ala Val Lys Met
           25                  30                  35
   tca gaa gaa gaa gaa gat ctc att tct cgg atg tat aaa ctc gtt ggc       318
   Ser Glu Glu Glu Glu Asp Leu Ile Ser Arg Met Tyr Lys Leu Val Gly
       40                  45                  50
   gac agg tgg gag ttg atc gcc gga agg atc ccg gga cgg acg ccg gag       366
   Asp Arg Trp Glu Leu Ile Ala Gly Arg Ile Pro Gly Arg Thr Pro Glu
   55                  60                  65                  70
   gag ata gag aga tat tgg ctt atg aaa cac ggc gtc gtt ttt gcc aac       414
   Glu Ile Glu Arg Tyr Trp Leu Met Lys His Gly Val Val Phe Ala Asn
                   75                  80                  85
   aga cga aga gac ttt ttt agg aaa tga ttttttttgt ttggattaaa           461
   Arg Arg Arg Asp Phe Phe Arg Lys
               90
   agaaaatttt cctctcctta attcacaaga caagaaaaaa aggaaatgta cctgtccttg     521
   aattactatt ttggaatgta taattatcta tatatataag aagaaaaaat tgcttaggaa     581
   ttt                                                                   584

<210>  6

<211>  94

<212>  PRT

<213>  Arabidopsis thaliana


<400>  6

Met Phe Arg Ser Asp Lys Ala Glu Lys Met Asp Lys Arg Arg Arg Arg
1               5                   10                  15

Gln Ser Lys Ala Lys Ala Ser Cys Ser Glu Glu Val Ser Ser Ile Glu
            20                  25                  30

Trp Glu Ala Val Lys Met Ser Glu Glu Glu Glu Asp Leu Ile Ser Arg
        35                  40                  45

Met Tyr Lys Leu Val Gly Asp Arg Trp Glu Leu Ile Ala Gly Arg Ile
    50                  55                  60

Pro Gly Arg Thr Pro Glu Glu Ile Glu Arg Tyr Trp Leu Met Lys His
65                  70                  75                  80

Gly Val Val Phe Ala Asn Arg Arg Arg Asp Phe Phe Arg Lys
                85                  90

<210>  7

<211>  407

<212>  DNA

<213>  Arabidopsis thaliana


<220>

<221>  CDS

<222>  (10)..(348)

<223>  G226



<400>  7
ccagtagtt atg gat aat acc aac cgt ctt cgt ctt cgt cgc ggt ccc agt      51
          Met Asp Asn Thr Asn Arg Leu Arg Leu Arg Arg Gly Pro Ser
          1               5                   10

ctt agg caa act aag ttc act cga tcc cga tat gac tct gaa gaa gtg        99
Leu Arg Gln Thr Lys Phe Thr Arg Ser Arg Tyr Asp Ser Glu Glu Val
15                  20                  25                  30

agt agc atc gaa tgg gag ttt atc agt atg acc gaa caa gaa gaa gat          147
Ser Ser Ile Glu Trp Glu Phe Ile Ser Met Thr Glu Gln Glu Glu Asp
                35                  40                  45

ctc atc tct cga atg tac aga ctt gtc ggt aat agg tgg gat tta ata          195
Leu Ile Ser Arg Met Tyr Arg Leu Val Gly Asn Arg Trp Asp Leu Ile
            50                  55                  60

gca gga aga gtc gta gga aga aag gca aat gag att gag aga tac tgg          243
Ala Gly Arg Val Val Gly Arg Lys Ala Asn Glu Ile Glu Arg Tyr Trp
        65                  70                  75

att atg aga aac tct gac tat ttt tct cac aaa cga cga cgt ctt aat          291
Ile Met Arg Asn Ser Asp Tyr Phe Ser His Lys Arg Arg Arg Leu Asn
    80                  85                  90

aat tct ccc ttt ttt tct act tct cct ctt aat ctc caa gaa aat cta          339
Asn Ser Pro Phe Phe Ser Thr Ser Pro Leu Asn Leu Gln Glu Asn Leu
95                  100                 105                 110

aaa ttg taa agaaatcaaa ataaaagctt tcaatcataa aagtagaaca                 388
Lys Leu

aatcttgaat gtcttctca                                                     407

<210>  8

<211>  112

<212>  PRT

<213>  Arabidopsis thaliana


<400>  8

Met Asp Asn Thr Asn Arg Leu Arg Leu Arg Arg Gly Pro Ser Leu Arg
1               5                   10                  15

Gln Thr Lys Phe Thr Arg Ser Arg Tyr Asp Ser Glu Glu Val Ser Ser
            20                  25                  30

Ile Glu Trp Glu Phe Ile Ser Met Thr Glu Gln Glu Glu Asp Leu Ile
        35                  40                  45

Ser Arg Met Tyr Arg Leu Val Gly Asn Arg Trp Asp Leu Ile Ala Gly
    50                  55                  60

Arg Val Val Gly Arg Lys Ala Asn Glu Ile Glu Arg Tyr Trp Ile Met
65                  70                  75                  80

Arg Asn Ser Asp Tyr Phe Ser His Lys Arg Arg Arg Leu Asn Asn Ser
                85                  90                  95

Pro Phe Phe Ser Thr Ser Pro Leu Asn Leu Gln Glu Asn Leu Lys Leu
            100                 105                 110

<210>  9

<211>  1547

<212>  DNA

<213>  Arabidopsis

<220>

<221>  CDS

<222>  (312)..(1310)

<223>  G256

<400>  9
tcgtgagcgt tgtgtttctc ctcaacattc aaagtcttta gtgaaacctc tcttgtaaga      60

agccaaaaaa ataaagagaa agattcaaag aaggaaagaa attgaggatg actatttcaa     120

gtccaaagag agattttgag tagaccctct tcacaaaaat ccaatcttag agtcttacta     180

gttactatct agcttacata cacagagaca ctataccaaa aatccaatct tattagagta     240

cttactatat agcttacaca tacacacaca cgaagtacta tttcaacgat caagagcgtg     300

tgcgtgagga t atg ggt aga cca cct tgt tgc gag aag att gag gtg aag     350
             Met Gly Arg Pro Pro Cys Cys Glu Lys Ile Glu Val Lys
             1               5                   10

aaa gga cca tgg act ccc gaa gaa gac ata atc ttg gtc tct tat atc     398
Lys Gly Pro Trp Thr Pro Glu Glu Asp Ile Ile Leu Val Ser Tyr Ile
    15                  20                  25

caa caa cac ggc cct gga aat tgg aga tct gtc cct gca aac acc ggt     446
Gln Gln His Gly Pro Gly Asn Trp Arg Ser Val Pro Ala Asn Thr Gly
30                  35                  40                  45

ttg cta agg tgt agc aag agt tgc aga ctt aga tgg act aat tac ctt     494
Leu Leu Arg Cys Ser Lys Ser Cys Arg Leu Arg Trp Thr Asn Tyr Leu
                50                  55                  60

cgt ccc ggg atc aaa cga gga aat ttc act caa ccg gaa gag aag atg     542
Arg Pro Gly Ile Lys Arg Gly Asn Phe Thr Gln Pro Glu Glu Lys Met
            65                  70                  75

atc atc cac ctt caa gct ctt ttg gga aat aga tgg gca gct ata gca     590
Ile Ile His Leu Gln Ala Leu Leu Gly Asn Arg Trp Ala Ala Ile Ala
        80                  85                  90

tca tat cta cct cag agg acc gac aat gat atc aag aac tac tgg aac     638
Ser Tyr Leu Pro Gln Arg Thr Asp Asn Asp Ile Lys Asn Tyr Trp Asn
    95                  100                 105

act cat ctt aaa aag aaa cta gtg atg atg aag ttt caa aat ggt atc     686
Thr His Leu Lys Lys Lys Leu Val Met Met Lys Phe Gln Asn Gly Ile
110                 115                 120                 125

atc aac gaa aac aaa acc aat ctg gca aca gat att tcg tct tgt aat     734
Ile Asn Glu Asn Lys Thr Asn Leu Ala Thr Asp Ile Ser Ser Cys Asn
                130                 135                 140

aat aac aac aat gga tgt aat cac aac aaa agg acc acc aac aaa ggc 782
Asn Asn Asn Asn Gly Cys Asn His Asn Lys Arg Thr Thr Asn Lys Gly
            145                 150                 155

caa tgg gag aaa aaa ctt caa aca gac atc aac atg gcc aaa caa gcc 830
Gln Trp Glu Lys Lys Leu Gln Thr Asp Ile Asn Met Ala Lys Gln Ala
        160                 165                 170

tta ttc caa gcc ttg tca ctt gac caa cca tct tca ttg atc cct ccc 878
Leu Phe Gln Ala Leu Ser Leu Asp Gln Pro Ser Ser Leu Ile Pro Pro
    175                 180                 185

gat cct gac tca cca aaa cct cat cat cat tct acc acc act tat gcc 926
Asp Pro Asp Ser Pro Lys Pro His His His Ser Thr Thr Thr Tyr Ala
190                 195                 200                 205

tca agc aca gat aac atc tct aaa tta ctc cag aac tgg aca agc tca 974
Ser Ser Thr Asp Asn Ile Ser Lys Leu Leu Gln Asn Trp Thr Ser Ser
                210                 215                 220

tca tcg tca aag cct aac act tca tca gtc tcc aac aac cgg agc tca 1022
Ser Ser Ser Lys Pro Asn Thr Ser Ser Val Ser Asn Asn Arg Ser Ser
            225                 230                 235

agc ccc ggt gaa gga gga ctt ttt gat cat cac tct ttg ttc tca tcg 1070
Ser Pro Gly Glu Gly Gly Leu Phe Asp His His Ser Leu Phe Ser Ser
        240                 245                 250

aat tca gaa tct gga tca gtt gat gag aag ctg aat ttg atg tcc gag 1118
Asn Ser Glu Ser Gly Ser Val Asp Glu Lys Leu Asn Leu Met Ser Glu
    255                 260                 265

aca agc atg ttc aaa ggt gag agc aag cca gac ata gac atg gaa gct 1166
Thr Ser Met Phe Lys Gly Glu Ser Lys Pro Asp Ile Asp Met Glu Ala
270                 275                 280                 285

aca cct act act act act act act act gat gat caa ggc tcg ttg tca 1214
Thr Pro Thr Thr Thr Thr Thr Thr Thr Asp Asp Gln Gly Ser Leu Ser
                290                 295                 300

ttg atc gag aaa tgg ttg ttt gat gat caa ggc ttg gtt cag tgt gat 1262
Leu Ile Glu Lys Trp Leu Phe Asp Asp Gln Gly Leu Val Gln Cys Asp
            305                 310                 315

gat agt caa gaa gat ctc atc gac gtg tct tta gag gag tta aaa taa 1310
Asp Ser Gln Glu Asp Leu Ile Asp Val Ser Leu Glu Glu Leu Lys
        320                 325                 330

tgataacaac agtcaagatt tgttctataa gaaaataaaa cgtatagaac aacgataaag 1370

ctagctaggt ttattaattt ttctttcttt tgtcttttct ctatgatctt tagttacatt 1430

ttattttact gtgtggcttg cttgtggtca agtcgatgaa gatcaaactg tgatatacta 1490

tttatatgta aagtactata aagttaagag tagttgaata aaaaaaaaaa aaaaaaa 1547

<210> 10

<211> 332

<212> PRT

<213> Arabidopsis

<400> 10

Met Gly Arg Pro Pro Cys Cys Glu Lys Ile Glu Val Lys Lys Gly Pro
1               5                   10                  15

Trp Thr Pro Glu Glu Asp Ile Ile Leu Val Ser Tyr Ile Gln Gln His
            20                  25                  30

Gly Pro Gly Asn Trp Arg Ser Val Pro Ala Asn Thr Gly Leu Leu Arg
        35                  40                  45

Cys Ser Lys Ser Cys Arg Leu Arg Trp Thr Asn Tyr Leu Arg Pro Gly
    50                  55                  60

Ile Lys Arg Gly Asn Phe Thr Gln Pro Glu Glu Lys Met Ile Ile His
65                  70                  75                  80

Leu Gln Ala Leu Leu Gly Asn Arg Trp Ala Ala Ile Ala Ser Tyr Leu
                85                  90                  95

Pro Gln Arg Thr Asp Asn Asp Ile Lys Asn Tyr Trp Asn Thr His Leu
            100                 105                 110

Lys Lys Lys Leu Val Met Met Lys Phe Gln Asn Gly Ile Ile Asn Glu
        115                 120                 125

Asn Lys Thr Asn Leu Ala Thr Asp Ile Ser Ser Cys Asn Asn Asn Asn
    130                 135                 140

Asn Gly Cys Asn His Asn Lys Arg Thr Thr Asn Lys Gly Gln Trp Glu
145                 150                 155                 160

Lys Lys Leu Gln Thr Asp Ile Asn Met Ala Lys Gln Ala Leu Phe Gln
                165                 170                 175

Ala Leu Ser Leu Asp Gln Pro Ser Ser Leu Ile Pro Pro Asp Pro Asp
            180                 185                 190

Ser Pro Lys Pro His His His Ser Thr Thr Thr Tyr Ala Ser Ser Thr
        195                 200                 205

Asp Asn Ile Ser Lys Leu Leu Gln Asn Trp Thr Ser Ser Ser Ser Ser
    210                 215                 220

Lys Pro Asn Thr Ser Ser Val Ser Asn Asn Arg Ser Ser Ser Pro Gly
225                 230                 235                 240

Glu Gly Gly Leu Phe Asp His His Ser Leu Phe Ser Ser Asn Ser Glu
                245                 250                 255

Ser Gly Ser Val Asp Glu Lys Leu Asn Leu Met Ser Glu Thr Ser Met
            260                 265                 270

Phe Lys Gly Glu Ser Lys Pro Asp Ile Asp Met Glu Ala Thr Pro Thr
        275                 280                 285

Thr Thr Thr Thr Thr Thr Asp Asp Gln Gly Ser Leu Ser Leu Ile Glu
    290                 295                 300

Lys Trp Leu Phe Asp Asp Gln Gly Leu Val Gln Cys Asp Asp Ser Gln
305                 310                 315                 320

Glu Asp Leu Ile Asp Val Ser Leu Glu Glu Leu Lys
                325                 330

<210>  11

<211>  2405

<212>  DNA

<213>  Arabidopsis thaliana

<220>

<221>  CDS

<222>  (381)..(2213)

<223>  G419

<400>  11
aagccacaca atctcttttc ttctctctct ctctgttata tctcttctgt ttaattcttt      60

tattcttctt cgtctatctt ctcctataat ctcttctctc tccctcttca cctaaagaat     120

aagaagaaaa ataattcaca tctttatgca aactactttc ttgtagggtt ttaggagcta     180

tctctattgt cttggttctg atacaaagtt ttgtaatttt catggtatga gaagatttgc     240

ctttctattt tgtttattgg ttctttttaa ctttttcttg gagatgggtt cttgtagatc     300

ttaatgaaac ttctgttttt gtcccaaaaa gagttttctt ttttcttctc ttctttttgg     360

gttttcaatt cttgagagac atg gca aga gat cag ttc tat ggt cac aat aac     413
                      Met Ala Arg Asp Gln Phe Tyr Gly His Asn Asn
                      1               5                   10

cat cat cat caa gag caa caa cat caa atg att aat cag atc caa ggg       461
His His His Gln Glu Gln Gln His Gln Met Ile Asn Gln Ile Gln Gly
            15                  20                  25

ttt gat gag aca aac caa aac cca acc gat cat cat cat tac aat cat       509
Phe Asp Glu Thr Asn Gln Asn Pro Thr Asp His His His Tyr Asn His
        30                  35                  40

cag atc ttt ggc tca aac tcc aac atg ggt atg atg ata gac ttc tct       557
Gln Ile Phe Gly Ser Asn Ser Asn Met Gly Met Met Ile Asp Phe Ser
    45                  50                  55

aag caa caa cag att agg atg aca agt ggt tcg gat cat cat cat cat 605
Lys Gln Gln Gln Ile Arg Met Thr Ser Gly Ser Asp His His His His
60                  65                  70                  75

cat cat cag aca agt ggt ggt act gat cag aat cag ctt ctg gaa gat 653
His His Gln Thr Ser Gly Gly Thr Asp Gln Asn Gln Leu Leu Glu Asp
                80                  85                  90

tct tca tct gcc atg aga cta tgc aat gtt aat aat gat ttc cca agt 701
Ser Ser Ser Ala Met Arg Leu Cys Asn Val Asn Asn Asp Phe Pro Ser
            95                  100                 105

gaa gta aat gat gag aga cca cca caa aga cca agc caa ggt ctt tcc 749
Glu Val Asn Asp Glu Arg Pro Pro Gln Arg Pro Ser Gln Gly Leu Ser
        110                 115                 120

ctt tct ctc tcc tct tca aat cct aca agc atc agt ctc caa tct ttc 797
Leu Ser Leu Ser Ser Ser Asn Pro Thr Ser Ile Ser Leu Gln Ser Phe
    125                 130                 135

gaa ctc aga ccc caa caa caa caa caa ggg tat tcc ggt aat aaa tca 845
Glu Leu Arg Pro Gln Gln Gln Gln Gln Gly Tyr Ser Gly Asn Lys Ser
140                 145                 150                 155

aca caa cat cag aat ctc caa cac acg cag atg atg atg atg atg atg 893
Thr Gln His Gln Asn Leu Gln His Thr Gln Met Met Met Met Met Met
                160                 165                 170

aat agt cac cac caa aac aac aac aat aac aat cat cag cat cat aat 941
Asn Ser His His Gln Asn Asn Asn Asn Asn Asn His Gln His His Asn
            175                 180                 185

cat cat cag ttt cag att ggg agt tcc aag tat ttg agt cca gct caa 989
His His Gln Phe Gln Ile Gly Ser Ser Lys Tyr Leu Ser Pro Ala Gln
        190                 195                 200

gag cta ctg agt gag ttt tgc agt ctt gga gta aag gaa agc gat gaa 1037
Glu Leu Leu Ser Glu Phe Cys Ser Leu Gly Val Lys Glu Ser Asp Glu
    205                 210                 215

gaa gtg atg atg atg aag cat aag aag aag caa aag ggt aaa caa caa 1085
Glu Val Met Met Met Lys His Lys Lys Lys Gln Lys Gly Lys Gln Gln
220                 225                 230                 235

gaa gag tgg gac aca agt cac cac agc aac aat gat caa cat gac caa 1133
Glu Glu Trp Asp Thr Ser His His Ser Asn Asn Asp Gln His Asp Gln
                240                 245                 250

tct gcg act act tct tca aag aaa cat gtt cca cca ctt cac tct ctt 1181
Ser Ala Thr Thr Ser Ser Lys Lys His Val Pro Pro Leu His Ser Leu
            255                 260                 265

gag ttc atg gaa ctt cag aaa aga aaa gcc aag ttg ctc tcc atg ctc 1229
Glu Phe Met Glu Leu Gln Lys Arg Lys Ala Lys Leu Leu Ser Met Leu
        270                 275                 280

gaa gag ctt aaa aga aga tat gga cat tac cga gag caa atg aga gtt 1277
Glu Glu Leu Lys Arg Arg Tyr Gly His Tyr Arg Glu Gln Met Arg Val
    285                 290                 295

gcg gcg gca gcc ttt gaa gcg gcg gtt gga cta gga ggg gca gag ata 1325
Ala Ala Ala Ala Phe Glu Ala Ala Val Gly Leu Gly Gly Ala Glu Ile
300                 305                 310                 315

tac act gcg tta gcg tca agg gca atg tca aga cac ttt cgg tgt tta 1373
Tyr Thr Ala Leu Ala Ser Arg Ala Met Ser Arg His Phe Arg Cys Leu
                320                 325                 330

aaa gac gga ctt gtg gga cag att caa gca aca agt caa gct ttg gga 1421
Lys Asp Gly Leu Val Gly Gln Ile Gln Ala Thr Ser Gln Ala Leu Gly
            335                 340                 345

gag aga gaa gag gat aat cgt gcg gtt tct att gca gca cgt gga gaa 1469
Glu Arg Glu Glu Asp Asn Arg Ala Val Ser Ile Ala Ala Arg Gly Glu
        350                 355                 360

act cca cgg ttg aga ttg ctc gat caa gct ttg cgg caa cag aaa tcg 1517
Thr Pro Arg Leu Arg Leu Leu Asp Gln Ala Leu Arg Gln Gln Lys Ser
    365                 370                 375

tat cgc caa atg act ctt gtt gac gct cat cct tgg cgt cca caa cgc 1565
Tyr Arg Gln Met Thr Leu Val Asp Ala His Pro Trp Arg Pro Gln Arg
380                 385                 390                 395

ggc ttg cct gaa cgc gca gtc aca acg ttg aga gct tgg ctc ttt gaa 1613
Gly Leu Pro Glu Arg Ala Val Thr Thr Leu Arg Ala Trp Leu Phe Glu
                400                 405                 410

cac ttt ctt cac cca tat ccg agc gat gtt gat aag cat ata ttg gcc 1661
His Phe Leu His Pro Tyr Pro Ser Asp Val Asp Lys His Ile Leu Ala
            415                 420                 425

cga caa act ggt tta tca aga agt cag gta tca aat tgg ttt att aat 1709
Arg Gln Thr Gly Leu Ser Arg Ser Gln Val Ser Asn Trp Phe Ile Asn
        430                 435                 440

gca aga gtt agg cta tgg aaa cca atg att gaa gaa atg tac tgt gaa 1757
Ala Arg Val Arg Leu Trp Lys Pro Met Ile Glu Glu Met Tyr Cys Glu
    445                 450                 455

gaa aca aga agt gaa caa atg gag att aca aac ccg atg atg atc gat 1805
Glu Thr Arg Ser Glu Gln Met Glu Ile Thr Asn Pro Met Met Ile Asp
460                 465                 470                 475

act aaa ccg gac ccg gac cag ttg atc cgt gtc gaa ccg gaa tct tta 1853
Thr Lys Pro Asp Pro Asp Gln Leu Ile Arg Val Glu Pro Glu Ser Leu
                480                 485                 490

tcc tca ata gtg aca aac cct aca tcc aaa tcc ggt cac aac tca acc 1901
Ser Ser Ile Val Thr Asn Pro Thr Ser Lys Ser Gly His Asn Ser Thr
            495                 500                 505

cat gga acg atg tcg tta ggg tca acg ttt gac ttt tcc ttg tac ggt 1949
His Gly Thr Met Ser Leu Gly Ser Thr Phe Asp Phe Ser Leu Tyr Gly
        510                 515                 520

aac caa gct gtg aca tac gct ggt gaa gga ggg cca cgt ggt gac gtt 1997
Asn Gln Ala Val Thr Tyr Ala Gly Glu Gly Gly Pro Arg Gly Asp Val
    525                 530                 535

tcc ttg acg ctt ggg tta caa cgt aac gat ggt aac ggt ggt gtg agt 2045
Ser Leu Thr Leu Gly Leu Gln Arg Asn Asp Gly Asn Gly Gly Val Ser
540                 545                 550                 555

tta gcg ttg tct cca gtg acg gct caa ggt ggc caa ctt ttc tac ggt 2093
Leu Ala Leu Ser Pro Val Thr Ala Gln Gly Gly Gln Leu Phe Tyr Gly
                560                 565                 570

aga gac cac att gaa gaa gga ccg gtt caa tat tca gcg tcg atg tta 2141
Arg Asp His Ile Glu Glu Gly Pro Val Gln Tyr Ser Ala Ser Met Leu
            575                 580                 585

gat gat gat caa gtt cag aat ttg cct tat agg aat ttg atg gga gct 2189
Asp Asp Asp Gln Val Gln Asn Leu Pro Tyr Arg Asn Leu Met Gly Ala
        590                 595                 600

caa tta ctt cat gat att gtt tga gattaaaaga ttaggaccaa agttatcgat    2243
Gln Leu Leu His Asp Ile Val
    605                 610

acatattttc caaaaccgat tcggttatgt aacggtttag ttagataaaa accaaattag    2303

atatttatat ataccgttgt ctgattggat tggaggattg gtggacaagg agatattatt    2363

aatgtatgag ttagttggtt cgtcaaaaaa aaaaaaaaaa aa                       2405

<210>  12

<211>  610

<212>  PRT

<213>  Arabidopsis thaliana

<400>  12

Met Ala Arg Asp Gln Phe Tyr Gly His Asn Asn His His His Gln Glu
1               5                   10                  15

Gln Gln His Gln Met Ile Asn Gln Ile Gln Gly Phe Asp Glu Thr Asn
            20                  25                  30

Gln Asn Pro Thr Asp His His His Tyr Asn His Gln Ile Phe Gly Ser
        35                  40                  45

Asn Ser Asn Met Gly Met Met Ile Asp Phe Ser Lys Gln Gln Gln Ile
    50                  55                  60

Arg Met Thr Ser Gly Ser Asp His His His His His His Gln Thr Ser
65                  70                  75                  80

Gly Gly Thr Asp Gln Asn Gln Leu Leu Glu Asp Ser Ser Ser Ala Met
                85                  90                  95

Arg Leu Cys Asn Val Asn Asn Asp Phe Pro Ser Glu Val Asn Asp Glu
            100                 105                 110

Arg Pro Pro Gln Arg Pro Ser Gln Gly Leu Ser Leu Ser Leu Ser Ser
        115                 120                 125

Ser Asn Pro Thr Ser Ile Ser Leu Gln Ser Phe Glu Leu Arg Pro Gln
    130                 135                 140

Gln Gln Gln Gln Gly Tyr Ser Gly Asn Lys Ser Thr Gln His Gln Asn
145                 150                 155                 160

Leu Gln His Thr Gln Met Met Met Met Met Met Asn Ser His His Gln
                165                 170                 175

Asn Asn Asn Asn Asn Asn His Gln His His Asn His His Gln Phe Gln
            180                 185                 190

Ile Gly Ser Ser Lys Tyr Leu Ser Pro Ala Gln Glu Leu Leu Ser Glu
        195                 200                 205

Phe Cys Ser Leu Gly Val Lys Glu Ser Asp Glu Glu Val Met Met Met
    210                 215                 220

Lys His Lys Lys Lys Gln Lys Gly Lys Gln Gln Glu Glu Trp Asp Thr
225                 230                 235                 240

Ser His His Ser Asn Asn Asp Gln His Asp Gln Ser Ala Thr Thr Ser
                245                 250                 255

Ser Lys Lys His Val Pro Pro Leu His Ser Leu Glu Phe Met Glu Leu
            260                 265                 270

Gln Lys Arg Lys Ala Lys Leu Leu Ser Met Leu Glu Glu Leu Lys Arg
        275                 280                 285

Arg Tyr Gly His Tyr Arg Glu Gln Met Arg Val Ala Ala Ala Ala Phe
    290                 295                 300

Glu Ala Ala Val Gly Leu Gly Gly Ala Glu Ile Tyr Thr Ala Leu Ala
305                 310                 315                 320

Ser Arg Ala Met Ser Arg His Phe Arg Cys Leu Lys Asp Gly Leu Val
                325                 330                 335

Gly Gln Ile Gln Ala Thr Ser Gln Ala Leu Gly Glu Arg Glu Glu Asp
            340                 345                 350

Asn Arg Ala Val Ser Ile Ala Ala Arg Gly Glu Thr Pro Arg Leu Arg
        355                 360                 365

Leu Leu Asp Gln Ala Leu Arg Gln Gln Lys Ser Tyr Arg Gln Met Thr
    370                 375                 380

Leu Val Asp Ala His Pro Trp Arg Pro Gln Arg Gly Leu Pro Glu Arg
385                 390                 395                 400

Ala Val Thr Thr Leu Arg Ala Trp Leu Phe Glu His Phe Leu His Pro
                405                 410                 415

Tyr Pro Ser Asp Val Asp Lys His Ile Leu Ala Arg Gln Thr Gly Leu
            420                 425                 430

Ser Arg Ser Gln Val Ser Asn Trp Phe Ile Asn Ala Arg Val Arg Leu
        435                 440                 445

Trp Lys Pro Met Ile Glu Glu Met Tyr Cys Glu Glu Thr Arg Ser Glu
    450                 455                 460

Gln Met Glu Ile Thr Asn Pro Met Met Ile Asp Thr Lys Pro Asp Pro
465                 470                 475                 480

Asp Gln Leu Ile Arg Val Glu Pro Glu Ser Leu Ser Ser Ile Val Thr
                485                 490                 495

Asn Pro Thr Ser Lys Ser Gly His Asn Ser Thr His Gly Thr Met Ser
            500                 505                 510

Leu Gly Ser Thr Phe Asp Phe Ser Leu Tyr Gly Asn Gln Ala Val Thr
        515                 520                 525

Tyr Ala Gly Glu Gly Gly Pro Arg Gly Asp Val Ser Leu Thr Leu Gly
    530                 535                 540

Leu Gln Arg Asn Asp Gly Asn Gly Gly Val Ser Leu Ala Leu Ser Pro
545                 550                 555                 560

Val Thr Ala Gln Gly Gly Gln Leu Phe Tyr Gly Arg Asp His Ile Glu
                565                 570                 575

Glu Gly Pro Val Gln Tyr Ser Ala Ser Met Leu Asp Asp Asp Gln Val
            580                 585                 590

Gln Asn Leu Pro Tyr Arg Asn Leu Met Gly Ala Gln Leu Leu His Asp
        595                 600                 605

Ile Val
    610

<210>  13

<211>  989

<212>  DNA

<213>  Arabidopsis thaliana


<220>

<221>  CDS

<222>  (41)..(664)

<223>  G464


<400>  13
ctctgctggt atcattggag tctagggttt tgttattgac atg cgt ggt gtg tca       55

Met Arg Gly Val Ser
1               5

gaa ttg gag gtg ggg aag agt aat ctt ccg gcg gag agt gag ctg gaa     103
Glu Leu Glu Val Gly Lys Ser Asn Leu Pro Ala Glu Ser Glu Leu Glu
            10                  15                  20

ttg gga tta ggg ctc agc ctc ggt ggt ggc gcg tgg aaa gag cgt ggg     151
Leu Gly Leu Gly Leu Ser Leu Gly Gly Gly Ala Trp Lys Glu Arg Gly
        25                  30                  35

agg att ctt act gct aag gat ttt cct tcc gtt ggg tct aaa cgc tct     199
Arg Ile Leu Thr Ala Lys Asp Phe Pro Ser Val Gly Ser Lys Arg Ser
    40                  45                  50

gct gaa tct tcc tct cac caa gga gct tct cct cct cgt tca agt caa     247
Ala Glu Ser Ser Ser His Gln Gly Ala Ser Pro Pro Arg Ser Ser Gln
    55                  60                  65

gtg gta gga tgg cca cca att ggg tta cac agg atg aac agt ttg gtt     295
Val Val Gly Trp Pro Pro Ile Gly Leu His Arg Met Asn Ser Leu Val
70                  75                  80                  85

aat aac caa gct atg aag gca gca aga gcg gaa gaa gga gac ggg gag     343
Asn Asn Gln Ala Met Lys Ala Ala Arg Ala Glu Glu Gly Asp Gly Glu
            90                  95                  100

aag aaa gtt gtg aag aat ggt gag ctc aaa gat gtg tca atg aag gtg     391
Lys Lys Val Val Lys Asn Gly Glu Leu Lys Asp Val Ser Met Lys Val
        105                 110                 115

aat ccg aaa gtt cag ggc tta ggg ttt gtt aag gtg aat atg gat gga     439
Asn Pro Lys Val Gln Gly Leu Gly Phe Val Lys Val Asn Met Asp Gly
    120                 125                 130

gtt ggt ata ggc aga aaa gtg gat atg aga gct cat tcg tct tac gaa     487
Val Gly Ile Gly Arg Lys Val Asp Met Arg Ala His Ser Ser Tyr Glu
    135                 140                 145

aac ttg gct cag acg ctt gag gaa atg ttc ttt gga atg aca ggt act     535
Asn Leu Ala Gln Thr Leu Glu Glu Met Phe Phe Gly Met Thr Gly Thr
150                 155                 160                 165

act tgt cga gaa acg gtt aaa cct tta agg ctt tta gat gga tca tca     583
Thr Cys Arg Glu Thr Val Lys Pro Leu Arg Leu Leu Asp Gly Ser Ser
            170                 175                 180

gac ttt gta ctc act tat gaa gat aag ggg att gga tgc ttg ttg gag     631
Asp Phe Val Leu Thr Tyr Glu Asp Lys Gly Ile Gly Cys Leu Leu Glu
        185                 190                 195

atg ttc cat gga gaa tgt tta tca act cgg tga aaaggcttcg gatcatggga     684
Met Phe His Gly Glu Cys Leu Ser Thr Arg
    200                 205

acctcagaag ctagtggact agctccaaga cgtcaagagc agaaggatag acaaagaaac     744

aaccctgttt agcttccctt ccaaagctgg cattgtttat gtattgtttg aggtttgcaa     804

tttactcgat actttttgaa gaaagtattt tggagaatat ggataaaagc atgcagaagc     864

ttagatatga tttgaatccg gttttcggat atggttttgc ttaggtcatt caattcgtag     924

ttttccagtt tgtttcttct ttggctgtgt accaattatc tatgttctgt gagagaaagc     984

tcttg                                                                 989

<210>  14

<211>  207

<212>  PRT

<213>  Arabidopsis thaliana


<400>  14

Met Arg Gly Val Ser Glu Leu Glu Val Gly Lys Ser Asn Leu Pro Ala
1               5                   10                  15

Glu Ser Glu Leu Glu Leu Gly Leu Gly Leu Ser Leu Gly Gly Gly Ala
            20                  25                  30

Trp Lys Glu Arg Gly Arg Ile Leu Thr Ala Lys Asp Phe Pro Ser Val
        35                  40                  45

Gly Ser Lys Arg Ser Ala Glu Ser Ser Ser His Gln Gly Ala Ser Pro
    50                  55                  60

Pro Arg Ser Ser Gln Val Val Gly Trp Pro Pro Ile Gly Leu His Arg
65                  70                  75                  80

Met Asn Ser Leu Val Asn Asn Gln Ala Met Lys Ala Ala Arg Ala Glu
                85                  90                  95

Glu Gly Asp Gly Glu Lys Lys Val Val Lys Asn Gly Glu Leu Lys Asp
            100                 105                 110

Val Ser Met Lys Val Asn Pro Lys Val Gln Gly Leu Gly Phe Val Lys
        115                 120                 125

Val Asn Met Asp Gly Val Gly Ile Gly Arg Lys Val Asp Met Arg Ala
    130                 135                 140

His Ser Ser Tyr Glu Asn Leu Ala Gln Thr Leu Glu Glu Met Phe Phe
145                 150                 155                 160

Gly Met Thr Gly Thr Thr Cys Arg Glu Thr Val Lys Pro Leu Arg Leu
                165                 170                 175

Leu Asp Gly Ser Ser Asp Phe Val Leu Thr Tyr Glu Asp Lys Gly Ile
            180                 185                 190

Gly Cys Leu Leu Glu Met Phe His Gly Glu Cys Leu Ser Thr Arg
        195                 200                 205

<210>  15

<211>  1065

<212>  DNA

<213>  Arabidopsis thaliana

<220>

<221>  CDS

<222>  (188)..(760)

<223>  G482

<400>  15
tcgacccacg cgtccggaca cttaacaatt cacaccttct ctttttactc ttcctaaaac      60

cctaaatttc ctcgcttcag tcttcccact caagtcaacc accaattgaa ttcgatttcg     120

aatcattgat ggaaatgatt tgaaaaaaga gtaaagttta tttttttatt ccttgtaatt     180

ttcagaa atg ggg gat tcc gac agg gat tcc ggt gga ggg caa aac ggg       229
        Met Gly Asp Ser Asp Arg Asp Ser Gly Gly Gly Gln Asn Gly
        1               5                   10

aac aac cag aac gga cag tcc tcc ttg tct cca aga gag caa gac agg       277
Asn Asn Gln Asn Gly Gln Ser Ser Leu Ser Pro Arg Glu Gln Asp Arg
15                  20                  25                  30

ttc ttg ccg atc gct aac gtc agc cgg atc atg aag aag gcc ttg ccc       325
Phe Leu Pro Ile Ala Asn Val Ser Arg Ile Met Lys Lys Ala Leu Pro
                35                  40                  45

gcc aac gcc aag atc tct aaa gat gcc aaa gag acg atg cag gag tgt       373
Ala Asn Ala Lys Ile Ser Lys Asp Ala Lys Glu Thr Met Gln Glu Cys
            50                  55                  60

gtc tcc gag ttc atc agc ttc gtc acc gga gaa gca tct gat aag tgt       421
Val Ser Glu Phe Ile Ser Phe Val Thr Gly Glu Ala Ser Asp Lys Cys
        65                  70                  75

cag aag gag aag agg aag acg atc aac gga gac gat ttg ctc tgg gct       469
Gln Lys Glu Lys Arg Lys Thr Ile Asn Gly Asp Asp Leu Leu Trp Ala
    80                  85                  90

atg act act cta ggt ttt gag gat tat gtt gag cca ttg aaa gtt tac       517
Met Thr Thr Leu Gly Phe Glu Asp Tyr Val Glu Pro Leu Lys Val Tyr
95                  100                 105                 110

ttg cag agg ttt agg gag atc gaa ggg gag agg act gga cta ggg agg       565
Leu Gln Arg Phe Arg Glu Ile Glu Gly Glu Arg Thr Gly Leu Gly Arg
                115                 120                 125

cca cag act ggt ggt gag gtc gga gag cat cag aga gat gct gtc gga       613
Pro Gln Thr Gly Gly Glu Val Gly Glu His Gln Arg Asp Ala Val Gly
            130                 135                 140

gat ggc ggt ggg ttc tac ggt ggt ggt ggg atg cag tat cac caa           661
Asp Gly Gly Gly Phe Tyr Gly Gly Gly Gly Met Gln Tyr His Gln
        145                 150                 155

cat cat cag ttt ctt cac cag cag aac cat atg tat gga gcc aca ggt       709
His His Gln Phe Leu His Gln Gln Asn His Met Tyr Gly Ala Thr Gly
    160                 165                 170

ggc ggt agc gac agt gga ggt gga gct gcc tcc ggt agg aca agg act 757
Gly Gly Ser Asp Ser Gly Gly Gly Ala Ala Ser Gly Arg Thr Arg Thr
175 180 185 190

taa caaagattgg tgaagtggat ctctctctgt atatagatac ataaatacat 810

gtatacacat gcctattttt acgacccata taaggtatct atcatgtgat agaacgaaca 870

ttggtgttgg tgatgtaaaa tcagatgtgc attaagggtt tagattttga ggctgtgtaa 930

aagaagatca agtgtgcttt gttggacaat aggattcact aacgaatctg cttcattgga 990

tcttgtatgt aactaaagcc attgtattga atgcaaatgt tttcatttgg gatgctttaa 1050

aaaaaaaaaa aaaaa 1065

<210> 16

<211> 190

<212> PRT

<213> Arabidopsis thaliana

<400> 16

Met Gly Asp Ser Asp Arg Asp Ser Gly Gly Gly Gln Asn Gly Asn Asn
1               5                   10                  15

Gln Asn Gly Gln Ser Ser Leu Ser Pro Arg Glu Gln Asp Arg Phe Leu
            20                  25                  30

Pro Ile Ala Asn Val Ser Arg Ile Met Lys Lys Ala Leu Pro Ala Asn
        35                  40                  45

Ala Lys Ile Ser Lys Asp Ala Lys Glu Thr Met Gln Glu Cys Val Ser
    50                  55                  60

Glu Phe Ile Ser Phe Val Thr Gly Glu Ala Ser Asp Lys Cys Gln Lys
65                  70                  75                  80

Glu Lys Arg Lys Thr Ile Asn Gly Asp Asp Leu Leu Trp Ala Met Thr
                85                  90                  95

Thr Leu Gly Phe Glu Asp Tyr Val Glu Pro Leu Lys Val Tyr Leu Gln
            100                 105                 110

Arg Phe Arg Glu Ile Glu Gly Glu Arg Thr Gly Leu Gly Arg Pro Gln
        115                 120                 125

Thr Gly Gly Glu Val Gly Glu His Gln Arg Asp Ala Val Gly Asp Gly
    130                 135                 140

Gly Gly Phe Tyr Gly Gly Gly Gly Gly Met Gln Tyr His Gln His His
145                 150                 155                 160

Gln Phe Leu His Gln Gln Asn His Met Tyr Gly Ala Thr Gly Gly Gly
                165                 170                 175

Ser Asp Ser Gly Gly Gly Ala Ala Ser Gly Arg Thr Arg Thr
            180                 185                 190

<210>  17

<211>  1409

<212>  DNA

<213>  Arabidopsis thaliana


<220>

<221>  CDS

<222>  (224)..(1093)

<223>  G502



<400>  17
ttgatgccgc tcaatcccac tatccttcgc aaggaccctt cctctatata aggaagttca      60

tttcatttgg agaggacacg ctgacaagct gactctagca gatctgggac cgtcgaccca     120

cgcgtccgaa ttgattagga taggatcagg atcatcctca acaacctcct cctaattcct     180

cctccattca tagtaacaat aatattaaga aagagggtaa act atg tca gaa tta       235
                                                Met Ser Glu Leu
                                                1

tta cag ttg cct cca ggt ttc cga ttt cac cct acc gat gaa gag ctt       283
Leu Gln Leu Pro Pro Gly Phe Arg Phe His Pro Thr Asp Glu Glu Leu
5                   10                  15                  20

gtc atg cac tat ctc tgc cgc aaa tgt gcc tct cag tcc atc gcc gtt       331
Val Met His Tyr Leu Cys Arg Lys Cys Ala Ser Gln Ser Ile Ala Val
                25                  30                  35

ccg atc atc gct gag atc gat ctc tac aaa tac gat cca tgg gag ctt       379
Pro Ile Ile Ala Glu Ile Asp Leu Tyr Lys Tyr Asp Pro Trp Glu Leu
            40                  45                  50

cct ggt tta gcc ttg tat ggt gag aag gaa tgg tac ttc ttc tct ccc       427
Pro Gly Leu Ala Leu Tyr Gly Glu Lys Glu Trp Tyr Phe Phe Ser Pro
        55                  60                  65

agg gac aga aaa tat ccc aac ggt tcg cgt cct aac cgg tcc gct ggt       475
Arg Asp Arg Lys Tyr Pro Asn Gly Ser Arg Pro Asn Arg Ser Ala Gly
    70                  75                  80

tct ggt tac tgg aaa gct acc gga gct gat aaa ccg atc gga cta cct       523
Ser Gly Tyr Trp Lys Ala Thr Gly Ala Asp Lys Pro Ile Gly Leu Pro
85                  90                  95                  100

aaa ccg gtc gga att aag aaa gct ctt gtt ttc tac gcc ggc aaa gct       571

Lys Pro Val Gly Ile Lys Lys Ala Leu Val Phe Tyr Ala Gly Lys Ala
                105                 110                 115

cca aag gga gag aaa acc aat tgg atc atg cac gag tac cgt ctc gcc      619
Pro Lys Gly Glu Lys Thr Asn Trp Ile Met His Glu Tyr Arg Leu Ala
            120                 125                 130

gac gtt gac cgg tcc gtt cgc aag aag aag aat agt ctc agg ctg gat      667
Asp Val Asp Arg Ser Val Arg Lys Lys Lys Asn Ser Leu Arg Leu Asp
        135                 140                 145

gat tgg gtt ctc tgc cgg att tac aac aaa aaa gga gct acc gag agg      715
Asp Trp Val Leu Cys Arg Ile Tyr Asn Lys Lys Gly Ala Thr Glu Arg
    150                 155                 160

cgg gga cca ccg cct ccg gtt gtt tac ggc gac gaa atc atg gag gag      763
Arg Gly Pro Pro Pro Pro Val Val Tyr Gly Asp Glu Ile Met Glu Glu
165                 170                 175                 180

aag ccg aag gtg acg gag atg gtt atg cct ccg ccg ccg caa cag aca      811
Lys Pro Lys Val Thr Glu Met Val Met Pro Pro Pro Pro Gln Gln Thr
                185                 190                 195

agt gag ttc gcg tat ttc gac acg tcg gat tcg gtg ccg aag ctg cat      859
Ser Glu Phe Ala Tyr Phe Asp Thr Ser Asp Ser Val Pro Lys Leu His
            200                 205                 210

act acg gat tcg agt tgc tcg gag cag gtg gtg tcg ccg gag ttc acg      907
Thr Thr Asp Ser Ser Cys Ser Glu Gln Val Val Ser Pro Glu Phe Thr
        215                 220                 225

agc gag gtt cag agc gag ccc aag tgg aaa gat tgg tcg gcc gta agt      955
Ser Glu Val Gln Ser Glu Pro Lys Trp Lys Asp Trp Ser Ala Val Ser
    230                 235                 240

aat gac aat aac aat acc ctt gat ttt ggg ttt aat tac att gat gcc     1003
Asn Asp Asn Asn Asn Thr Leu Asp Phe Gly Phe Asn Tyr Ile Asp Ala
245                 250                 255                 260

acc gtg gat aac gcg ttt gga gga gga ggg agt agt aat cag atg ttt     1051
Thr Val Asp Asn Ala Phe Gly Gly Gly Gly Ser Ser Asn Gln Met Phe
                265                 270                 275

ccg cta cag gat atg ttc atg tac atg cag aag cct tac tag             1093
Pro Leu Gln Asp Met Phe Met Tyr Met Gln Lys Pro Tyr
            280                 285

aagggaattc ctttcctgcc gccgaaacgc aacgcaaaac gaccctcgtt tttgcgttta   1153

tggcaacacg agaccgtttt atatggtcaa tgagtgtgcc gattcggcca ttagatttct   1213

gttcagtctt cgtttattct atagaccgtc cgatttcaga tcatccctaa tcggacggtg   1273

gtcgttggat gtatcagtag tgtattactg tgttaggtag aagaaaatcc acttgttctt   1333

aaattggcat aaaagtcaga agctaatatt tatatgtgcc gcaatcaatt taatattttc   1393

tgtctaaaaa aaaaaa                                                   1409

<210>  18

<211>  289

<212>  PRT

<213>  Arabidopsis thaliana

<400> 18

Met Ser Glu Leu Leu Gln Leu Pro Pro Gly Phe Arg Phe His Pro Thr
1               5                   10                  15

Asp Glu Glu Leu Val Met His Tyr Leu Cys Arg Lys Cys Ala Ser Gln
            20                  25                  30

Ser Ile Ala Val Pro Ile Ile Ala Glu Ile Asp Leu Tyr Lys Tyr Asp
        35                  40                  45

Pro Trp Glu Leu Pro Gly Leu Ala Leu Tyr Gly Glu Lys Glu Trp Tyr
    50                  55                  60

Phe Phe Ser Pro Arg Asp Arg Lys Tyr Pro Asn Gly Ser Arg Pro Asn
65                  70                  75                  80

Arg Ser Ala Gly Ser Gly Tyr Trp Lys Ala Thr Gly Ala Asp Lys Pro
                85                  90                  95

Ile Gly Leu Pro Lys Pro Val Gly Ile Lys Lys Ala Leu Val Phe Tyr
            100                 105                 110

Ala Gly Lys Ala Pro Lys Gly Glu Lys Thr Asn Trp Ile Met His Glu
        115                 120                 125

Tyr Arg Leu Ala Asp Val Asp Arg Ser Val Arg Lys Lys Lys Asn Ser
    130                 135                 140

Leu Arg Leu Asp Asp Trp Val Leu Cys Arg Ile Tyr Asn Lys Lys Gly
145                 150                 155                 160

Ala Thr Glu Arg Arg Gly Pro Pro Pro Pro Val Val Tyr Gly Asp Glu
                165                 170                 175

Ile Met Glu Glu Lys Pro Lys Val Thr Glu Met Val Met Pro Pro Pro
            180                 185                 190

Pro Gln Gln Thr Ser Glu Phe Ala Tyr Phe Asp Thr Ser Asp Ser Val
        195                 200                 205

Pro Lys Leu His Thr Thr Asp Ser Ser Cys Ser Glu Gln Val Val Ser
    210                 215                 220

Pro Glu Phe Thr Ser Glu Val Gln Ser Glu Pro Lys Trp Lys Asp Trp
225                 230                 235                 240

Ser Ala Val Ser Asn Asp Asn Asn Asn Thr Leu Asp Phe Gly Phe Asn
                245                 250                 255

Tyr Ile Asp Ala Thr Val Asp Asn Ala Phe Gly Gly Gly Gly Ser Ser
            260                 265                 270

Asn Gln Met Phe Pro Leu Gln Asp Met Phe Met Tyr Met Gln Lys Pro
        275                 280                 285

Tyr

<210>  19
<211>  1481
<212>  DNA
<213>  Arabidopsis thaliana

<220>
<221>  CDS
<222>  (181)..(1188)
<223>  G526

<400>  19
cgacccacgc gtccgagatt ctctcccagc tagctttctc aattcatttt tctttcttca      60

tcttcttctt gtgtgatctc tctttccaaa taagcttatc attcttacaa aaatatttct     120

gggtttctga tattgttctt gttctcttga atctttatta cttgaaaaac atataaagtg     180

atg gcg gtt gtg gtt gaa gaa ggt gtg gtg ttg aat cat gga ggt gaa       228
Met Ala Val Val Val Glu Glu Gly Val Val Leu Asn His Gly Gly Glu
1               5                   10                  15

gag ctt gtg gat ttg cca cct ggt ttc agg ttt cat cca aca gac gaa       276
Glu Leu Val Asp Leu Pro Pro Gly Phe Arg Phe His Pro Thr Asp Glu
            20                  25                  30

gag atc ata aca tgt tac ctt aag gag aag gtt tta aac agc cga ttc       324
Glu Ile Ile Thr Cys Tyr Leu Lys Glu Lys Val Leu Asn Ser Arg Phe
        35                  40                  45

acg gct gtg gcc atg gga gaa gct gat ctc aac aag tgt gag cct tgg       372
Thr Ala Val Ala Met Gly Glu Ala Asp Leu Asn Lys Cys Glu Pro Trp
    50                  55                  60

gat ttg cca aag agg gca aag atg ggg gag aaa gag ttc tac ttc ttc       420
Asp Leu Pro Lys Arg Ala Lys Met Gly Glu Lys Glu Phe Tyr Phe Phe
65                  70                  75                  80

tgt caa agg gac agg aag tat ccg act ggg atg agg acg aac cgt gcg       468
Cys Gln Arg Asp Arg Lys Tyr Pro Thr Gly Met Arg Thr Asn Arg Ala
                85                  90                  95

acg gag tca gga tac tgg aaa gcc acc ggg aag gat aag gag atc ttc       516
Thr Glu Ser Gly Tyr Trp Lys Ala Thr Gly Lys Asp Lys Glu Ile Phe
            100                 105                 110

aaa ggc aaa ggt tgt ctc gtt ggg atg aag aaa aca ctt gtg ttt tat 564
Lys Gly Lys Gly Cys Leu Val Gly Met Lys Lys Thr Leu Val Phe Tyr
        115                 120                 125

aga gga aga gct cca aaa ggt gaa aag act aat tgg gtc atg cat gaa 612
Arg Gly Arg Ala Pro Lys Gly Glu Lys Thr Asn Trp Val Met His Glu
    130                 135                 140

tat cgt ctt gaa ggc aaa tat tcg tat tac aat ctc cca aaa tct gca 660
Tyr Arg Leu Glu Gly Lys Tyr Ser Tyr Tyr Asn Leu Pro Lys Ser Ala
145                 150                 155                 160

agg gac gaa tgg gtc gtg tgt agg gtt ttt cac aag aac aat cct tct 708
Arg Asp Glu Trp Val Val Cys Arg Val Phe His Lys Asn Asn Pro Ser
                165                 170                 175

acc aca acc caa cca atg acg aga ata ccc gtt gaa gat ttc aca agg 756
Thr Thr Thr Gln Pro Met Thr Arg Ile Pro Val Glu Asp Phe Thr Arg
            180                 185                 190

atg gat tct cta gag aac att gat cat ctc cta gac ttc tca tct ctt 804
Met Asp Ser Leu Glu Asn Ile Asp His Leu Leu Asp Phe Ser Ser Leu
        195                 200                 205

cct cct ctc ata gac ccg agt ttc atg agt caa acc gaa caa cca aac 852
Pro Pro Leu Ile Asp Pro Ser Phe Met Ser Gln Thr Glu Gln Pro Asn
    210                 215                 220

ttc aaa ccc atc aac cct cca act tac gat atc tca tca cca atc caa 900
Phe Lys Pro Ile Asn Pro Pro Thr Tyr Asp Ile Ser Ser Pro Ile Gln
225                 230                 235                 240

ccc cat cat ttc aat tct tac caa tca atc ttt aac cac cag gtt ttt 948
Pro His His Phe Asn Ser Tyr Gln Ser Ile Phe Asn His Gln Val Phe
                245                 250                 255

ggt tct gct tcg ggc tct acg tac aac aac aac aac gag atg atc aag 996
Gly Ser Ala Ser Gly Ser Thr Tyr Asn Asn Asn Asn Glu Met Ile Lys
            260                 265                 270

atg gag caa tca ctt gtt agt gta tct caa gaa aca tgc cta agc tca 1044
Met Glu Gln Ser Leu Val Ser Val Ser Gln Glu Thr Cys Leu Ser Ser
        275                 280                 285

gat gtg aac gcg aac atg act aca acc acg gag gta tct tcg ggt cct 1092
Asp Val Asn Ala Asn Met Thr Thr Thr Thr Glu Val Ser Ser Gly Pro
    290                 295                 300

gta atg aaa caa gaa atg ggg atg atg gga atg gtg aat ggt agc aag 1140
Val Met Lys Gln Glu Met Gly Met Met Gly Met Val Asn Gly Ser Lys
305                 310                 315                 320

tcg tat gaa gat cta tgt gac ttg agg ggg gac ttg tgg gac ttc taa 1188
Ser Tyr Glu Asp Leu Cys Asp Leu Arg Gly Asp Leu Trp Asp Phe
                325                 330                 335

ttaatcattt gactgtggtg aaagagtata tttgttggga tttaaatcat gttagttaat 1248

acatatacat ataggattta ctagaggctt aatcctagtt aactattttc acttcattga 1308

tattatttaa ttagttgatt gtttaattag tttatacttt atagtgtggt taaaaaagaa 1368

aagaaaggat tgtgataatt tgggatttta gtgcataagt tatatctcaa tgtaaactgt 1428

atttgtatcc aaaaaaaaaa aaaaaaaaaa aaaaaaaaaa aaaaaaaaaa aaa 1481

<210> 20

<211> 335

<212> PRT

<213> Arabidopsis thaliana

<400> 20

Met Ala Val Val Val Glu Glu Gly Val Val Leu Asn His Gly Gly Glu
1               5                   10                  15

Glu Leu Val Asp Leu Pro Pro Gly Phe Arg Phe His Pro Thr Asp Glu
            20                  25                  30

Glu Ile Ile Thr Cys Tyr Leu Lys Glu Lys Val Leu Asn Ser Arg Phe
        35                  40                  45

Thr Ala Val Ala Met Gly Glu Ala Asp Leu Asn Lys Cys Glu Pro Trp
    50                  55                  60

Asp Leu Pro Lys Arg Ala Lys Met Gly Glu Lys Glu Phe Tyr Phe Phe
65                  70                  75                  80

Cys Gln Arg Asp Arg Lys Tyr Pro Thr Gly Met Arg Thr Asn Arg Ala
                85                  90                  95

Thr Glu Ser Gly Tyr Trp Lys Ala Thr Gly Lys Asp Lys Glu Ile Phe
            100                 105                 110

Lys Gly Lys Gly Cys Leu Val Gly Met Lys Lys Thr Leu Val Phe Tyr
        115                 120                 125

Arg Gly Arg Ala Pro Lys Gly Glu Lys Thr Asn Trp Val Met His Glu
    130                 135                 140

Tyr Arg Leu Glu Gly Lys Tyr Ser Tyr Tyr Asn Leu Pro Lys Ser Ala
145                 150                 155                 160

Arg Asp Glu Trp Val Val Cys Arg Val Phe His Lys Asn Asn Pro Ser
                165                 170                 175

Thr Thr Thr Gln Pro Met Thr Arg Ile Pro Val Glu Asp Phe Thr Arg
            180                 185                 190

Met Asp Ser Leu Glu Asn Ile Asp His Leu Leu Asp Phe Ser Ser Leu
        195                 200                 205

Pro Pro Leu Ile Asp Pro Ser Phe Met Ser Gln Thr Glu Gln Pro Asn
    210                 215                 220

Phe Lys Pro Ile Asn Pro Pro Thr Tyr Asp Ile Ser Ser Pro Ile Gln
225                 230                 235                 240

Pro His His Phe Asn Ser Tyr Gln Ser Ile Phe Asn His Gln Val Phe
                245                 250                 255

Gly Ser Ala Ser Gly Ser Thr Tyr Asn Asn Asn Asn Glu Met Ile Lys
            260                 265                 270

Met Glu Gln Ser Leu Val Ser Val Ser Gln Glu Thr Cys Leu Ser Ser
        275                 280                 285

Asp Val Asn Ala Asn Met Thr Thr Thr Thr Glu Val Ser Ser Gly Pro
    290                 295                 300

Val Met Lys Gln Glu Met Gly Met Met Gly Met Val Asn Gly Ser Lys
305                 310                 315                 320

Ser Tyr Glu Asp Leu Cys Asp Leu Arg Gly Asp Leu Trp Asp Phe
                325                 330                 335

<210>  21

<211>  890

<212>  DNA

<213>  Arabidopsis thaliana

<220>

<221>  CDS

<222>  (55)..(738)

<223>  G545

<400>  21
gcaaccttca aactaaaact cgagagacaa gaaatcctca gaatctttaa ctta atg      57
                                                            Met
                                                            1

gcg ctc gag gct ctt aca tca cca aga tta gct tct ccg att cct cct      105
Ala Leu Glu Ala Leu Thr Ser Pro Arg Leu Ala Ser Pro Ile Pro Pro
            5                   10                  15

ttg ttc gaa gat tct tca gtc ttc cat gga gtc gag cac tgg aca aag      153
Leu Phe Glu Asp Ser Ser Val Phe His Gly Val Glu His Trp Thr Lys
        20                  25                  30

ggt aag cga tct aag aga tca aga tcc gat ttc cac cac caa aac ctc      201
Gly Lys Arg Ser Lys Arg Ser Arg Ser Asp Phe His His Gln Asn Leu
    35                  40                  45

act gag gaa gag tat cta gct ttt tgc ctc atg ctt ctc gct cgc gac      249

Thr Glu Glu Glu Tyr Leu Ala Phe Cys Leu Met Leu Leu Ala Arg Asp
50                  55                  60                  65

aac cgt cag cct cct cct cct ccg gcg gtg gag aag ttg agc tac aag      297
Asn Arg Gln Pro Pro Pro Pro Pro Ala Val Glu Lys Leu Ser Tyr Lys
                70                  75                  80

tgt agc gtc tgc gac aag acg ttc tct tct tac caa gct ctc ggt ggt      345
Cys Ser Val Cys Asp Lys Thr Phe Ser Ser Tyr Gln Ala Leu Gly Gly
            85                  90                  95

cac aag gca agc cac cgt aag aac tta tca cag act ctc tcc ggc gga      393
His Lys Ala Ser His Arg Lys Asn Leu Ser Gln Thr Leu Ser Gly Gly
        100                 105                 110

gga gat gat cat tca acc tcg tcg gcg aca acc aca tcc gcc gtg act      441
Gly Asp Asp His Ser Thr Ser Ser Ala Thr Thr Thr Ser Ala Val Thr
    115                 120                 125

act gga agt ggg aaa tca cac gtt tgc acc atc tgt aac aag tct ttt      489
Thr Gly Ser Gly Lys Ser His Val Cys Thr Ile Cys Asn Lys Ser Phe
130                 135                 140                 145

cct tcc ggt caa gct ctc ggc gga cac aag cgg tgc cac tac gaa gga      537
Pro Ser Gly Gln Ala Leu Gly Gly His Lys Arg Cys His Tyr Glu Gly
                150                 155                 160

aac aac aac atc aac act agt agc gtg tcc aac tcc gaa ggt gcg ggg      585
Asn Asn Asn Ile Asn Thr Ser Ser Val Ser Asn Ser Glu Gly Ala Gly
            165                 170                 175

tcc act agc cac gtt agc agt agc cac cgt ggg ttt gac ctc aac atc      633
Ser Thr Ser His Val Ser Ser Ser His Arg Gly Phe Asp Leu Asn Ile
        180                 185                 190

cct ccg atc cct gaa ttc tcg atg gtc aac gga gac gac gaa gtc atg      681
Pro Pro Ile Pro Glu Phe Ser Met Val Asn Gly Asp Asp Glu Val Met
    195                 200                 205

agc cct atg ccg gcg aag aag cct cgg ttt gac ttt ccg gtc aaa ctt      729
Ser Pro Met Pro Ala Lys Lys Pro Arg Phe Asp Phe Pro Val Lys Leu
210                 215                 220                 225

caa ctt taa ggaaatttac ttagacgata agatttcgtt tgtatactgt              778
Gln Leu

tgagagttgt gtaggaattt gttgactgta cataccaaat tggactttga ctgattccaa    838

ttcttcttgt tctttcattt taaaaattat taaaccgatt ctttaccaca aa            890

<210>  22

<211>  227

<212>  PRT

<213>  Arabidopsis thaliana

<400>  22

Met Ala Leu Glu Ala Leu Thr Ser Pro Arg Leu Ala Ser Pro Ile Pro
1               5                   10                  15

Pro Leu Phe Glu Asp Ser Ser Val Phe His Gly Val Glu His Trp Thr
            20                  25                  30

Lys Gly Lys Arg Ser Lys Arg Ser Arg Ser Asp Phe His His Gln Asn
        35                  40                  45

Leu Thr Glu Glu Glu Tyr Leu Ala Phe Cys Leu Met Leu Leu Ala Arg
    50                  55                  60

Asp Asn Arg Gln Pro Pro Pro Pro Pro Ala Val Glu Lys Leu Ser Tyr
65                  70                  75                  80

Lys Cys Ser Val Cys Asp Lys Thr Phe Ser Ser Tyr Gln Ala Leu Gly
                85                  90                  95

Gly His Lys Ala Ser His Arg Lys Asn Leu Ser Gln Thr Leu Ser Gly
            100                 105                 110

Gly Gly Asp Asp His Ser Thr Ser Ser Ala Thr Thr Thr Ser Ala Val
        115                 120                 125

Thr Thr Gly Ser Gly Lys Ser His Val Cys Thr Ile Cys Asn Lys Ser
    130                 135                 140

Phe Pro Ser Gly Gln Ala Leu Gly Gly His Lys Arg Cys His Tyr Glu
145                 150                 155                 160

Gly Asn Asn Asn Ile Asn Thr Ser Ser Val Ser Asn Ser Glu Gly Ala
                165                 170                 175

Gly Ser Thr Ser His Val Ser Ser Ser His Arg Gly Phe Asp Leu Asn
            180                 185                 190

Ile Pro Pro Ile Pro Glu Phe Ser Met Val Asn Gly Asp Asp Glu Val
        195                 200                 205

Met Ser Pro Met Pro Ala Lys Lys Pro Arg Phe Asp Phe Pro Val Lys
    210                 215                 220

Leu Gln Leu
225

<210>  23

<211>  1413

<212>  DNA

<213>  Arabidopsis thaliana


<220>

<221>  CDS

<222>  (86)..(1168)

<223>  G561

<400>  23
aatttgtttt tttttctttt gtgggttcaa ttcgaattgt tttccctgag actcaagtta      60

ctgtgtcatt actctgcatt gagca atg ggt agc aac gaa gaa gga aac ccc      112
                            Met Gly Ser Asn Glu Glu Gly Asn Pro
                            1               5

act aac aac tct gat aag cca tcg caa gct gct gct cct gag cag agt      160
Thr Asn Asn Ser Asp Lys Pro Ser Gln Ala Ala Ala Pro Glu Gln Ser
10                  15                  20                  25

aat gtt cat gtg tat cat cat gac tgg gct gct atg cag gca tat tat      208
Asn Val His Val Tyr His His Asp Trp Ala Ala Met Gln Ala Tyr Tyr
                30                  35                  40

ggg cct aga gtt ggt ata cct caa tat tac aac tca aat ttg gcg cct      256
Gly Pro Arg Val Gly Ile Pro Gln Tyr Tyr Asn Ser Asn Leu Ala Pro
            45                  50                  55

ggt cat gct cca ccg cct tat atg tgg gcg tct cca tcg cca atg atg      304
Gly His Ala Pro Pro Pro Tyr Met Trp Ala Ser Pro Ser Pro Met Met
        60                  65                  70

gct cct tat gga gca cca tat cca cca ttt tgc cct cct ggt gga gtt      352
Ala Pro Tyr Gly Ala Pro Tyr Pro Pro Phe Cys Pro Pro Gly Gly Val
    75                  80                  85

tat gct cat cct ggt gtt caa atg ggc tca caa cca caa ggt cct gtt      400
Tyr Ala His Pro Gly Val Gln Met Gly Ser Gln Pro Gln Gly Pro Val
90                  95                  100                 105

tct caa tca gca tct gga gtt aca acc cct ttg acc att gat gca cca      448
Ser Gln Ser Ala Ser Gly Val Thr Thr Pro Leu Thr Ile Asp Ala Pro
                110                 115                 120

gct aat tca gct gga aac tca gat cat ggg ttc atg aaa aag ctg aaa      496
Ala Asn Ser Ala Gly Asn Ser Asp His Gly Phe Met Lys Lys Leu Lys
            125                 130                 135

gag ttc gat gga ctt gca atg tca ata agc aat aac aaa gtt ggg agt      544
Glu Phe Asp Gly Leu Ala Met Ser Ile Ser Asn Asn Lys Val Gly Ser
        140                 145                 150

gct gaa cat agc agc agt gaa cat agg agt tct cag agc tcc gag aat      592
Ala Glu His Ser Ser Ser Glu His Arg Ser Ser Gln Ser Ser Glu Asn
    155                 160                 165

gat ggc tct agc aat ggt agt gat ggt aat aca act ggg gga gaa caa      640
Asp Gly Ser Ser Asn Gly Ser Asp Gly Asn Thr Thr Gly Gly Glu Gln
170                 175                 180                 185

tct agg agg aaa aga agg caa caa aga tca cca agc act ggt gaa aga      688
Ser Arg Arg Lys Arg Arg Gln Gln Arg Ser Pro Ser Thr Gly Glu Arg
                190                 195                 200

ccc tca tct caa aac agt ctg cct ctt aga ggt gaa aat gag aaa ccc      736
Pro Ser Ser Gln Asn Ser Leu Pro Leu Arg Gly Glu Asn Glu Lys Pro
            205                 210                 215

gat gtg act atg ggg act cct gtt atg ccc aca gca atg agt ttc caa          784
Asp Val Thr Met Gly Thr Pro Val Met Pro Thr Ala Met Ser Phe Gln
        220                 225                 230

aac tct gct ggc atg aac ggt gtg cca cag cca tgg aat gaa aaa gag          832
Asn Ser Ala Gly Met Asn Gly Val Pro Gln Pro Trp Asn Glu Lys Glu
    235                 240                 245

gtt aaa cga gag aag aga aaa cag tca aac cga gaa tct gct agg agg          880
Val Lys Arg Glu Lys Arg Lys Gln Ser Asn Arg Glu Ser Ala Arg Arg
250                 255                 260                 265

tca aga ctg agg aag cag gct gaa aca gaa caa cta tct gtc aaa gtt          928
Ser Arg Leu Arg Lys Gln Ala Glu Thr Glu Gln Leu Ser Val Lys Val
                270                 275                 280

gac gca tta gta gct gag aac atg tct ctg agg tct aaa cta ggc cag          976
Asp Ala Leu Val Ala Glu Asn Met Ser Leu Arg Ser Lys Leu Gly Gln
            285                 290                 295

cta aac aat gag tct gag aaa cta cgg ctg gag aac gaa gct ata ttg          1024
Leu Asn Asn Glu Ser Glu Lys Leu Arg Leu Glu Asn Glu Ala Ile Leu
        300                 305                 310

gat caa ctg aaa gcg caa gca aca ggg aaa aca gag aac ctg atc tct          1072
Asp Gln Leu Lys Ala Gln Ala Thr Gly Lys Thr Glu Asn Leu Ile Ser
    315                 320                 325

cga gtt gat aag aac aac tct gta tca ggt agc aaa act gtg cag cat          1120
Arg Val Asp Lys Asn Asn Ser Val Ser Gly Ser Lys Thr Val Gln His
330                 335                 340                 345

caa ctg tta aat gca agt ccg ata acc gat cct gtc gcg gct agc tga          1168
Gln Leu Leu Asn Ala Ser Pro Ile Thr Asp Pro Val Ala Ala Ser
                350                 355                 360

ccgtggccgc aacaatgaga acccgatatt tcttcctttg ggttgtgatt gtaacttaaa          1228

aggagacttt ttgtttttat tcttagattt gtagctctct gcatagtgag cataaattga          1288

tgtaatatgg tttaagagat tcggtgttct ctggtgtgtg ctgcaaccac ataattggtg          1348

atagataggt ttagttatat aagcaaatgt attagagata aggggagaca tatttgatgg          1408

tcttt                                                                      1413

<210>  24

<211>  360

<212>  PRT

<213>  Arabidopsis thaliana


<400>  24

Met Gly Ser Asn Glu Glu Gly Asn Pro Thr Asn Asn Ser Asp Lys Pro
1               5                   10                  15


Ser Gln Ala Ala Ala Pro Glu Gln Ser Asn Val His Val Tyr His His
            20                  25                  30

Asp Trp Ala Ala Met Gln Ala Tyr Tyr Gly Pro Arg Val Gly Ile Pro
        35                  40                  45

Gln Tyr Tyr Asn Ser Asn Leu Ala Pro Gly His Ala Pro Pro Pro Tyr
    50                  55                  60

Met Trp Ala Ser Pro Ser Pro Met Met Ala Pro Tyr Gly Ala Pro Tyr
65                  70                  75                  80

Pro Pro Phe Cys Pro Pro Gly Gly Val Tyr Ala His Pro Gly Val Gln
                85                  90                  95

Met Gly Ser Gln Pro Gln Gly Pro Val Ser Gln Ser Ala Ser Gly Val
            100                 105                 110

Thr Thr Pro Leu Thr Ile Asp Ala Pro Ala Asn Ser Ala Gly Asn Ser
        115                 120                 125

Asp His Gly Phe Met Lys Lys Leu Lys Glu Phe Asp Gly Leu Ala Met
    130                 135                 140

Ser Ile Ser Asn Asn Lys Val Gly Ser Ala Glu His Ser Ser Ser Glu
145                 150                 155                 160

His Arg Ser Ser Gln Ser Ser Glu Asn Asp Gly Ser Ser Asn Gly Ser
                165                 170                 175

Asp Gly Asn Thr Thr Gly Gly Glu Gln Ser Arg Arg Lys Arg Arg Gln
            180                 185                 190

Gln Arg Ser Pro Ser Thr Gly Glu Arg Pro Ser Ser Gln Asn Ser Leu
        195                 200                 205

Pro Leu Arg Gly Glu Asn Glu Lys Pro Asp Val Thr Met Gly Thr Pro
    210                 215                 220

Val Met Pro Thr Ala Met Ser Phe Gln Asn Ser Ala Gly Met Asn Gly
225                 230                 235                 240

Val Pro Gln Pro Trp Asn Glu Lys Glu Val Lys Arg Glu Lys Arg Lys
                245                 250                 255

Gln Ser Asn Arg Glu Ser Ala Arg Arg Ser Arg Leu Arg Lys Gln Ala
            260                 265                 270

Glu Thr Glu Gln Leu Ser Val Lys Val Asp Ala Leu Val Ala Glu Asn
        275                 280                 285

Met Ser Leu Arg Ser Lys Leu Gly Gln Leu Asn Asn Glu Ser Glu Lys
    290                 295                 300

Leu Arg Leu Glu Asn Glu Ala Ile Leu Asp Gln Leu Lys Ala Gln Ala
305                 310                 315                 320

Thr Gly Lys Thr Glu Asn Leu Ile Ser Arg Val Asp Lys Asn Asn Ser
                325                 330                 335

Val Ser Gly Ser Lys Thr Val Gln His Gln Leu Leu Asn Ala Ser Pro
            340                 345                 350

Ile Thr Asp Pro Val Ala Ala Ser
        355                 360

<210>  25

<211>  1087

<212>  DNA

<213>  Arabidopsis thaliana


<220>

<221>  CDS

<222>  (104)..(952)

<223>  G664



<400>  25
caatccctca atataaaata acaagtagaa ttgatctgcc tatatataag attttgagac      60

gaaataagat ctaaaccaca agaaagaaag taaacataaa agt atg gga agg tca       115
                                                Met Gly Arg Ser
                                                1

ccg tgc tgt gag aaa gct cac aca aac aaa gga gca tgg acg aaa gaa       163
Pro Cys Cys Glu Lys Ala His Thr Asn Lys Gly Ala Trp Thr Lys Glu
5                   10                  15                  20

gag gac gag agg ctc gtc gcc tac att aaa gct cat gga gaa ggc tgc       211
Glu Asp Glu Arg Leu Val Ala Tyr Ile Lys Ala His Gly Glu Gly Cys
                25                  30                  35

tgg aga tct ctc ccc aaa gcc gcc gga ctt ctt cgc tgt ggc aag agc       259
Trp Arg Ser Leu Pro Lys Ala Ala Gly Leu Leu Arg Cys Gly Lys Ser
            40                  45                  50

tgc cgt ctc cgg tgg atc aac tat ctc cgg cct gac ctt aag cgt gga       307
Cys Arg Leu Arg Trp Ile Asn Tyr Leu Arg Pro Asp Leu Lys Arg Gly
        55                  60                  65

aac ttc acc gag gaa gaa gac gaa ctc atc atc aag ctc cat agc ctt       355
Asn Phe Thr Glu Glu Glu Asp Glu Leu Ile Ile Lys Leu His Ser Leu
    70                  75                  80

ctt ggc aac aaa tgg tcg ctt att gcc ggg aga tta ccg gga aga aca       403
Leu Gly Asn Lys Trp Ser Leu Ile Ala Gly Arg Leu Pro Gly Arg Thr
85                  90                  95                  100

gat aac gag ata aag aac tat tgg aac acg cat ata cga aga aag ctt     451
Asp Asn Glu Ile Lys Asn Tyr Trp Asn Thr His Ile Arg Arg Lys Leu
                105                 110                 115

ata aac aga ggg att gat cca acg agt cat aga cca atc caa gaa tca     499
Ile Asn Arg Gly Ile Asp Pro Thr Ser His Arg Pro Ile Gln Glu Ser
            120                 125                 130

tca gct tct caa gat tct aaa cct aca caa cta gaa cca gtt acg agt     547
Ser Ala Ser Gln Asp Ser Lys Pro Thr Gln Leu Glu Pro Val Thr Ser
        135                 140                 145

aat acc att aat atc tca ttc act tct gct cca aag gtc gaa acg ttc     595
Asn Thr Ile Asn Ile Ser Phe Thr Ser Ala Pro Lys Val Glu Thr Phe
    150                 155                 160

cat gaa agt ata agc ttt ccg gga aaa tca gag aaa atc tca atg ctt     643
His Glu Ser Ile Ser Phe Pro Gly Lys Ser Glu Lys Ile Ser Met Leu
165                 170                 175                 180

acg ttc aaa gaa gaa aaa gat gag tgc cca gtt caa gaa aag ttc cca     691
Thr Phe Lys Glu Glu Lys Asp Glu Cys Pro Val Gln Glu Lys Phe Pro
                185                 190                 195

gat ttg aat ctt gag ctc aga atc agt ctt cct gat gat gtt gat cgt     739
Asp Leu Asn Leu Glu Leu Arg Ile Ser Leu Pro Asp Asp Val Asp Arg
            200                 205                 210

ctt caa ggg cat gga aag tca aca acg cca cgt tgt ttc aag tgc agc     787
Leu Gln Gly His Gly Lys Ser Thr Thr Pro Arg Cys Phe Lys Cys Ser
        215                 220                 225

tta ggg atg ata aac ggc atg gag tgc aga tgc gga aga atg aga tgc     835
Leu Gly Met Ile Asn Gly Met Glu Cys Arg Cys Gly Arg Met Arg Cys
    230                 235                 240

gat gta gtc gga ggt agc agc aag ggg agt gac atg agc aat gga ttt     883
Asp Val Val Gly Gly Ser Ser Lys Gly Ser Asp Met Ser Asn Gly Phe
245                 250                 255                 260

gat ttt tta ggg ttg gca aag aaa gag acc act tct ctt ttg ggc ttt     931
Asp Phe Leu Gly Leu Ala Lys Lys Glu Thr Thr Ser Leu Leu Gly Phe
                265                 270                 275

cga agc ttg gag atg aaa taa tattgtcaaa ttttaggcgt aactgtacaa     982
Arg Ser Leu Glu Met Lys
            280

aacttttgcc tagataattt gaaagtatat cttcaacttg tatgagaaat ttaactggtg    1042

aattataata tatagaattt gtttttaaa aaaaaaaaa aaaaa                      1087

<210>  26

<211>  282

<212>  PRT

<213>  Arabidopsis thaliana


<400>  26

Met Gly Arg Ser Pro Cys Cys Glu Lys Ala His Thr Asn Lys Gly Ala
1               5                   10                  15

Trp Thr Lys Glu Glu Asp Glu Arg Leu Val Ala Tyr Ile Lys Ala His
            20                  25                  30

Gly Glu Gly Cys Trp Arg Ser Leu Pro Lys Ala Ala Gly Leu Leu Arg
        35                  40                  45

Cys Gly Lys Ser Cys Arg Leu Arg Trp Ile Asn Tyr Leu Arg Pro Asp
    50                  55                  60

Leu Lys Arg Gly Asn Phe Thr Glu Glu Glu Asp Glu Leu Ile Ile Lys
65                  70                  75                  80

Leu His Ser Leu Leu Gly Asn Lys Trp Ser Leu Ile Ala Gly Arg Leu
                85                  90                  95

Pro Gly Arg Thr Asp Asn Glu Ile Lys Asn Tyr Trp Asn Thr His Ile
            100                 105                 110

Arg Arg Lys Leu Ile Asn Arg Gly Ile Asp Pro Thr Ser His Arg Pro
        115                 120                 125

Ile Gln Glu Ser Ser Ala Ser Gln Asp Ser Lys Pro Thr Gln Leu Glu
    130                 135                 140

Pro Val Thr Ser Asn Thr Ile Asn Ile Ser Phe Thr Ser Ala Pro Lys
145                 150                 155                 160

Val Glu Thr Phe His Glu Ser Ile Ser Phe Pro Gly Lys Ser Glu Lys
                165                 170                 175

Ile Ser Met Leu Thr Phe Lys Glu Glu Lys Asp Glu Cys Pro Val Gln
            180                 185                 190

Glu Lys Phe Pro Asp Leu Asn Leu Glu Leu Arg Ile Ser Leu Pro Asp
        195                 200                 205

Asp Val Asp Arg Leu Gln Gly His Gly Lys Ser Thr Thr Pro Arg Cys
    210                 215                 220

Phe Lys Cys Ser Leu Gly Met Ile Asn Gly Met Glu Cys Arg Cys Gly
225                 230                 235                 240

Arg Met Arg Cys Asp Val Val Gly Gly Ser Ser Lys Gly Ser Asp Met
                245                 250                 255

Ser Asn Gly Phe Asp Phe Leu Gly Leu Ala Lys Lys Glu Thr Thr Ser
            260                 265                 270

Leu Leu Gly Phe Arg Ser Leu Glu Met Lys
        275                 280

<210> 27

<211> 228

<212> DNA

<213> Arabidopsis thaliana

<220>

<221> CDS

<222> (1)..(228)

<223> G682

<400> 27
atg gat aac cat cgc agg act aag caa ccc aag acc aac tcc atc gtt 48
Met Asp Asn His Arg Arg Thr Lys Gln Pro Lys Thr Asn Ser Ile Val
1               5                   10                  15

act tct tct tct gaa gaa gtg agt agt ctt gag tgg gaa gtt gtg aac 96
Thr Ser Ser Ser Glu Glu Val Ser Ser Leu Glu Trp Glu Val Val Asn
            20                  25                  30

atg agt caa gaa gaa gaa gat ttg gtc tct cga atg cat aag ctt gtc 144
Met Ser Gln Glu Glu Glu Asp Leu Val Ser Arg Met His Lys Leu Val
        35                  40                  45

ggt gac agg tgg gaa ctg ata gct ggg agg atc cca gga aga acc gct 192
Gly Asp Arg Trp Glu Leu Ile Ala Gly Arg Ile Pro Gly Arg Thr Ala
    50                  55                  60

gga gaa att gag agg ttt tgg gtc atg aaa aat tga 228
Gly Glu Ile Glu Arg Phe Trp Val Met Lys Asn
65                  70                  75

<210> 28

<211> 75

<212> PRT

<213> Arabidopsis thaliana

<400> 28

Met Asp Asn His Arg Arg Thr Lys Gln Pro Lys Thr Asn Ser Ile Val
1               5                   10                  15

Thr Ser Ser Ser Glu Glu Val Ser Ser Leu Glu Trp Glu Val Val Asn
            20                  25                  30

Met Ser Gln Glu Glu Glu Asp Leu Val Ser Arg Met His Lys Leu Val
        35                  40                  45

Gly Asp Arg Trp Glu Leu Ile Ala Gly Arg Ile Pro Gly Arg Thr Ala
    50                  55                  60

Gly Glu Ile Glu Arg Phe Trp Val Met Lys Asn
65                  70                  75

<210>  29

<211>  480

<212>  DNA

<213>  Arabidopsis thaliana

<220>

<221>  CDS

<222>  (1)..(480)

<223>  G911

<400>  29
atg ggt ctt cct gaa gat ttc atc acc gag ctt cag att cca ggt tac        48
Met Gly Leu Pro Glu Asp Phe Ile Thr Glu Leu Gln Ile Pro Gly Tyr
1               5                   10                  15

ata tta aag ata ctt tac gtc atc ggt ttc ttt aga gac atg gtc gat        96
Ile Leu Lys Ile Leu Tyr Val Ile Gly Phe Phe Arg Asp Met Val Asp
            20                  25                  30

gct ctt tgt cct tac att ggt cta cct agt ttt cta gac cac aac gag       144
Ala Leu Cys Pro Tyr Ile Gly Leu Pro Ser Phe Leu Asp His Asn Glu
        35                  40                  45

acc tct gga ccc gat ccg acc cga cac gct ctc tct acg tca gcg agt       192
Thr Ser Gly Pro Asp Pro Thr Arg His Ala Leu Ser Thr Ser Ala Ser
    50                  55                  60

ctt gct aac gag ttg atc ccg gtg gtt cgg ttc tcg gat ctt ccg acc       240
Leu Ala Asn Glu Leu Ile Pro Val Val Arg Phe Ser Asp Leu Pro Thr
65                  70                  75                  80

gat ccg gaa gat tgt tgt acg gtt tgt ttg tca gat ttt gag tcc gac       288
Asp Pro Glu Asp Cys Cys Thr Val Cys Leu Ser Asp Phe Glu Ser Asp
                85                  90                  95

gat aag gtt agg cag cta ccc aag tgt gga cac gtg ttt cat cat cat       336
Asp Lys Val Arg Gln Leu Pro Lys Cys Gly His Val Phe His His His
            100                 105                 110

tgt tta gac cgt tgg atc gtt gac tac aac aag atg aaa tgt ccg gtt       384
Cys Leu Asp Arg Trp Ile Val Asp Tyr Asn Lys Met Lys Cys Pro Val
        115                 120                 125

tgt cgg cac cgg ttc tta ccg aaa gaa aag tac acg caa tgt gat tgg       432
Cys Arg His Arg Phe Leu Pro Lys Glu Lys Tyr Thr Gln Cys Asp Trp
    130                 135                 140

ggt tct ggt tca gat tgg ttt agt gat gaa gtg gaa agt acc aac taa          480
Gly Ser Gly Ser Asp Trp Phe Ser Asp Glu Val Glu Ser Thr Asn
145                 150                 155

<210>  30

<211>  159

<212>  PRT

<213>  Arabidopsis thaliana

<400>  30

Met Gly Leu Pro Glu Asp Phe Ile Thr Glu Leu Gln Ile Pro Gly Tyr
1               5                   10                  15

Ile Leu Lys Ile Leu Tyr Val Ile Gly Phe Phe Arg Asp Met Val Asp
            20                  25                  30

Ala Leu Cys Pro Tyr Ile Gly Leu Pro Ser Phe Leu Asp His Asn Glu
        35                  40                  45

Thr Ser Gly Pro Asp Pro Thr Arg His Ala Leu Ser Thr Ser Ala Ser
    50                  55                  60

Leu Ala Asn Glu Leu Ile Pro Val Val Arg Phe Ser Asp Leu Pro Thr
65                  70                  75                  80

Asp Pro Glu Asp Cys Cys Thr Val Cys Leu Ser Asp Phe Glu Ser Asp
                85                  90                  95

Asp Lys Val Arg Gln Leu Pro Lys Cys Gly His Val Phe His His His
            100                 105                 110

Cys Leu Asp Arg Trp Ile Val Asp Tyr Asn Lys Met Lys Cys Pro Val
        115                 120                 125

Cys Arg His Arg Phe Leu Pro Lys Glu Lys Tyr Thr Gln Cys Asp Trp
    130                 135                 140

Gly Ser Gly Ser Asp Trp Phe Ser Asp Glu Val Glu Ser Thr Asn
145                 150                 155

<210>  31

<211>  1221

<212>  DNA

<213>  Arabidopsis thaliana

<220>

<221> CDS

<222> (162)..(1013)

<223> G964

<400> 31
atttctcttc cacaaagagt cctaacttcg agttgaaaca aacaccattt ctcatctcta 60

tctcagaaag aacaaaccat ttcgtgttct ttctttctct attctcataa ggaaatataa 120

ttcctgaaac tgttgagttc ttgtgaaagg aaataaaaaa c atg atg atg ggc aaa 176
Met Met Met Gly Lys
1               5

gaa gat cta ggt ttg agc cta agc tta ggg ttt tca caa aat cac aat 224
Glu Asp Leu Gly Leu Ser Leu Ser Leu Gly Phe Ser Gln Asn His Asn
                10                  15                  20

cct ctt cag atg aat ctg aat cct aac tct tca tta tca aac aat ctc 272
Pro Leu Gln Met Asn Leu Asn Pro Asn Ser Ser Leu Ser Asn Asn Leu
            25                  30                  35

cag aga ctc cca tgg aac caa aca ttc gat cct aca tca gat ctt cgc 320
Gln Arg Leu Pro Trp Asn Gln Thr Phe Asp Pro Thr Ser Asp Leu Arg
        40                  45                  50

aag ata gac gtg aac agt ttt cca tca acg gtt aac tgc gag gaa gac 368
Lys Ile Asp Val Asn Ser Phe Pro Ser Thr Val Asn Cys Glu Glu Asp
    55                  60                  65

aca gga gtt tcg tca cca aac agt acg atc tca agc acc att agc ggg 416
Thr Gly Val Ser Ser Pro Asn Ser Thr Ile Ser Ser Thr Ile Ser Gly
70                  75                  80                  85

aag aga agt gag aga gaa gga atc tcc gga acc ggc gtt ggc tcc ggc 464
Lys Arg Ser Glu Arg Glu Gly Ile Ser Gly Thr Gly Val Gly Ser Gly
                90                  95                  100

gac gat cac gac gag atc act ccg gat cga ggg tac tca cgt gga acc 512
Asp Asp His Asp Glu Ile Thr Pro Asp Arg Gly Tyr Ser Arg Gly Thr
            105                 110                 115

tca gat gaa gaa gaa gac ggg ggc gaa acg tcg agg aag aag ctc agg 560
Ser Asp Glu Glu Glu Asp Gly Gly Glu Thr Ser Arg Lys Lys Leu Arg
        120                 125                 130

tta tca aaa gat cag tct gct ttt ctc gaa gag act ttc aaa gaa cac 608
Leu Ser Lys Asp Gln Ser Ala Phe Leu Glu Glu Thr Phe Lys Glu His
    135                 140                 145

aac act ctc aat ccc aaa cag aag cta gct ttg gct aag aag ctg aac 656
Asn Thr Leu Asn Pro Lys Gln Lys Leu Ala Leu Ala Lys Lys Leu Asn
150                 155                 160                 165

ttg acg gca aga caa gtg gaa gtg tgg ttc caa aac aga aga gct aga 704
Leu Thr Ala Arg Gln Val Glu Val Trp Phe Gln Asn Arg Arg Ala Arg
                170                 175                 180

acc aag tta aag caa acg gag gta gat tgc gaa tac ttg aaa cgg tgc 752
Thr Lys Leu Lys Gln Thr Glu Val Asp Cys Glu Tyr Leu Lys Arg Cys
            185                 190                 195

gta gag aag cta acg gaa gag aac cgg aga ctt cag aaa gag gct atg 800
Val Glu Lys Leu Thr Glu Glu Asn Arg Arg Leu Gln Lys Glu Ala Met
        200                 205                 210

gag ctt cga act ctc aag ctg tct cca caa ttc tac ggt cag atg act 848
Glu Leu Arg Thr Leu Lys Leu Ser Pro Gln Phe Tyr Gly Gln Met Thr
    215                 220                 225

cca cca act aca ctc atc atg tgt cct tcg tgc gag cgt gta gct ggt 896
Pro Pro Thr Thr Leu Ile Met Cys Pro Ser Cys Glu Arg Val Ala Gly
230                 235                 240                 245

cca tca tca tcg aac cat cac cac aat cac agg ccg gtt tcg att aac 944
Pro Ser Ser Ser Asn His His His Asn His Arg Pro Val Ser Ile Asn
                250                 255                 260

ccg tgg att gct tgt gct ggt cag gtg gct cat ggg ctg aat ttt gaa 992
Pro Trp Ile Ala Cys Ala Gly Gln Val Ala His Gly Leu Asn Phe Glu
            265                 270                 275

gcc ttg cgt cca cga tcg taa ttttagtgg tgggggaagg gtgttttggg 1043
Ala Leu Arg Pro Arg Ser
        280

ttttttcatt atcgttatat agtctatctg tgtggggtca ttgtaatttt ggatgattgg 1103

ccttctcatg aactagtcat atgtatgatg caaccttaaa aatatttcaa gtagcaaaac 1163

ttaattacaa acttgctata ttaaccaaaa attatgaaaa aaaaaaaaaa aaaaaaa 1221

<210> 32

<211> 283

<212> PRT

<213> Arabidopsis thaliana

<400> 32

Met Met Met Gly Lys Glu Asp Leu Gly Leu Ser Leu Ser Leu Gly Phe
1               5                   10                  15

Ser Gln Asn His Asn Pro Leu Gln Met Asn Leu Asn Pro Asn Ser Ser
            20                  25                  30

Leu Ser Asn Asn Leu Gln Arg Leu Pro Trp Asn Gln Thr Phe Asp Pro
        35                  40                  45

Thr Ser Asp Leu Arg Lys Ile Asp Val Asn Ser Phe Pro Ser Thr Val
    50                  55                  60

Asn Cys Glu Glu Asp Thr Gly Val Ser Ser Pro Asn Ser Thr Ile Ser
65                  70                  75                  80

Ser Thr Ile Ser Gly Lys Arg Ser Glu Arg Glu Gly Ile Ser Gly Thr
                85                  90                  95

Gly Val Gly Ser Gly Asp Asp His Asp Glu Ile Thr Pro Asp Arg Gly
            100                 105                 110

Tyr Ser Arg Gly Thr Ser Asp Glu Glu Glu Asp Gly Gly Glu Thr Ser
        115                 120                 125

Arg Lys Lys Leu Arg Leu Ser Lys Asp Gln Ser Ala Phe Leu Glu Glu
    130                 135                 140

Thr Phe Lys Glu His Asn Thr Leu Asn Pro Lys Gln Lys Leu Ala Leu
145                 150                 155                 160

Ala Lys Lys Leu Asn Leu Thr Ala Arg Gln Val Glu Val Trp Phe Gln
                165                 170                 175

Asn Arg Arg Ala Arg Thr Lys Leu Lys Gln Thr Glu Val Asp Cys Glu
            180                 185                 190

Tyr Leu Lys Arg Cys Val Glu Lys Leu Thr Glu Glu Asn Arg Arg Leu
        195                 200                 205

Gln Lys Glu Ala Met Glu Leu Arg Thr Leu Lys Leu Ser Pro Gln Phe
    210                 215                 220

Tyr Gly Gln Met Thr Pro Pro Thr Thr Leu Ile Met Cys Pro Ser Cys
225                 230                 235                 240

Glu Arg Val Ala Gly Pro Ser Ser Ser Asn His His His Asn His Arg
                245                 250                 255

Pro Val Ser Ile Asn Pro Trp Ile Ala Cys Ala Gly Gln Val Ala His
            260                 265                 270

Gly Leu Asn Phe Glu Ala Leu Arg Pro Arg Ser
        275                 280

<210>  33

<211>  1249

<212>  DNA

<213>  Arabidopsis thaliana


<220>

<221>  CDS

<222>  (82)..(918)

<223>  G394

<400> 33
gaaattctta acaaacaatt ttcttcataa tattaattct caagatctta aagattatat 60

taatacgaag agaaaattca a atg ggt ctt gat gat tca tgc aac aca ggt 111
Met Gly Leu Asp Asp Ser Cys Asn Thr Gly
1               5                   10

ctt gtt ctt ggt tta ggc ctc tca cca acg cct aat aat tac aat cat 159
Leu Val Leu Gly Leu Gly Leu Ser Pro Thr Pro Asn Asn Tyr Asn His
                15                  20                  25

gcc atc aag aaa tct tcc tcc act gtg gac cat cgt ttc atc agg ctc 207
Ala Ile Lys Lys Ser Ser Ser Thr Val Asp His Arg Phe Ile Arg Leu
            30                  35                  40

gat ccg tcg ttg act cta agc cta tcc ggt gag agc tac aag atc aag 255
Asp Pro Ser Leu Thr Leu Ser Leu Ser Gly Glu Ser Tyr Lys Ile Lys
        45                  50                  55

act ggt gcc ggc gcc ggc gac caa att tgc cgg cag acc tcg tcc cac 303
Thr Gly Ala Gly Ala Gly Asp Gln Ile Cys Arg Gln Thr Ser Ser His
    60                  65                  70

agc ggc atc tca tct ttc tcg agc gga agg gta aag aga gaa aga gaa 351
Ser Gly Ile Ser Ser Phe Ser Ser Gly Arg Val Lys Arg Glu Arg Glu
75                  80                  85                  90

atc tcc ggc ggc gat gga gaa gaa gag gcg gag gag acg acg gag aga 399
Ile Ser Gly Gly Asp Gly Glu Glu Glu Ala Glu Glu Thr Thr Glu Arg
                95                  100                 105

gtg gtg tgt tcg aga gtg agt gat gat cat gac gat gaa gaa ggt gtt 447
Val Val Cys Ser Arg Val Ser Asp Asp His Asp Asp Glu Glu Gly Val
            110                 115                 120

agt gct cgt aaa aag ctt aga ctc act aaa caa caa tct gct ctt ctc 495
Ser Ala Arg Lys Lys Leu Arg Leu Thr Lys Gln Gln Ser Ala Leu Leu
        125                 130                 135

gaa gat aac ttc aaa ctt cat agc acc ctt aat ccc aag caa aaa caa 543
Glu Asp Asn Phe Lys Leu His Ser Thr Leu Asn Pro Lys Gln Lys Gln
    140                 145                 150

gct ctt gcg aga cag ctg aat cta agg cct aga caa gtt gaa gtg tgg 591
Ala Leu Ala Arg Gln Leu Asn Leu Arg Pro Arg Gln Val Glu Val Trp
155                 160                 165                 170

ttc caa aac agg aga gct aga aca aaa cta aag caa aca gaa gtg gat 639
Phe Gln Asn Arg Arg Ala Arg Thr Lys Leu Lys Gln Thr Glu Val Asp
                175                 180                 185

tgt gag ttt ttg aag aaa tgt tgc gag act tta acg gat gag aat aga 687
Cys Glu Phe Leu Lys Lys Cys Cys Glu Thr Leu Thr Asp Glu Asn Arg
            190                 195                 200

agg ctt caa aaa gag ctt caa gac ctt aag gct tta aaa ttg tct caa 735
Arg Leu Gln Lys Glu Leu Gln Asp Leu Lys Ala Leu Lys Leu Ser Gln
        205                 210                 215

ccg ttt tac atg cac atg ccg gcg gcg act ttg act atg tgc cct tct 783
Pro Phe Tyr Met His Met Pro Ala Ala Thr Leu Thr Met Cys Pro Ser
    220                 225                 230

tgt gag aga ctc ggc ggt ggt ggt gtc gga gga gat acg acg gcg gtt 831
Cys Glu Arg Leu Gly Gly Gly Gly Val Gly Gly Asp Thr Thr Ala Val
235                 240                 245                 250

gat gaa gaa acg gcg aaa gga gct ttc tcc atc gtc aca aag cct cgt        879
Asp Glu Glu Thr Ala Lys Gly Ala Phe Ser Ile Val Thr Lys Pro Arg
                255                 260                 265

ttc tat aac cct ttc act aat cct tct gca gca tgt tag ttacttatta        928
Phe Tyr Asn Pro Phe Thr Asn Pro Ser Ala Ala Cys
            270                 275

gttatttaat tctttttgtt ggtttttttt ttgtttctta aatcaaatta ggaattagtt     988

agaagataaa tcccagggaa aaaatattac gttgaaattg gggggaaatg gggtatagtc    1048

tttatagata agactcttca acgattccac tttatttttc ggtgggattg ttggttgatg    1108

aagaaaaaaa aatagtttgt aattacaggt ttaaatatgt agagaaaaaa tgacgaatat    1168

gtattatctt gttttttttt ccttcgaata tgtattacgg taatataaat ttgcttgtaa    1228

aaataataaa tatattattt g                                              1249

<210>  34

<211>  278

<212>  PRT

<213>  Arabidopsis thaliana

<400>  34

Met Gly Leu Asp Asp Ser Cys Asn Thr Gly Leu Val Leu Gly Leu Gly
1               5                   10                  15

Leu Ser Pro Thr Pro Asn Asn Tyr Asn His Ala Ile Lys Lys Ser Ser
            20                  25                  30

Ser Thr Val Asp His Arg Phe Ile Arg Leu Asp Pro Ser Leu Thr Leu
        35                  40                  45

Ser Leu Ser Gly Glu Ser Tyr Lys Ile Lys Thr Gly Ala Gly Ala Gly
    50                  55                  60

Asp Gln Ile Cys Arg Gln Thr Ser Ser His Ser Gly Ile Ser Ser Phe
65                  70                  75                  80

Ser Ser Gly Arg Val Lys Arg Glu Arg Glu Ile Ser Gly Gly Asp Gly
                85                  90                  95

Glu Glu Glu Ala Glu Glu Thr Thr Glu Arg Val Val Cys Ser Arg Val
            100                 105                 110

Ser Asp Asp His Asp Asp Glu Glu Gly Val Ser Ala Arg Lys Lys Leu
        115                 120                 125

Arg Leu Thr Lys Gln Gln Ser Ala Leu Leu Glu Asp Asn Phe Lys Leu
    130                 135                 140

His Ser Thr Leu Asn Pro Lys Gln Lys Gln Ala Leu Ala Arg Gln Leu
145                 150                 155                 160

Asn Leu Arg Pro Arg Gln Val Glu Val Trp Phe Gln Asn Arg Arg Ala
                165                 170                 175

Arg Thr Lys Leu Lys Gln Thr Glu Val Asp Cys Glu Phe Leu Lys Lys
            180                 185                 190

Cys Cys Glu Thr Leu Thr Asp Glu Asn Arg Arg Leu Gln Lys Glu Leu
        195                 200                 205

Gln Asp Leu Lys Ala Leu Lys Leu Ser Gln Pro Phe Tyr Met His Met
    210                 215                 220

Pro Ala Ala Thr Leu Thr Met Cys Pro Ser Cys Glu Arg Leu Gly Gly
225                 230                 235                 240

Gly Gly Val Gly Gly Asp Thr Thr Ala Val Asp Glu Glu Thr Ala Lys
                245                 250                 255

Gly Ala Phe Ser Ile Val Thr Lys Pro Arg Phe Tyr Asn Pro Phe Thr
            260                 265                 270

Asn Pro Ser Ala Ala Cys
        275

<210>  35

<211>  1147

<212>  DNA

<213>  Arabidopsis thaliana


<220>

<221>  CDS

<222>  (33)..(695)

<223>  G489



<400>  35
   tggatcaaca agaccatgga cagtctggag ct atg aac tat ggc aca aac cca        53
                                       Met Asn Tyr Gly Thr Asn Pro
                                       1               5
   tac caa acc aac ccg atg agc acc act gct gct act gta gca gga ggt       101
   Tyr Gln Thr Asn Pro Met Ser Thr Thr Ala Ala Thr Val Ala Gly Gly
           10                  15                  20
   gcg gca caa cca ggc cag ctg gcg ttc cac cag atc cat cag cag cag     149
   Ala Ala Gln Pro Gly Gln Leu Ala Phe His Gln Ile His Gln Gln Gln
       25                  30                  35
   cag cag caa cag ctg gca cag cag ctt caa gca ttt tgg gag aac caa     197
   Gln Gln Gln Gln Leu Ala Gln Gln Leu Gln Ala Phe Trp Glu Asn Gln
   40                  45                  50                  55
   ttc aaa gag att gag aag act acc gat ttc aag aac cac agc ctt ccc     245
   Phe Lys Glu Ile Glu Lys Thr Thr Asp Phe Lys Asn His Ser Leu Pro
                   60                  65                  70
   ctt gcg aga atc aag aaa atc atg aaa gcg gat gaa gat gtc cgt atg     293
   Leu Ala Arg Ile Lys Lys Ile Met Lys Ala Asp Glu Asp Val Arg Met
               75                  80                  85
   atc tcg gct gag gcg ccg gtc gtg ttt gca agg gcc tgt gag atg ttc     341
   Ile Ser Ala Glu Ala Pro Val Val Phe Ala Arg Ala Cys Glu Met Phe
           90                  95                  100
   atc ctg gag ctg aca ctc agg tcg tgg aac cac act gag gag aat aag     389
   Ile Leu Glu Leu Thr Leu Arg Ser Trp Asn His Thr Glu Glu Asn Lys
       105                 110                 115
   agg cgg acg ttg cag aag aac gat att gct gct gct gtg act aga acc     437
   Arg Arg Thr Leu Gln Lys Asn Asp Ile Ala Ala Ala Val Thr Arg Thr
   120                 125                 130                 135
   gat att ttt gat ttc ctt gtg gat att gtt ccc cgg gag gat ctc cga     485
   Asp Ile Phe Asp Phe Leu Val Asp Ile Val Pro Arg Glu Asp Leu Arg
                   140                 145                 150
   gat gaa gtc ttg gga agt att ccg agg ggc act gtc ccg gaa gct gct     533
   Asp Glu Val Leu Gly Ser Ile Pro Arg Gly Thr Val Pro Glu Ala Ala
               155                 160                 165
   gct gct ggt tac ccg tat gga tac ttg cct gca gga act gct cca ata     581
   Ala Ala Gly Tyr Pro Tyr Gly Tyr Leu Pro Ala Gly Thr Ala Pro Ile
           170                 175                 180
   gga aat ccg gga atg gtt atg ggt aat ccc ggt ggt gcg tat cca cct     629
   Gly Asn Pro Gly Met Val Met Gly Asn Pro Gly Gly Ala Tyr Pro Pro
       185                 190                 195
   aat cct tat atg ggt caa cca atg tgg caa caa cag gca cct gac caa     677
   Asn Pro Tyr Met Gly Gln Pro Met Trp Gln Gln Gln Ala Pro Asp Gln
   200                 205                 210                 215
   cct gac cag gaa aat tag caagaaactg tgagtcttcc agcttcgcgg     725
   Pro Asp Gln Glu Asn
                   220
   ccgctctaga caggcctcgt accggatcct ctagctagag ctttcgttcg tatcatcggt     785
   ttcgacaacg ttcgtcaagt tcaatgcatc agtttcattg cgcacacacc agaatcctac     845
   tgagtttgag tattatggca ttgggaaaac tgtttttctt gtccatttgt tgtgcttgta     905
   atttactgtg tttttattcg ggttttcgct atcgaactgt gaaatggaaa tggatggaga     965
   agagttaatg aatgatatgg ccttttgttc attctcaaat taatattatt tggtttttct     1025
   cttatttgtg gggatgaatt tgaaattata agagatatgc aaacattttg tttgagtaaa     1085
   atgtgcaaat cgtggcctct aatgacccga agttaatatg aggagtaaaa cacttgtagg     1145
   tg     1147

<210> 36

<211> 220

<212> PRT

<213> Arabidopsis thaliana

<400> 36

Met Asn Tyr Gly Thr Asn Pro Tyr Gln Thr Asn Pro Met Ser Thr Thr
1               5                   10                  15

Ala Ala Thr Val Ala Gly Gly Ala Ala Gln Pro Gly Gln Leu Ala Phe
            20                  25                  30

His Gln Ile His Gln Gln Gln Gln Gln Gln Gln Leu Ala Gln Gln Leu
        35                  40                  45

Gln Ala Phe Trp Glu Asn Gln Phe Lys Glu Ile Glu Lys Thr Thr Asp
    50                  55                  60

Phe Lys Asn His Ser Leu Pro Leu Ala Arg Ile Lys Lys Ile Met Lys
65                  70                  75                  80

Ala Asp Glu Asp Val Arg Met Ile Ser Ala Glu Ala Pro Val Val Phe
                85                  90                  95

Ala Arg Ala Cys Glu Met Phe Ile Leu Glu Leu Thr Leu Arg Ser Trp
            100                 105                 110

Asn His Thr Glu Glu Asn Lys Arg Arg Thr Leu Gln Lys Asn Asp Ile
        115                 120                 125

Ala Ala Ala Val Thr Arg Thr Asp Ile Phe Asp Phe Leu Val Asp Ile
    130                 135                 140

Val Pro Arg Glu Asp Leu Arg Asp Glu Val Leu Gly Ser Ile Pro Arg
145                 150                 155                 160

Gly Thr Val Pro Glu Ala Ala Ala Ala Gly Tyr Pro Tyr Gly Tyr Leu
                165                 170                 175

Pro Ala Gly Thr Ala Pro Ile Gly Asn Pro Gly Met Val Met Gly Asn
            180                 185                 190

Pro Gly Gly Ala Tyr Pro Pro Asn Pro Tyr Met Gly Gln Pro Met Trp
        195                 200                 205

Gln Gln Gln Ala Pro Asp Gln Pro Asp Gln Glu Asn
    210                 215                 220

<210> 37

<211> 1262

<212> DNA

<213> Arabidopsis thaliana

<220>

<221> CDS

<222> (217)..(957)

<223> G463

<400> 37
ctcgagctac gtcaggggtc tctttctgtt tgtttgtttt cttgtttcct tctctctctc 60

tctttctttc tttgtcttcc tttcccaggt tgtttttttt tgctctctct gccttcttga 120

ctttcaaaag actctttctt tcttttggat tgattttgga ttctagggct ctctttcttt 180

tagtgggttt ttgttgttgt tgttgtggtc tctctg atg att act gaa ctt gag 234
Met Ile Thr Glu Leu Glu
1               5

atg ggg aaa ggt gag agt gag ctt gag ctt ggt cta ggg ctg agt ctt 282
Met Gly Lys Gly Glu Ser Glu Leu Glu Leu Gly Leu Gly Leu Ser Leu
            10                  15                  20

ggc ggt gga acg gcg gcc aag att ggt aaa tca ggt ggt ggt ggc gcg 330
Gly Gly Gly Thr Ala Ala Lys Ile Gly Lys Ser Gly Gly Gly Gly Ala
        25                  30                  35

tgg gga gag cgt gga agg ctt ttg acg gct aag gat ttt cct tct gtt 378
Trp Gly Glu Arg Gly Arg Leu Leu Thr Ala Lys Asp Phe Pro Ser Val
    40                  45                  50

ggt tct aaa cgt gct gct gat tct gct tct cat gct ggt tca tct cct 426
Gly Ser Lys Arg Ala Ala Asp Ser Ala Ser His Ala Gly Ser Ser Pro
55                  60                  65                  70

cct cgt tca agt caa gtt gtt gga tgg cct cct ata ggg tca cac agg 474
Pro Arg Ser Ser Gln Val Val Gly Trp Pro Pro Ile Gly Ser His Arg
                75                  80                  85

atg aac agt ttg gtt aat aac caa gct aca aag tca gca aga gaa gaa 522
Met Asn Ser Leu Val Asn Asn Gln Ala Thr Lys Ser Ala Arg Glu Glu
            90                  95                  100

gaa gaa gct ggt aag aag aaa gtg aaa gat gat gaa cct aaa gat gtg 570
Glu Glu Ala Gly Lys Lys Lys Val Lys Asp Asp Glu Pro Lys Asp Val
        105                 110                 115

aca aag aaa gtg aat ggg aaa gta caa gtt gga ttt att aag gtg aac 618
Thr Lys Lys Val Asn Gly Lys Val Gln Val Gly Phe Ile Lys Val Asn
    120                 125                 130

atg gat gga gtt gct ata gga aga aaa gtg gat ttg aat gct cat tct 666

Met Asp Gly Val Ala Ile Gly Arg Lys Val Asp Leu Asn Ala His Ser
135                 140                 145                 150

tct tac gag aat ttg gcg caa aca ttg gaa gat atg ttc ttt cgc act      714
Ser Tyr Glu Asn Leu Ala Gln Thr Leu Glu Asp Met Phe Phe Arg Thr
                155                 160                 165

aat ccg ggt act gtc ggg tta acc agt cag ttc act aaa ccg ttg agg      762
Asn Pro Gly Thr Val Gly Leu Thr Ser Gln Phe Thr Lys Pro Leu Arg
            170                 175                 180

ctt tta gat gga tcg tct gag ttt gta ctt act tat gaa gat aag gaa      810
Leu Leu Asp Gly Ser Ser Glu Phe Val Leu Thr Tyr Glu Asp Lys Glu
        185                 190                 195

gga gat tgg atg ctt gtt ggt gat gtt cca tgg aga atg ttc atc aac      858
Gly Asp Trp Met Leu Val Gly Asp Val Pro Trp Arg Met Phe Ile Asn
    200                 205                 210

tcg gtg aaa agg cta cgt gtg atg aaa acc tct gaa gct aat gga ctc      906
Ser Val Lys Arg Leu Arg Val Met Lys Thr Ser Glu Ala Asn Gly Leu
215                 220                 225                 230

gct gca cga aat caa gaa cca aac gag aga cag cga aag cag ccg gtt      954
Ala Ala Arg Asn Gln Glu Pro Asn Glu Arg Gln Arg Lys Gln Pro Val
                235                 240                 245

tag atctcttttc gacgttacgg tgttacaggt tttatatttt ggggttttgc      1007

aagtctgaga tacttctgaa gcaagcataa gctagattga tcttatatcc agtttgtgta      1067

ttttcttggt tcttataatg gtttttactg gttttcttta gttttttttt ttgctgtctt      1127

ttaattttcg gttgcgattt cactatatac tatggatgga agagaatgct ctttatatct      1187

tttactacac tgtaaatatt tgaagcttat ctaatatcgt ttttaagggt taaaaaaccc      1247

tgacgtagcc tcgag      1262

<210>  38

<211>  246

<212>  PRT

<213>  Arabidopsis thaliana


<400>  38

Met Ile Thr Glu Leu Glu Met Gly Lys Gly Glu Ser Glu Leu Glu Leu
1               5                   10                  15


Gly Leu Gly Leu Ser Leu Gly Gly Gly Thr Ala Ala Lys Ile Gly Lys
            20                  25                  30


Ser Gly Gly Gly Gly Ala Trp Gly Glu Arg Gly Arg Leu Leu Thr Ala
        35                  40                  45


Lys Asp Phe Pro Ser Val Gly Ser Lys Arg Ala Ala Asp Ser Ala Ser
    50                  55                  60

His Ala Gly Ser Ser Pro Pro Arg Ser Ser Gln Val Val Gly Trp Pro
65                  70                  75                  80

Pro Ile Gly Ser His Arg Met Asn Ser Leu Val Asn Asn Gln Ala Thr
                85                  90                  95

Lys Ser Ala Arg Glu Glu Glu Glu Ala Gly Lys Lys Lys Val Lys Asp
            100                 105                 110

Asp Glu Pro Lys Asp Val Thr Lys Lys Val Asn Gly Lys Val Gln Val
        115                 120                 125

Gly Phe Ile Lys Val Asn Met Asp Gly Val Ala Ile Gly Arg Lys Val
    130                 135                 140

Asp Leu Asn Ala His Ser Ser Tyr Glu Asn Leu Ala Gln Thr Leu Glu
145                 150                 155                 160

Asp Met Phe Phe Arg Thr Asn Pro Gly Thr Val Gly Leu Thr Ser Gln
                165                 170                 175

Phe Thr Lys Pro Leu Arg Leu Leu Asp Gly Ser Ser Glu Phe Val Leu
            180                 185                 190

Thr Tyr Glu Asp Lys Glu Gly Asp Trp Met Leu Val Gly Asp Val Pro
        195                 200                 205

Trp Arg Met Phe Ile Asn Ser Val Lys Arg Leu Arg Val Met Lys Thr
    210                 215                 220

Ser Glu Ala Asn Gly Leu Ala Ala Arg Asn Gln Glu Pro Asn Glu Arg
225                 230                 235                 240

Gln Arg Lys Gln Pro Val
                245

<210>  39

<211>  905

<212>  DNA

<213>  Arabidopsis thaliana


<220>

<221>  CDS

<222>  (76)..(837)

<223>  G767

<400> 39
cgaagtttca actttcaaac atatctttac agttctttct tgctaaacaa caataaaggg 60

gaaaataggt taatt atg atg aaa tct ggg gct gat ttg caa ttt cca cca 111
Met Met Lys Ser Gly Ala Asp Leu Gln Phe Pro Pro
1               5                   10

gga ttt aga ttt cat cct acg gat gag gag cta gtc ctc atg tat ctc 159
Gly Phe Arg Phe His Pro Thr Asp Glu Glu Leu Val Leu Met Tyr Leu
        15                  20                  25

tgt cgt aaa tgc gcg tcg cag ccg atc cct gct ccg att atc acc gaa 207
Cys Arg Lys Cys Ala Ser Gln Pro Ile Pro Ala Pro Ile Ile Thr Glu
    30                  35                  40

ctc gat ttg tac cga tat gat cct tgg gac ctt ccc gac atg gct ttg 255
Leu Asp Leu Tyr Arg Tyr Asp Pro Trp Asp Leu Pro Asp Met Ala Leu
45                  50                  55                  60

tac ggt gaa aag gag tgg tat ttt ttc tca cca aga gat cga aag tat 303
Tyr Gly Glu Lys Glu Trp Tyr Phe Phe Ser Pro Arg Asp Arg Lys Tyr
                65                  70                  75

cca aac ggt tca aga ccc aac cgt gca gct ggt act gga tat tgg aaa 351
Pro Asn Gly Ser Arg Pro Asn Arg Ala Ala Gly Thr Gly Tyr Trp Lys
            80                  85                  90

gct acc gga gct gat aaa cca ata ggt cgt cct aaa ccg gtt ggt att 399
Ala Thr Gly Ala Asp Lys Pro Ile Gly Arg Pro Lys Pro Val Gly Ile
        95                  100                 105

aag aag gct cta gtg ttt tac tcg gga aaa cct cca aat gga gag aaa 447
Lys Lys Ala Leu Val Phe Tyr Ser Gly Lys Pro Pro Asn Gly Glu Lys
    110                 115                 120

acc aat tgg att atg cac gaa tac cgg ctc gct gac gtt gac cgg tcg 495
Thr Asn Trp Ile Met His Glu Tyr Arg Leu Ala Asp Val Asp Arg Ser
125                 130                 135                 140

gtt cgt aag aaa aac agt cta aga ttg gac gat tgg gta ttg tgt cgt 543
Val Arg Lys Lys Asn Ser Leu Arg Leu Asp Asp Trp Val Leu Cys Arg
                145                 150                 155

ata tat aac aag aaa ggt gtc atc gag aag cga cga agc gat atc gag 591
Ile Tyr Asn Lys Lys Gly Val Ile Glu Lys Arg Arg Ser Asp Ile Glu
            160                 165                 170

gac ggg tta aag cct gtg act gac acg tgt cca ccg gaa tct gtg gcg 639
Asp Gly Leu Lys Pro Val Thr Asp Thr Cys Pro Pro Glu Ser Val Ala
        175                 180                 185

aga ttg atc tcc ggc tcg gag caa gcg gtg tca ccg gaa ttc acg tgt 687
Arg Leu Ile Ser Gly Ser Glu Gln Ala Val Ser Pro Glu Phe Thr Cys
    190                 195                 200

agc aac ggt cgg ttg agt aat gcc ctt gat ttt ccg ttt aat tac gta 735
Ser Asn Gly Arg Leu Ser Asn Ala Leu Asp Phe Pro Phe Asn Tyr Val
205                 210                 215                 220

gat gcc atc gcc gat aac gag att gtg tca cgg cta ttg ggc ggg aat 783
Asp Ala Ile Ala Asp Asn Glu Ile Val Ser Arg Leu Leu Gly Gly Asn
                225                 230                 235

cag atg tgg tcg acg acg ctt gat cca ctt gtg gtt agg cag gga act 831
Gln Met Trp Ser Thr Thr Leu Asp Pro Leu Val Val Arg Gln Gly Thr
            240                 245                 250

ttc tga gttgtcacgt gcgattagag ttagtggaaa gtggaaacta tcactgtctg        887
Phe

ttttcgcacg tgtcgggc                                                   905

<210>  40

<211>  253

<212>  PRT

<213>  Arabidopsis thaliana


<400>  40

Met Met Lys Ser Gly Ala Asp Leu Gln Phe Pro Pro Gly Phe Arg Phe
1               5                   10                  15

His Pro Thr Asp Glu Glu Leu Val Leu Met Tyr Leu Cys Arg Lys Cys
            20                  25                  30

Ala Ser Gln Pro Ile Pro Ala Pro Ile Ile Thr Glu Leu Asp Leu Tyr
        35                  40                  45

Arg Tyr Asp Pro Trp Asp Leu Pro Asp Met Ala Leu Tyr Gly Glu Lys
    50                  55                  60

Glu Trp Tyr Phe Phe Ser Pro Arg Asp Arg Lys Tyr Pro Asn Gly Ser
65                  70                  75                  80

Arg Pro Asn Arg Ala Ala Gly Thr Gly Tyr Trp Lys Ala Thr Gly Ala
                85                  90                  95

Asp Lys Pro Ile Gly Arg Pro Lys Pro Val Gly Ile Lys Lys Ala Leu
            100                 105                 110

Val Phe Tyr Ser Gly Lys Pro Pro Asn Gly Glu Lys Thr Asn Trp Ile
        115                 120                 125

Met His Glu Tyr Arg Leu Ala Asp Val Asp Arg Ser Val Arg Lys Lys
    130                 135                 140

Asn Ser Leu Arg Leu Asp Asp Trp Val Leu Cys Arg Ile Tyr Asn Lys
145                 150                 155                 160

Lys Gly Val Ile Glu Lys Arg Arg Ser Asp Ile Glu Asp Gly Leu Lys
                165                 170                 175

Pro Val Thr Asp Thr Cys Pro Pro Glu Ser Val Ala Arg Leu Ile Ser
            180                 185                 190

Gly Ser Glu Gln Ala Val Ser Pro Glu Phe Thr Cys Ser Asn Gly Arg
        195                 200                 205

Leu Ser Asn Ala Leu Asp Phe Pro Phe Asn Tyr Val Asp Ala Ile Ala
    210                 215                 220

Asp Asn Glu Ile Val Ser Arg Leu Leu Gly Gly Asn Gln Met Trp Ser
225                 230                 235                 240

Thr Thr Leu Asp Pro Leu Val Val Arg Gln Gly Thr Phe
                245                 250

<210>  41

<211>  1479

<212>  DNA

<213>  Arabidopsis thaliana

<220>

<221>  CDS

<222>  (192)..(962)

<223>  G765

<400>  41
tttaactgca tagttcttcc tcaaattgct catacctttg cttctctttc tttctttatt      60

caacctctta tttcttcttc agcaccaacg tgtatctgca gtaaacacat atccatcgat     120

cgatctcctc aaaaagttat tgttttcttg aaggattttt cttgttcttg atcaagcata     180

catatatata g atg gtg gaa gaa ggc ggc gta gtt gtg aat caa gga gga     230
             Met Val Glu Glu Gly Gly Val Val Val Asn Gln Gly Gly
             1               5                   10

gat cag gag gtg gtg gat ttg cct ccg ggg ttt cgg ttt cac cct act      278
Asp Gln Glu Val Val Asp Leu Pro Pro Gly Phe Arg Phe His Pro Thr
    15                  20                  25

gat gaa gag ata ata act cac tac ctc aaa gag aag gtc ttc aac atc      326
Asp Glu Glu Ile Ile Thr His Tyr Leu Lys Glu Lys Val Phe Asn Ile
30                  35                  40                  45

cga ttt acc gcg gca gcg att ggt caa gcc gac ctc aac aag aac gag      374
Arg Phe Thr Ala Ala Ala Ile Gly Gln Ala Asp Leu Asn Lys Asn Glu
                50                  55                  60

cca tgg gat cta cca aag att gca aag atg ggg gag aag gag ttt tac      422
Pro Trp Asp Leu Pro Lys Ile Ala Lys Met Gly Glu Lys Glu Phe Tyr
            65                  70                  75

ttt ttc tgc cag agg gat cgg aag tat ccg acc ggg atg agg acg aac      470
Phe Phe Cys Gln Arg Asp Arg Lys Tyr Pro Thr Gly Met Arg Thr Asn
        80                  85                  90

cgt gcg acc gtg tct ggt tat tgg aag gcg acc ggg aag gac aag gag     518
Arg Ala Thr Val Ser Gly Tyr Trp Lys Ala Thr Gly Lys Asp Lys Glu
    95                  100                 105

atc ttt aga ggc aaa ggt tgt ctt gtt ggg atg aag aaa aca ctt gtt     566
Ile Phe Arg Gly Lys Gly Cys Leu Val Gly Met Lys Lys Thr Leu Val
110                 115                 120                 125

ttc tat aca gga aga gct cct aaa ggt gaa aag acc aat tgg gtt atg     614
Phe Tyr Thr Gly Arg Ala Pro Lys Gly Glu Lys Thr Asn Trp Val Met
                130                 135                 140

cat gaa tat cgt ctt gat gga aaa tat tct tat cat aac ctc ccc aaa     662
His Glu Tyr Arg Leu Asp Gly Lys Tyr Ser Tyr His Asn Leu Pro Lys
            145                 150                 155

acc gca agg gat gaa tgg gtg gtg tgt agg gtt ttt cac aag aac gct     710
Thr Ala Arg Asp Glu Trp Val Val Cys Arg Val Phe His Lys Asn Ala
        160                 165                 170

cct agt act aca atc act act aca aaa caa ctc tca agg att gat tct     758
Pro Ser Thr Thr Ile Thr Thr Thr Lys Gln Leu Ser Arg Ile Asp Ser
    175                 180                 185

ctt gat aac att gat cat ctc tta gac ttc tca tct ctc cct cct ctc     806
Leu Asp Asn Ile Asp His Leu Leu Asp Phe Ser Ser Leu Pro Pro Leu
190                 195                 200                 205

ata gat ccg ggt ttc ttg ggt caa ccc gcc caa gct tct ccg gtg ccc     854
Ile Asp Pro Gly Phe Leu Gly Gln Pro Ala Gln Ala Ser Pro Val Pro
                210                 215                 220

gtc aac aac acg atc tca aac ctg tct cca cca tcc tac aac cgc acc     902
Val Asn Asn Thr Ile Ser Asn Leu Ser Pro Pro Ser Tyr Asn Arg Thr
            225                 230                 235

agt cga caa cac tta cct tcc tac cca agc tct caa ttt ccc tta cca     950
Ser Arg Gln His Leu Pro Ser Tyr Pro Ser Ser Gln Phe Pro Leu Pro
        240                 245                 250

ctc ggt ccc taa ttccggatct gatttcggct acggggcagg ttcaggcaat     1002
Leu Gly Pro
    255

aataacaaag gtatgatcaa gttggagcat tctcttgtga gtgtgtctca agaaaccggt     1062

ttgagttccg atgtgaacac aaccgcaacg ccagagatat cttcttatcc aatgatgatg     1122

aatccggcaa tgatggatgg tagcaagtca gcgtgtgatg gtcttgatga cttgatcttc     1182

tgggaagatt tatatactag ctaaatttgg gaaaaggtta tttgttaatt gtgattgaag     1242

agtggcatat tgattactcg tctagtgttt ttaatcgtgt aattagttcg tatataatat     1302

acatgtacat aagatcatta ggtttattag gcattggact ttagttcggt gattgcttac     1362

ctagttttta gcttgagaaa aaaggctgtc attggggtta tgtttctttg tgattaactt     1422

gtacatatat acatttaaat taaacgtatg gtttaaatcg tttaaaaaaa aaaaaaa     1479

<210>  42

<211>  256

<212>  PRT

<213>  Arabidopsis thaliana

<400>  42

Met Val Glu Glu Gly Gly Val Val Val Asn Gln Gly Gly Asp Gln Glu
1               5                   10                  15

Val Val Asp Leu Pro Pro Gly Phe Arg Phe His Pro Thr Asp Glu Glu
            20                  25                  30

Ile Ile Thr His Tyr Leu Lys Glu Lys Val Phe Asn Ile Arg Phe Thr
        35                  40                  45

Ala Ala Ala Ile Gly Gln Ala Asp Leu Asn Lys Asn Glu Pro Trp Asp
    50                  55                  60

Leu Pro Lys Ile Ala Lys Met Gly Glu Lys Glu Phe Tyr Phe Phe Cys
65                  70                  75                  80

Gln Arg Asp Arg Lys Tyr Pro Thr Gly Met Arg Thr Asn Arg Ala Thr
                85                  90                  95

Val Ser Gly Tyr Trp Lys Ala Thr Gly Lys Asp Lys Glu Ile Phe Arg
            100                 105                 110

Gly Lys Gly Cys Leu Val Gly Met Lys Lys Thr Leu Val Phe Tyr Thr
        115                 120                 125

Gly Arg Ala Pro Lys Gly Glu Lys Thr Asn Trp Val Met His Glu Tyr
    130                 135                 140

Arg Leu Asp Gly Lys Tyr Ser Tyr His Asn Leu Pro Lys Thr Ala Arg
145                 150                 155                 160

Asp Glu Trp Val Val Cys Arg Val Phe His Lys Asn Ala Pro Ser Thr
                165                 170                 175

Thr Ile Thr Thr Thr Lys Gln Leu Ser Arg Ile Asp Ser Leu Asp Asn
            180                 185                 190

Ile Asp His Leu Leu Asp Phe Ser Ser Leu Pro Pro Leu Ile Asp Pro
        195                 200                 205

Gly Phe Leu Gly Gln Pro Ala Gln Ala Ser Pro Val Pro Val Asn Asn
    210                 215                 220

Thr Ile Ser Asn Leu Ser Pro Pro Ser Tyr Asn Arg Thr Ser Arg Gln
225                 230                 235                 240

His Leu Pro Ser Tyr Pro Ser Ser Gln Phe Pro Leu Pro Leu Gly Pro
                245                 250                 255

<210>  43

<211>  825

<212>  DNA

<213>  Arabidopsis thaliana

<220>

<221>  CDS

<222>  (1)..(825)

<223>  G197

<400>  43
atg gga agg tct cct tgc tgt gag aaa gac cac aca aac aaa gga gct        48
Met Gly Arg Ser Pro Cys Cys Glu Lys Asp His Thr Asn Lys Gly Ala
1               5                   10                  15

tgg act aag gaa gaa gac gat aag ctc atc tct tac atc aaa gct cac        96
Trp Thr Lys Glu Glu Asp Asp Lys Leu Ile Ser Tyr Ile Lys Ala His
            20                  25                  30

ggt gaa ggt tgt tgg cgt tct ctt cct aga tcc gcc ggt ctt caa cgt       144
Gly Glu Gly Cys Trp Arg Ser Leu Pro Arg Ser Ala Gly Leu Gln Arg
        35                  40                  45

tgc gga aaa agc tgt cgt ctc cga tgg att aac tat ctc cga cct gat       192
Cys Gly Lys Ser Cys Arg Leu Arg Trp Ile Asn Tyr Leu Arg Pro Asp
    50                  55                  60

ctc aag agg ggt aac ttc acc ctc gaa gaa gat gat ctc atc atc aaa       240
Leu Lys Arg Gly Asn Phe Thr Leu Glu Glu Asp Asp Leu Ile Ile Lys
65                  70                  75                  80

cta cat agc ctt ctc ggt aac aag tgg tct ctt att gcg acg aga tta       288
Leu His Ser Leu Leu Gly Asn Lys Trp Ser Leu Ile Ala Thr Arg Leu
                85                  90                  95

cca gga aga aca gat aac gag att aag aat tac tgg aac aca cat gtt       336
Pro Gly Arg Thr Asp Asn Glu Ile Lys Asn Tyr Trp Asn Thr His Val
            100                 105                 110

aag agg aag cta tta aga aaa ggg att gat ccg gcg act cat cga cct       384
Lys Arg Lys Leu Leu Arg Lys Gly Ile Asp Pro Ala Thr His Arg Pro
        115                 120                 125

atc aac gag acc aaa act tct caa gat tcg tct gat tct agt aaa aca       432
Ile Asn Glu Thr Lys Thr Ser Gln Asp Ser Ser Asp Ser Ser Lys Thr
    130                 135                 140

gag gac cct ctt gtc aag att ctc tct ttt ggt cct cag ctg gag aaa       480
Glu Asp Pro Leu Val Lys Ile Leu Ser Phe Gly Pro Gln Leu Glu Lys
145                 150                 155                 160

ata gca aat ttc ggg gac gag aga att caa aag aga gtt gag tac tca       528
Ile Ala Asn Phe Gly Asp Glu Arg Ile Gln Lys Arg Val Glu Tyr Ser
                165                 170                 175

gtt gtt gaa gaa aga tgt ctg gac ttg aat ctt gag ctt agg atc agt     576
Val Val Glu Glu Arg Cys Leu Asp Leu Asn Leu Glu Leu Arg Ile Ser
            180                 185                 190

cca cca tgg caa gac aag ctc cat gat gag agg aac cta agg ttt ggg     624
Pro Pro Trp Gln Asp Lys Leu His Asp Glu Arg Asn Leu Arg Phe Gly
        195                 200                 205

aga gtg aag tat agg tgc agt gcg tgc cgt ttt gga ttc ggg aac ggc     672
Arg Val Lys Tyr Arg Cys Ser Ala Cys Arg Phe Gly Phe Gly Asn Gly
    210                 215                 220

aag gag tgt agc tgt aat aat gtg aaa tgt caa aca gag gac agt agt     720
Lys Glu Cys Ser Cys Asn Asn Val Lys Cys Gln Thr Glu Asp Ser Ser
225                 230                 235                 240

agc agc agt tat tct tca acc gac att agt agt agc att ggt tat gac     768
Ser Ser Ser Tyr Ser Ser Thr Asp Ile Ser Ser Ser Ile Gly Tyr Asp
                245                 250                 255

ttc ttg ggt cta aac aac act agg gtt ttg gat ttt agc act ttg gaa     816
Phe Leu Gly Leu Asn Asn Thr Arg Val Leu Asp Phe Ser Thr Leu Glu
            260                 265                 270

atg aaa tga                                                         825
Met Lys

<210>  44

<211>  274

<212>  PRT

<213>  Arabidopsis thaliana

<400>  44

Met Gly Arg Ser Pro Cys Cys Glu Lys Asp His Thr Asn Lys Gly Ala
1               5                   10                  15

Trp Thr Lys Glu Glu Asp Asp Lys Leu Ile Ser Tyr Ile Lys Ala His
            20                  25                  30

Gly Glu Gly Cys Trp Arg Ser Leu Pro Arg Ser Ala Gly Leu Gln Arg
        35                  40                  45

Cys Gly Lys Ser Cys Arg Leu Arg Trp Ile Asn Tyr Leu Arg Pro Asp
    50                  55                  60

Leu Lys Arg Gly Asn Phe Thr Leu Glu Glu Asp Asp Leu Ile Ile Lys
65                  70                  75                  80

Leu His Ser Leu Leu Gly Asn Lys Trp Ser Leu Ile Ala Thr Arg Leu
                85                  90                  95

Pro Gly Arg Thr Asp Asn Glu Ile Lys Asn Tyr Trp Asn Thr His Val
            100                 105                 110

Lys Arg Lys Leu Leu Arg Lys Gly Ile Asp Pro Ala Thr His Arg Pro
        115                 120                 125

Ile Asn Glu Thr Lys Thr Ser Gln Asp Ser Ser Asp Ser Ser Lys Thr
    130                 135                 140

Glu Asp Pro Leu Val Lys Ile Leu Ser Phe Gly Pro Gln Leu Glu Lys
145                 150                 155                 160

Ile Ala Asn Phe Gly Asp Glu Arg Ile Gln Lys Arg Val Glu Tyr Ser
                165                 170                 175

Val Val Glu Glu Arg Cys Leu Asp Leu Asn Leu Glu Leu Arg Ile Ser
            180                 185                 190

Pro Pro Trp Gln Asp Lys Leu His Asp Glu Arg Asn Leu Arg Phe Gly
        195                 200                 205

Arg Val Lys Tyr Arg Cys Ser Ala Cys Arg Phe Gly Phe Gly Asn Gly
    210                 215                 220

Lys Glu Cys Ser Cys Asn Asn Val Lys Cys Gln Thr Glu Asp Ser Ser
225                 230                 235                 240

Ser Ser Ser Tyr Ser Ser Thr Asp Ile Ser Ser Ser Ile Gly Tyr Asp
                245                 250                 255

Phe Leu Gly Leu Asn Asn Thr Arg Val Leu Asp Phe Ser Thr Leu Glu
            260                 265                 270

Met Lys

<210>  45

<211>  918

<212>  DNA

<213>  Arabidopsis thalina

<220>

<221>  CDS

<222>  (30)..(839)

<223>  G255

<400> 45
agcatcatca tcatcagaag aagagagtc atg gga aga tct cct tgc tgc gag 53
Met Gly Arg Ser Pro Cys Cys Glu
1               5

aaa gaa cac atg aac aaa ggt gct tgg act aaa gaa gaa gat gag aga 101
Lys Glu His Met Asn Lys Gly Ala Trp Thr Lys Glu Glu Asp Glu Arg
    10                  15                  20

cta gtc tct tac atc aag tct cac ggt gaa ggt tgt tgg cga tct ctt 149
Leu Val Ser Tyr Ile Lys Ser His Gly Glu Gly Cys Trp Arg Ser Leu
25                  30                  35                  40

cct aga gcc gct ggt ctc ctt cgc tgc ggt aaa agc tgc cgt ctt cgg 197
Pro Arg Ala Ala Gly Leu Leu Arg Cys Gly Lys Ser Cys Arg Leu Arg
                45                  50                  55

tgg att aac tat ctc cga cct gat ctc aaa aga gga aac ttt aca cat 245
Trp Ile Asn Tyr Leu Arg Pro Asp Leu Lys Arg Gly Asn Phe Thr His
            60                  65                  70

gat gaa gat gaa ctt atc atc aag ctt cat agc ctc cta ggc aac aag 293
Asp Glu Asp Glu Leu Ile Ile Lys Leu His Ser Leu Leu Gly Asn Lys
        75                  80                  85

tgg tct ttg att gcg gcg aga tta cct gga aga aca gat aac gag atc 341
Trp Ser Leu Ile Ala Ala Arg Leu Pro Gly Arg Thr Asp Asn Glu Ile
    90                  95                  100

aag aac tac tgg aac aca cat ata aag agg aag ctt ttg agc aaa ggg 389
Lys Asn Tyr Trp Asn Thr His Ile Lys Arg Lys Leu Leu Ser Lys Gly
105                 110                 115                 120

att gat cca gcc act cat aga ggg atc aac gag gca aaa att tct gat 437
Ile Asp Pro Ala Thr His Arg Gly Ile Asn Glu Ala Lys Ile Ser Asp
                125                 130                 135

ttg aag aaa aca aag gac caa att gta aaa gat gtt tct ttt gtg aca 485
Leu Lys Lys Thr Lys Asp Gln Ile Val Lys Asp Val Ser Phe Val Thr
            140                 145                 150

aag ttt gag gaa aca gac aag tct ggg gac cag aag caa aat aag tat 533
Lys Phe Glu Glu Thr Asp Lys Ser Gly Asp Gln Lys Gln Asn Lys Tyr
        155                 160                 165

att cga aat ggg tta gtt tgc aaa gaa gag aga gtt gtt gtt gaa gaa 581
Ile Arg Asn Gly Leu Val Cys Lys Glu Glu Arg Val Val Val Glu Glu
    170                 175                 180

aaa ata ggc cca gat ttg aat ctt gag ctt agg atc agt cca cca tgg 629
Lys Ile Gly Pro Asp Leu Asn Leu Glu Leu Arg Ile Ser Pro Pro Trp
185                 190                 195                 200

caa aac cag aga gaa ata tct act tgc act gcg tcc cgt ttt tac atg 677
Gln Asn Gln Arg Glu Ile Ser Thr Cys Thr Ala Ser Arg Phe Tyr Met
                205                 210                 215

gaa aac gac atg gag tgt agt agt gaa act gtg aaa tgt caa aca gag 725
Glu Asn Asp Met Glu Cys Ser Ser Glu Thr Val Lys Cys Gln Thr Glu
            220                 225                 230

aat agt agc agc att agc tat tct tct att gat att agt agt agt aac 773
Asn Ser Ser Ser Ile Ser Tyr Ser Ser Ile Asp Ile Ser Ser Ser Asn
        235                 240                 245

gtt ggt tat gac ttc ttg ggt ttg aag aca aga att ttg gat ttt cga 821
Val Gly Tyr Asp Phe Leu Gly Leu Lys Thr Arg Ile Leu Asp Phe Arg
    250                 255                 260

agc ttg gaa atg aaa taa atgaatagta ttagattctt aatttgtagg          869
Ser Leu Glu Met Lys
265

tctgataatg aatgttagat tcgcggccct ctagacaggc ctcgtaccg          918

<210> 46

<211> 269

<212> PRT

<213> Arabidopsis thalina

<400> 46

Met Gly Arg Ser Pro Cys Cys Glu Lys Glu His Met Asn Lys Gly Ala
1               5                   10                  15

Trp Thr Lys Glu Glu Asp Glu Arg Leu Val Ser Tyr Ile Lys Ser His
            20                  25                  30

Gly Glu Gly Cys Trp Arg Ser Leu Pro Arg Ala Ala Gly Leu Leu Arg
        35                  40                  45

Cys Gly Lys Ser Cys Arg Leu Arg Trp Ile Asn Tyr Leu Arg Pro Asp
    50                  55                  60

Leu Lys Arg Gly Asn Phe Thr His Asp Glu Asp Glu Leu Ile Ile Lys
65                  70                  75                  80

Leu His Ser Leu Leu Gly Asn Lys Trp Ser Leu Ile Ala Ala Arg Leu
                85                  90                  95

Pro Gly Arg Thr Asp Asn Glu Ile Lys Asn Tyr Trp Asn Thr His Ile
            100                 105                 110

Lys Arg Lys Leu Leu Ser Lys Gly Ile Asp Pro Ala Thr His Arg Gly
        115                 120                 125

Ile Asn Glu Ala Lys Ile Ser Asp Leu Lys Lys Thr Lys Asp Gln Ile
    130                 135                 140

Val Lys Asp Val Ser Phe Val Thr Lys Phe Glu Glu Thr Asp Lys Ser
145                 150                 155                 160

Gly Asp Gln Lys Gln Asn Lys Tyr Ile Arg Asn Gly Leu Val Cys Lys
                165                 170                 175

Glu Glu Arg Val Val Val Glu Glu Lys Ile Gly Pro Asp Leu Asn Leu
            180                 185                 190

Glu Leu Arg Ile Ser Pro Pro Trp Gln Asn Gln Arg Glu Ile Ser Thr
        195                 200                 205

Cys Thr Ala Ser Arg Phe Tyr Met Glu Asn Asp Met Glu Cys Ser Ser
    210                 215                 220

Glu Thr Val Lys Cys Gln Thr Glu Asn Ser Ser Ser Ile Ser Tyr Ser
225                 230                 235                 240

Ser Ile Asp Ile Ser Ser Ser Asn Val Gly Tyr Asp Phe Leu Gly Leu
                245                 250                 255

Lys Thr Arg Ile Leu Asp Phe Arg Ser Leu Glu Met Lys
            260                 265

<210> 47

<211> 660

<212> DNA

<213> Arabidopsis thaliana

<220>

<221> CDS

<222> (48)..(521)

<223> G1113

<400> 47
aagctttatc tctttctttc tctcctctct atctttctct cacactc atg ggt ctt 56
Met Gly Leu
1

cca aca gat ttc aaa gag ctt cag att cca ggt tac gta cta aaa aca 104
Pro Thr Asp Phe Lys Glu Leu Gln Ile Pro Gly Tyr Val Leu Lys Thr
    5                   10                  15

ctt tac gtc atc ggt ttc ttt aga gac atg gtc gat gct ctt tgt cct 152
Leu Tyr Val Ile Gly Phe Phe Arg Asp Met Val Asp Ala Leu Cys Pro
20                  25                  30                  35

tac atc ggt cta cca agt ttt ctt gac cac aac gag acc tct cga tcc 200
Tyr Ile Gly Leu Pro Ser Phe Leu Asp His Asn Glu Thr Ser Arg Ser
                40                  45                  50

gac ccg acc cga ctc gct ctc tcc acg tca gca act ctt gcc aac gag 248
Asp Pro Thr Arg Leu Ala Leu Ser Thr Ser Ala Thr Leu Ala Asn Glu
            55                  60                  65

tta atc ccg gtg gtt cgt ttc tcc gat ctt tta acc gat ccg gaa gat 296
Leu Ile Pro Val Val Arg Phe Ser Asp Leu Leu Thr Asp Pro Glu Asp
        70                  75                  80

tgc tgc acg gtt tgc tta tcc gat ttt gta tcc gac gat aag att aga 344

Cys Cys Thr Val Cys Leu Ser Asp Phe Val Ser Asp Asp Lys Ile Arg
    85                  90                  95

cag ctg ccg aag tgt gga cac gtg ttt cat cat cgt tgt tta gac cgt      392
Gln Leu Pro Lys Cys Gly His Val Phe His His Arg Cys Leu Asp Arg
100                 105                 110                 115

tgg atc gtt gac tgt aat aag ata acg tgc ccg att tgt cgg aac cgg      440
Trp Ile Val Asp Cys Asn Lys Ile Thr Cys Pro Ile Cys Arg Asn Arg
                120                 125                 130

ttc tta ccg gag gaa aag tcc acg ccg ttt gat tgg ggt act tca gat      488
Phe Leu Pro Glu Glu Lys Ser Thr Pro Phe Asp Trp Gly Thr Ser Asp
            135                 140                 145

tgg ttt aga gat gaa gtg gag agt acc aac taa taatgatggt tttactttta    541
Trp Phe Arg Asp Glu Val Glu Ser Thr Asn
        150                 155

ctttttactt ttttcacggt aatatttttc tactgtataa ttctttcttc caaactactg    601

tataattcaa gtataagatt atgtaattgt gtatattagc atcaatcatc tttctttgt    660

<210>  48

<211>  157

<212>  PRT

<213>  Arabidopsis thaliana

<400>  48

Met Gly Leu Pro Thr Asp Phe Lys Glu Leu Gln Ile Pro Gly Tyr Val
1               5                   10                  15

Leu Lys Thr Leu Tyr Val Ile Gly Phe Phe Arg Asp Met Val Asp Ala
            20                  25                  30

Leu Cys Pro Tyr Ile Gly Leu Pro Ser Phe Leu Asp His Asn Glu Thr
        35                  40                  45

Ser Arg Ser Asp Pro Thr Arg Leu Ala Leu Ser Thr Ser Ala Thr Leu
    50                  55                  60

Ala Asn Glu Leu Ile Pro Val Val Arg Phe Ser Asp Leu Leu Thr Asp
65                  70                  75                  80

Pro Glu Asp Cys Cys Thr Val Cys Leu Ser Asp Phe Val Ser Asp Asp
                85                  90                  95

Lys Ile Arg Gln Leu Pro Lys Cys Gly His Val Phe His His Arg Cys
            100                 105                 110

Leu Asp Arg Trp Ile Val Asp Cys Asn Lys Ile Thr Cys Pro Ile Cys
        115                 120                 125

Arg Asn Arg Phe Leu Pro Glu Glu Lys Ser Thr Pro Phe Asp Trp Gly
    130                 135                 140

Thr Ser Asp Trp Phe Arg Asp Glu Val Glu Ser Thr Asn
145                 150                 155

<210>  49

<211>  1201

<212>  DNA

<213>  Arabidopsis thaliana

<220>

<221>  CDS

<222>  (148)..(996)

<223>  G398

<400>  49
   agaaggtttc tcttgtcctc catacactta gcacaactga taaatctttt gaggtaaaat      60
   cagctttaga tcaaggtttt tctagtcatc tctactcata aagatcaaag cttttgctat     120
   tctcattttc taccaagaga caatatc atg atg atg ggt aaa gag gat ttg ggt     174
                                 Met Met Met Gly Lys Glu Asp Leu Gly
                                 1               5
   tta agt ctt agc ttg gga ttt gca caa aac cat cct ctc cag cta aat       222
   Leu Ser Leu Ser Leu Gly Phe Ala Gln Asn His Pro Leu Gln Leu Asn
   10                  15                  20                  25
   ctt aaa ccc act tct tca cca atg tcc aat ctc cag atg ttt cca tgg       270
   Leu Lys Pro Thr Ser Ser Pro Met Ser Asn Leu Gln Met Phe Pro Trp
                   30                  35                  40
   aac caa acc ctt gtt tct tcc tca gat caa caa aag caa cag ttt ctt       318
   Asn Gln Thr Leu Val Ser Ser Ser Asp Gln Gln Lys Gln Gln Phe Leu
               45                  50                  55
   agg aaa atc gac gtg aac agc ttg cca aca acg gtg gat ttg gaa gag       366
   Arg Lys Ile Asp Val Asn Ser Leu Pro Thr Thr Val Asp Leu Glu Glu
           60                  65                  70
   gag aca gga gtt tcg tct cca aac agt acg atc tcg agc aca gtg agt       414
   Glu Thr Gly Val Ser Ser Pro Asn Ser Thr Ile Ser Ser Thr Val Ser
       75                  80                  85
   gga aag agg agg agt act gaa aga gaa ggt acc tcc ggt ggt ggt tgc       462
   Gly Lys Arg Arg Ser Thr Glu Arg Glu Gly Thr Ser Gly Gly Gly Cys
   90                  95                  100                 105
   gga gat gac ctt gac atc act cta gat aga tct tcc tca cgt gga acc       510
   Gly Asp Asp Leu Asp Ile Thr Leu Asp Arg Ser Ser Ser Arg Gly Thr
                   110                 115                 120
   tcc gat gaa gag gaa gat tac gga ggt gag act tgt agg aag aag ctt       558
   Ser Asp Glu Glu Glu Asp Tyr Gly Gly Glu Thr Cys Arg Lys Lys Leu
           125                 130                 135
   aga cta tcc aaa gat caa tcc gca gtt ctc gaa gac act ttc aaa gag      606
   Arg Leu Ser Lys Asp Gln Ser Ala Val Leu Glu Asp Thr Phe Lys Glu
       140                 145                 150
   cac aat act ctc aat ccc aaa cag aag ctg gct ttg gct aag aag cta      654
   His Asn Thr Leu Asn Pro Lys Gln Lys Leu Ala Leu Ala Lys Lys Leu
       155                 160                 165
   ggt tta aca gca aga caa gtg gaa gtg tgg ttc caa aac aga aga gca      702
   Gly Leu Thr Ala Arg Gln Val Glu Val Trp Phe Gln Asn Arg Arg Ala
   170                 175                 180                 185
   agg aca aag tta aag cag acc gaa gtg gat tgc gag tat ttg aaa aga      750
   Arg Thr Lys Leu Lys Gln Thr Glu Val Asp Cys Glu Tyr Leu Lys Arg
                   190                 195                 200
   tgt gtt gag aaa tta acg gaa gag aat cgg cgg ctc gag aaa gag gca      798
   Cys Val Glu Lys Leu Thr Glu Glu Asn Arg Arg Leu Glu Lys Glu Ala
               205                 210                 215
   gcg gaa cta aga gca tta aag ctt tca ccg cgg ttg tat ggt cag atg      846
   Ala Glu Leu Arg Ala Leu Lys Leu Ser Pro Arg Leu Tyr Gly Gln Met
           220                 225                 230
   agt cca ccg acc aca ctt ttg atg tgt cca tcg tgt gaa cgt gtg gcc      894
   Ser Pro Pro Thr Thr Leu Leu Met Cys Pro Ser Cys Glu Arg Val Ala
       235                 240                 245
   gga cca tcc tca tct aac cac aac cag cga tct gtc tca ttg agt cca      942
   Gly Pro Ser Ser Ser Asn His Asn Gln Arg Ser Val Ser Leu Ser Pro
   250                 255                 260                 265
   tgg ctc caa atg gcc cat ggg tca acc ttt gat gtg atg cgt cct agg      990
   Trp Leu Gln Met Ala His Gly Ser Thr Phe Asp Val Met Arg Pro Arg
                   270                 275                 280
   tct taa ctttaatgct gcttctatgg gttgtgtgtg ggtcattgta ctttttagat      1046
   Ser
   tattgactct cagctaatgt atccttaaaa gcctttttct acttttaaat ttactttaat      1106
   ctaattaaat tagttgtcca tgtcttcttg ataacaaaaa aatttataat tataaaaaaa      1166
   aaaaacagga taaaaaaaaa aaaaaaaaaa aaaaa      1201

<210>  50

<211>  282

<212>  PRT

<213>  Arabidopsis thaliana


<400>  50

Met Met Met Gly Lys Glu Asp Leu Gly Leu Ser Leu Ser Leu Gly Phe
1               5                   10                  15


Ala Gln Asn His Pro Leu Gln Leu Asn Leu Lys Pro Thr Ser Ser Pro
            20                  25                  30

Met Ser Asn Leu Gln Met Phe Pro Trp Asn Gln Thr Leu Val Ser Ser
        35                  40                  45

Ser Asp Gln Gln Lys Gln Gln Phe Leu Arg Lys Ile Asp Val Asn Ser
    50                  55                  60

Leu Pro Thr Thr Val Asp Leu Glu Glu Glu Thr Gly Val Ser Ser Pro
65                  70                  75                  80

Asn Ser Thr Ile Ser Ser Thr Val Ser Gly Lys Arg Arg Ser Thr Glu
                85                  90                  95

Arg Glu Gly Thr Ser Gly Gly Gly Cys Gly Asp Asp Leu Asp Ile Thr
            100                 105                 110

Leu Asp Arg Ser Ser Ser Arg Gly Thr Ser Asp Glu Glu Glu Asp Tyr
        115                 120                 125

Gly Gly Glu Thr Cys Arg Lys Lys Leu Arg Leu Ser Lys Asp Gln Ser
    130                 135                 140

Ala Val Leu Glu Asp Thr Phe Lys Glu His Asn Thr Leu Asn Pro Lys
145                 150                 155                 160

Gln Lys Leu Ala Leu Ala Lys Lys Leu Gly Leu Thr Ala Arg Gln Val
                165                 170                 175

Glu Val Trp Phe Gln Asn Arg Arg Ala Arg Thr Lys Leu Lys Gln Thr
            180                 185                 190

Glu Val Asp Cys Glu Tyr Leu Lys Arg Cys Val Glu Lys Leu Thr Glu
        195                 200                 205

Glu Asn Arg Arg Leu Glu Lys Glu Ala Ala Glu Leu Arg Ala Leu Lys
    210                 215                 220

Leu Ser Pro Arg Leu Tyr Gly Gln Met Ser Pro Pro Thr Thr Leu Leu
225                 230                 235                 240

Met Cys Pro Ser Cys Glu Arg Val Ala Gly Pro Ser Ser Ser Asn His
                245                 250                 255

Asn Gln Arg Ser Val Ser Leu Ser Pro Trp Leu Gln Met Ala His Gly
            260                 265                 270

Ser Thr Phe Asp Val Met Arg Pro Arg Ser
        275                 280

<210>  51

<211>  937

<212>  DNA

<213>  Arabidopsis thaliana


<220>

<221>  CDS

<222>  (120)..(797)

<223>  G395



<400>  51
gcacaatccc actatccttc gcaagaccct tcctctatat aaggaagttc atttcatttg      60

gagaggacac gctgacaagc tgactctagc agatctggta ccgtcgacaa ggaggaaga      119

atg ccc tta gga gca gct acg gtt gtg gag gag gaa gag gag gag gag      167
Met Pro Leu Gly Ala Ala Thr Val Val Glu Glu Glu Glu Glu Glu Glu
1               5                   10                  15

gaa gcg gtg cct agt atg tca gta tcg ccg ccg gat agt gta acg tcg      215
Glu Ala Val Pro Ser Met Ser Val Ser Pro Pro Asp Ser Val Thr Ser
            20                  25                  30

tcg ttt caa ttg gac ttt ggg att aaa agt tat ggt tat gag aga aga      263
Ser Phe Gln Leu Asp Phe Gly Ile Lys Ser Tyr Gly Tyr Glu Arg Arg
        35                  40                  45

agc aat aag aga gat att gat gat gaa gtg gag aga tca gcc tca aga      311
Ser Asn Lys Arg Asp Ile Asp Asp Glu Val Glu Arg Ser Ala Ser Arg
    50                  55                  60

gcc agc aac gaa gac aac gat gac gag aat gga tcc act agg aag aaa      359
Ala Ser Asn Glu Asp Asn Asp Asp Glu Asn Gly Ser Thr Arg Lys Lys
65                  70                  75                  80

ctt aga ctc tcc aaa gac caa tct gct ttt ctt gaa gac agc ttc aaa      407
Leu Arg Leu Ser Lys Asp Gln Ser Ala Phe Leu Glu Asp Ser Phe Lys
                85                  90                  95

gaa cac agt acc ctt aat cct aaa cag aag att gca ttg gcg aag cag      455
Glu His Ser Thr Leu Asn Pro Lys Gln Lys Ile Ala Leu Ala Lys Gln
            100                 105                 110

ttg aat ctt cgt cct cgt cag gtt gaa gtc tgg ttt caa aac aga cga      503
Leu Asn Leu Arg Pro Arg Gln Val Glu Val Trp Phe Gln Asn Arg Arg
        115                 120                 125

gcc agg aca aag ctg aag caa acg gaa gtg gac tgt gaa tac cta aag      551
Ala Arg Thr Lys Leu Lys Gln Thr Glu Val Asp Cys Glu Tyr Leu Lys
    130                 135                 140

aga tgc tgt gag tca cta acc gaa gaa aac cgg agg ctt caa aaa gag      599
Arg Cys Cys Glu Ser Leu Thr Glu Glu Asn Arg Arg Leu Gln Lys Glu
145                 150                 155                 160

gtt aaa gaa ttg aga acc ttg aag act tcc aca ccc ttt tac atg caa      647
Val Lys Glu Leu Arg Thr Leu Lys Thr Ser Thr Pro Phe Tyr Met Gln
                165                 170                 175

ctt ccg gcc act act ctc act atg tgc cct tct tgt gaa cgt gtt gcc 695
Leu Pro Ala Thr Thr Leu Thr Met Cys Pro Ser Cys Glu Arg Val Ala
            180                 185                 190

act tca gca gca cag ccc tcc acg tca gct gcc cac aac ctc tgt ttg 743
Thr Ser Ala Ala Gln Pro Ser Thr Ser Ala Ala His Asn Leu Cys Leu
        195                 200                 205

tcc acg tca tca ttg att ccg gtt aag cct cgg ccg gcc aaa caa gtt 791
Ser Thr Ser Ser Leu Ile Pro Val Lys Pro Arg Pro Ala Lys Gln Val
    210                 215                 220

tca tga aagcacctgc gaaatacagt ttgagcaaac gggcggccgc tctagacagg 847
Ser
225

cctcgtaccg gatcctctag ctagagcttt cgttcgtatc atcggtttcg acaacgttcg 907

tcaagttcaa tgacatcagt ttgattgcgc 937

<210> 52

<211> 225

<212> PRT

<213> Arabidopsis thaliana

<400> 52

Met Pro Leu Gly Ala Ala Thr Val Val Glu Glu Glu Glu Glu Glu Glu
1               5                   10                  15

Glu Ala Val Pro Ser Met Ser Val Ser Pro Pro Asp Ser Val Thr Ser
            20                  25                  30

Ser Phe Gln Leu Asp Phe Gly Ile Lys Ser Tyr Gly Tyr Glu Arg Arg
        35                  40                  45

Ser Asn Lys Arg Asp Ile Asp Asp Glu Val Glu Arg Ser Ala Ser Arg
    50                  55                  60

Ala Ser Asn Glu Asp Asn Asp Asp Glu Asn Gly Ser Thr Arg Lys Lys
65                  70                  75                  80

Leu Arg Leu Ser Lys Asp Gln Ser Ala Phe Leu Glu Asp Ser Phe Lys
                85                  90                  95

Glu His Ser Thr Leu Asn Pro Lys Gln Lys Ile Ala Leu Ala Lys Gln
            100                 105                 110

Leu Asn Leu Arg Pro Arg Gln Val Glu Val Trp Phe Gln Asn Arg Arg
        115                 120                 125

Ala Arg Thr Lys Leu Lys Gln Thr Glu Val Asp Cys Glu Tyr Leu Lys
    130                 135                 140

Arg Cys Cys Glu Ser Leu Thr Glu Glu Asn Arg Arg Leu Gln Lys Glu
145                 150                 155                 160

Val Lys Glu Leu Arg Thr Leu Lys Thr Ser Thr Pro Phe Tyr Met Gln
                165                 170                 175

Leu Pro Ala Thr Thr Leu Thr Met Cys Pro Ser Cys Glu Arg Val Ala
            180                 185                 190

Thr Ser Ala Ala Gln Pro Ser Thr Ser Ala Ala His Asn Leu Cys Leu
        195                 200                 205

Ser Thr Ser Ser Leu Ile Pro Val Lys Pro Arg Pro Ala Lys Gln Val
    210                 215                 220

Ser
225

<210>  53

<211>  927

<212>  DNA

<213>  Arabidopsis thaliana

<220>

<221>  CDS

<222>  (37)..(861)

<223>  G393

<400>  53
aaattttaaa ggcatatttt tttcatttcg actcag atg ggt ttt gat gat aca        54
                                        Met Gly Phe Asp Asp Thr
                                        1               5

tgc aac aca ggt ctt gtt ctt gga tta ggt ccc tca cca att tca aat       102
Cys Asn Thr Gly Leu Val Leu Gly Leu Gly Pro Ser Pro Ile Ser Asn
            10                  15                  20

aat tac aat agt acc atc aga caa tcc tcc gtt tac aag ctc gag ccg       150
Asn Tyr Asn Ser Thr Ile Arg Gln Ser Ser Val Tyr Lys Leu Glu Pro
        25                  30                  35

tcg ttg act cta tgc ctc tcg ggc gat ccc tcg gtt acc gtg gtg acc       198
Ser Leu Thr Leu Cys Leu Ser Gly Asp Pro Ser Val Thr Val Val Thr
    40                  45                  50

gga gct gac cag cta tgc cgt cag acg tca tct cac agc gga gtc tct       246
Gly Ala Asp Gln Leu Cys Arg Gln Thr Ser Ser His Ser Gly Val Ser
55                  60                  65                  70

tct ttc tca agc ggg agg gtg gtg aaa aga gag aga gac ggt ggc gaa     294
Ser Phe Ser Ser Gly Arg Val Val Lys Arg Glu Arg Asp Gly Gly Glu
                75                  80                  85

gag tcg ccg gag gag gaa gag atg acg gag aga gtt ata agt gat tac     342
Glu Ser Pro Glu Glu Glu Glu Met Thr Glu Arg Val Ile Ser Asp Tyr
            90                  95                  100

cat gaa gat gaa gaa ggt att agt gct aga aaa aaa ctt agg ctt acg     390
His Glu Asp Glu Glu Gly Ile Ser Ala Arg Lys Lys Leu Arg Leu Thr
        105                 110                 115

aaa caa caa tct gct ctt ctt gag gaa agc ttc aag gat cat agc acc     438
Lys Gln Gln Ser Ala Leu Leu Glu Glu Ser Phe Lys Asp His Ser Thr
    120                 125                 130

ctt aat ccc aaa caa aag caa gtt ctg gct aga cag ctg aat cta agg     486
Leu Asn Pro Lys Gln Lys Gln Val Leu Ala Arg Gln Leu Asn Leu Arg
135                 140                 145                 150

cct aga caa gtt gaa gta tgg ttt caa aat aga aga gcc agg aca aag     534
Pro Arg Gln Val Glu Val Trp Phe Gln Asn Arg Arg Ala Arg Thr Lys
                155                 160                 165

ctg aag caa aca gaa gta gat tgt gag ttt ttg aag aag tgt tgt gaa     582
Leu Lys Gln Thr Glu Val Asp Cys Glu Phe Leu Lys Lys Cys Cys Glu
            170                 175                 180

aca tta gca gat gag aac ata aga ctt cag aaa gag att caa gaa ctc     630
Thr Leu Ala Asp Glu Asn Ile Arg Leu Gln Lys Glu Ile Gln Glu Leu
        185                 190                 195

aaa acc cta aaa ttg act cag ccc ttt tac atg cac atg cct gca tcg     678
Lys Thr Leu Lys Leu Thr Gln Pro Phe Tyr Met His Met Pro Ala Ser
    200                 205                 210

act cta acg aag tgt cct tct tgt gag aga atc ggc ggc ggc ggc ggg     726
Thr Leu Thr Lys Cys Pro Ser Cys Glu Arg Ile Gly Gly Gly Gly Gly
215                 220                 225                 230

ggt aat gga gga gga ggt ggc ggc agc ggg gct acc gcg gtg att gta     774
Gly Asn Gly Gly Gly Gly Gly Gly Ser Gly Ala Thr Ala Val Ile Val
                235                 240                 245

gat gga agt acg gcc aaa gga gct ttc tct atc tcc tca aag cct cac     822
Asp Gly Ser Thr Ala Lys Gly Ala Phe Ser Ile Ser Ser Lys Pro His
            250                 255                 260

ttc ttc aac cct ttt act aac cca tct gca gct tgt tga atagttaatt     871
Phe Phe Asn Pro Phe Thr Asn Pro Ser Ala Ala Cys
        265                 270

cgtttaattt tattacttaa aatattaatt ttcttttttt ttttgggtgg catttt     927

<210>  54

<211>  274

<212>  PRT

<213>  Arabidopsis thaliana

<400>  54

Met Gly Phe Asp Asp Thr Cys Asn Thr Gly Leu Val Leu Gly Leu Gly
1               5                   10                  15

Pro Ser Pro Ile Ser Asn Asn Tyr Asn Ser Thr Ile Arg Gln Ser Ser
            20                  25                  30

Val Tyr Lys Leu Glu Pro Ser Leu Thr Leu Cys Leu Ser Gly Asp Pro
        35                  40                  45

Ser Val Thr Val Val Thr Gly Ala Asp Gln Leu Cys Arg Gln Thr Ser
    50                  55                  60

Ser His Ser Gly Val Ser Ser Phe Ser Ser Gly Arg Val Val Lys Arg
65                  70                  75                  80

Glu Arg Asp Gly Gly Glu Glu Ser Pro Glu Glu Glu Glu Met Thr Glu
                85                  90                  95

Arg Val Ile Ser Asp Tyr His Glu Asp Glu Glu Gly Ile Ser Ala Arg
            100                 105                 110

Lys Lys Leu Arg Leu Thr Lys Gln Gln Ser Ala Leu Leu Glu Glu Ser
        115                 120                 125

Phe Lys Asp His Ser Thr Leu Asn Pro Lys Gln Lys Gln Val Leu Ala
    130                 135                 140

Arg Gln Leu Asn Leu Arg Pro Arg Gln Val Glu Val Trp Phe Gln Asn
145                 150                 155                 160

Arg Arg Ala Arg Thr Lys Leu Lys Gln Thr Glu Val Asp Cys Glu Phe
                165                 170                 175

Leu Lys Lys Cys Cys Glu Thr Leu Ala Asp Glu Asn Ile Arg Leu Gln
            180                 185                 190

Lys Glu Ile Gln Glu Leu Lys Thr Leu Lys Leu Thr Gln Pro Phe Tyr
        195                 200                 205

Met His Met Pro Ala Ser Thr Leu Thr Lys Cys Pro Ser Cys Glu Arg
    210                 215                 220

Ile Gly Gly Gly Gly Gly Gly Asn Gly Gly Gly Gly Gly Gly Ser Gly
225                 230                 235                 240

Ala Thr Ala Val Ile Val Asp Gly Ser Thr Ala Lys Gly Ala Phe Ser
                245                 250                 255

Ile Ser Ser Lys Pro His Phe Phe Asn Pro Phe Thr Asn Pro Ser Ala
            260                 265                 270

Ala Cys

## Description

### RELATED APPLICATION INFORMATION

The present invention claims the benefit from US Provisional Patent Application Serial Nos. 60/166,228 filed November 17, 1999 and 60/197,899 filed April 17, 2000 and "Plant Trait Modification III" filed August 22, 2000.

### FIELD OF THE INVENTION

This invention relates to the field of plant biology. More particularly, the present invention pertains to compositions and methods for phenotypically modifying a plant.

### BACKGROUND OF THE INVENTION

Transcription factors can modulate gene expression, either increasing or decreasing (inducing or repressing) the rate of transcription. This modulation results in differential levels of gene expression at various developmental stages, in different tissues and cell types, and in response to different exogenous (e.g., environmental) and endogenous stimuli throughout the life cycle of the organism.

Because transcription factors are key controlling elements of biological pathways, altering the expression levels of one or more transcription factors can change entire biological pathways in an organism. For example, manipulation of the levels of selected transcription factors may result in increased expression of economically useful proteins or metabolic chemicals in plants or to improve other agriculturally relevant characteristics. Conversely, blocked or reduced expression of a transcription factor may reduce biosynthesis of unwanted compounds or remove an undesirable trait. Therefore, manipulating transcription factor levels in a plant offers tremendous potential in agricultural biotechnology for modifying a plant's traits.

The present invention provides novel transcription factors useful for modifying a plant's phenotype in desirable ways, such as modifying a plant's environmental stress tolerance.

### SUMMARY OF THE INVENTION

In a first aspect, the invention relates to a recombinant polynucleotide comprising a nucleotide sequence selected from the group consisting of: (a) a nucleotide sequence encoding a polypeptide comprising a sequence selected from SEQ ID Nos. 2N, where N=1-27, or a complementary nucleotide sequence thereof; (b) a nucleotide sequence encoding a polypeptide comprising a conservatively substituted variant of a polypeptide of (a); (c) a nucleotide sequence comprising a sequence selected from those of SEQ ID Nos. 2N-1, where N=1-27, or a complementary nucleotide sequence thereof; (d) a nucleotide sequence comprising silent substitutions in a nucleotide sequence of (c); (e) a nucleotide sequence which hybridizes under stringent conditions over substantially the entire length of a nucleotide sequence of one or more of: (a), (b), (c), or (d); (f) a nucleotide sequence comprising at least 15 consecutive nucleotides of a sequence of any of (a)-(e); (g) a nucleotide sequence comprising a subsequence or fragment of any of (a)-(f), which subsequence or fragment encodes a polypeptide having a biological activity that modifies a plant's environmental stress tolerance; (h) a nucleotide sequence having at least 30% sequence identity to a nucleotide sequence of any of (a)-(g); (i) a nucleotide sequence having at least 60% identity sequence identity to a nucleotide sequence of any of (a)-(g); (j) a nucleotide sequence which encodes a polypeptide having at least 30% identity sequence identity to a polypeptide of SEQ ID Nos. 2N, where N=1-27; (k) a nucleotide sequence which encodes a polypeptide having at least 60% identity sequence identity to a polypeptide of SEQ ID Nos. 2N, where N=1-27; and (1) a nucleotide sequence which encodes a conserved domain of a polypeptide having at least 65% sequence identity to a conserved domain of a polypeptide of SEQ ID Nos. 2N, where N=1-27. The recombinant polynucleotide may further comprise a constitutive, inducible, or tissue-active promoter operably linked to the nucleotide sequence. The invention also relates to compositions comprising at least two of the above described polynucleotides.

In a second aspect, the invention is an isolated or recombinant polypeptide comprising a subsequence of at least about 15 contiguous amino acids encoded by the recombinant or isolated polynucleotide described above.
In another aspect, the invention is a transgenic plant comprising one or more of the above described recombinant polynucleotides. In yet another aspect, the invention is a plant with altered expression levels of a polynucleotide described above or a plant with altered expression or activity levels of an above described polypeptide. Further, the invention may be a plant lacking a nucleotide sequence encoding a polypeptide comprising a sequence selected from SEQ ID Nos. 2N, where N=1-27.

The plant may be a soybean, wheat, corn, potato, cotton, rice, oilseed rape, sunflower, alfalfa, sugarcane, turf, banana, blackberry, blueberry, strawberry, raspberry, cantaloupe, carrot, cauliflower, coffee, cucumber, eggplant, grapes, honeydew, lettuce, mango, melon, onion, papaya, peas, peppers, pineapple, spinach, squash, sweet corn, tobacco, tomato, watermelon, rosaceous fruits, or vegetable brassicas plant.

In a further aspect, the invention relates to a cloning or expression vector comprising the isolated or recombinant polynucleotide described above or cells comprising the cloning or expression vector.

In yet a further aspect, the invention relates to a composition produced by incubating a polynucleotide of the invention with a nuclease, a restriction enzyme, a polymerase; a polymerase and a primer; a cloning vector, or with a cell.

Furthermore, the invention relates to a method for producing a plant having improved environmental stress tolerance. The method comprises altering the expression of an isolated or recombinant polynucleotide of the invention or altering the expression or activity of a polypeptide of the invention in a plant to produce a modified plant, and selecting the modified plant for modified environmental stress tolerance.

In another aspect, the invention relates to a method of identifying a factor that is modulated by or interacts with a polypeptide encoded by a polynucleotide of the invention. The method comprises expressing a polypeptide encoded by the polynucleotide in a plant; and identifying at least one factor that is modulated by or interacts with the polypeptide. In one embodiment the method for identifying modulating or interacting factors is by detecting binding by the polypeptide to a promoter sequence, or by detecting interactions between an additional protein and the polypeptide in a yeast two hybrid system, or by detecting expression of a factor by hybridization to a microarray, subtractive hybridization or differential display.

In yet another aspect, the invention is a method of identifying a molecule that modulates activity or expression of a polynucleotide or polypeptide of interest. The method comprises placing the molecule in contact with a plant comprising the polynucleotide or polypeptide encoded by the polynucleotide of the invention and monitoring one or more of the expression level of the polynucleotide in the plant, the expression level of the polypeptide in the plant, and modulation of an activity of the polypeptide in the plant.

In yet another aspect, the invention relates to an integrated system, computer or computer readable medium comprising one or more character strings corresponding to a polynucleotide of the invention, or to a polypeptide encoded by the polynucleotide. The integrated system, computer or computer readable medium may comprise a link between one or more sequence strings to a modified plant environmental stress tolerance phenotype.

In yet another aspect, the invention is a method for identifying a sequence similar or homologous to one or more polynucleotides of the invention, or one or more polypeptides encoded by the polynucleotides. The method comprises providing a sequence database; and, querying the sequence database with one or more target sequences corresponding to the one or more polynucleotides or to the one or more polypeptides to identify one or more sequence members of the database that display sequence similarity or homology to one or more of the one or more target sequences.

The method may further comprise of linking the one or more of the polynucleotides of the invention, or encoded polypeptides, to a modified plant environmental stress tolerance phenotype.

### BRIEF DESCRIPTION OF THE DRAWINGS

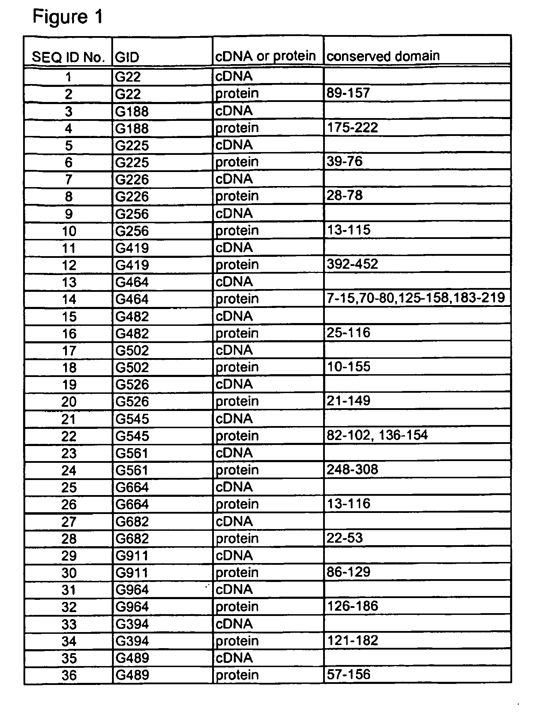

Figure 1 provides a table of exemplary polynucleotide and polypeptide sequences of the invention. The table includes from left to right for each sequence: the SEQ ID No., the internal code reference number (GID), whether the sequence is a polynucleotide or polypeptide sequence, and identification of any conserved domains for the polypeptide sequences.

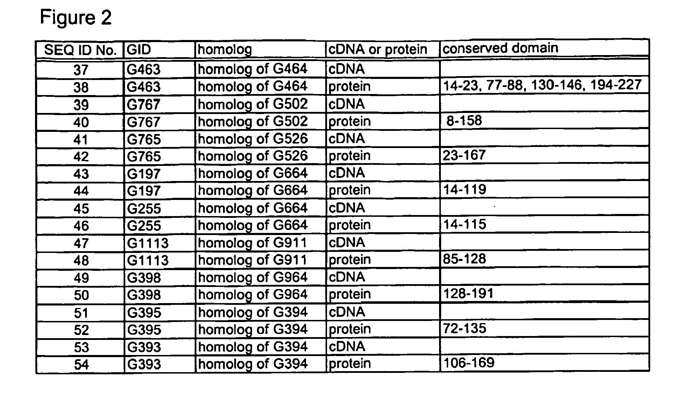

Figure 2 provides a table of exemplary sequences that are homologous to other sequences provided in the Sequence Listing and that are derived from *Arabidopsis thaliana*. The table includes from left to right: the SEQ ID No., the internal code reference number (GID), identification of the homologous sequence, whether the sequence is a polynucleotide or polypeptide sequence, and identification of any conserved domains for the polypeptide sequences.

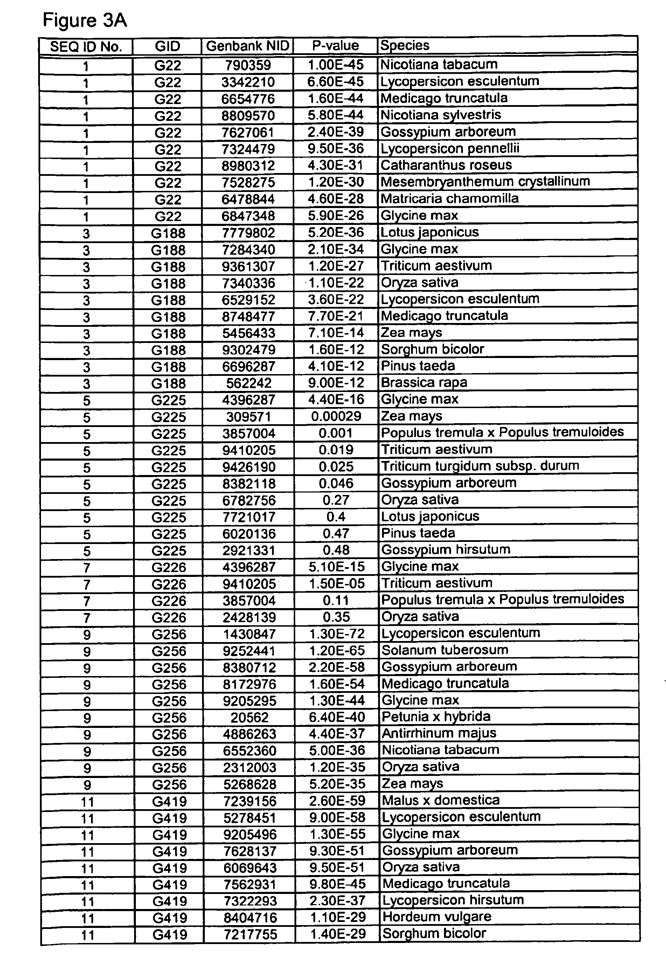

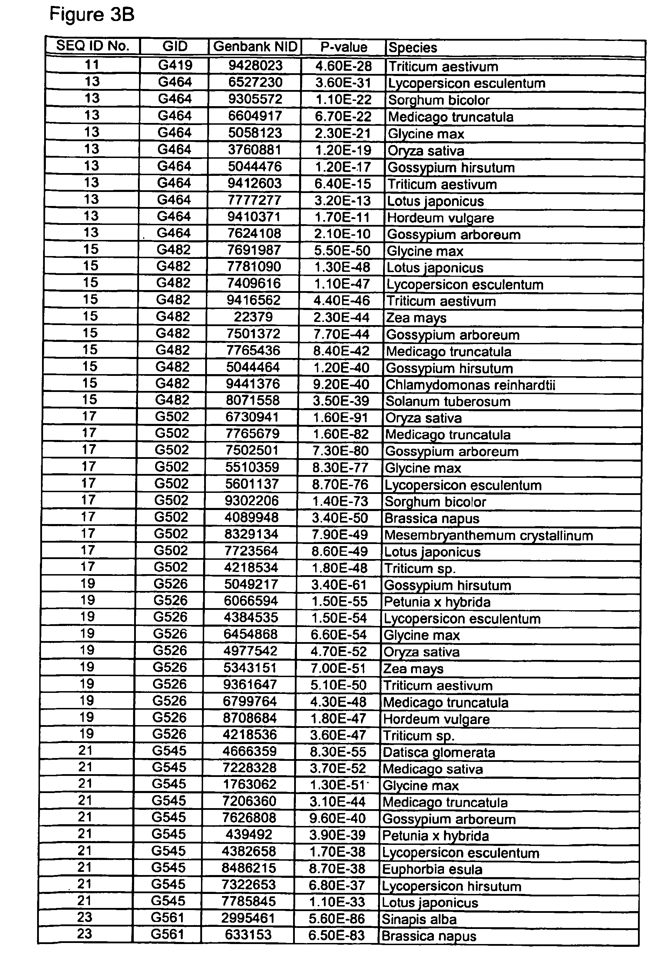

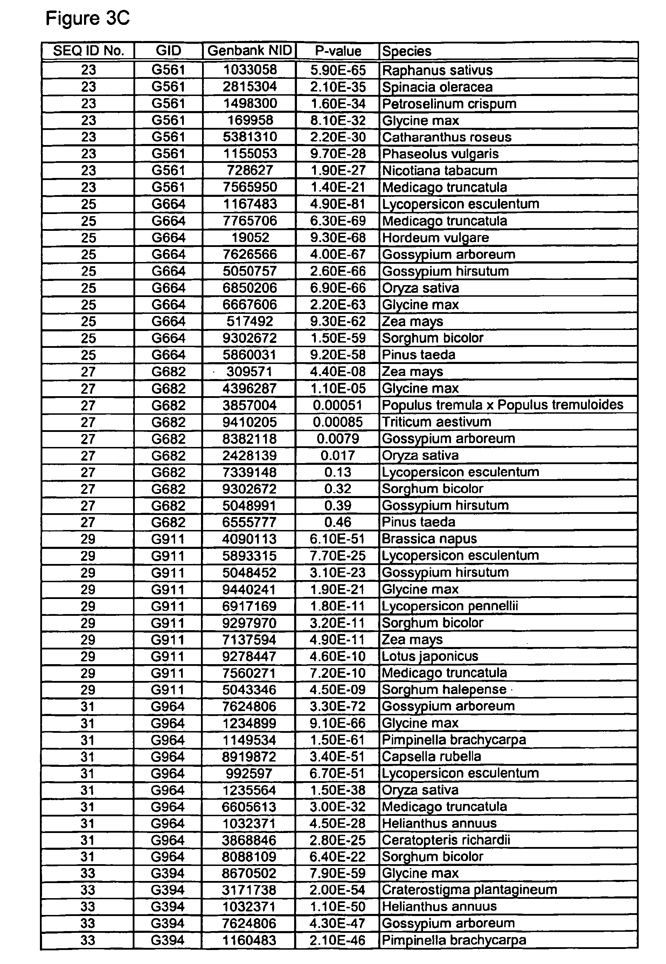

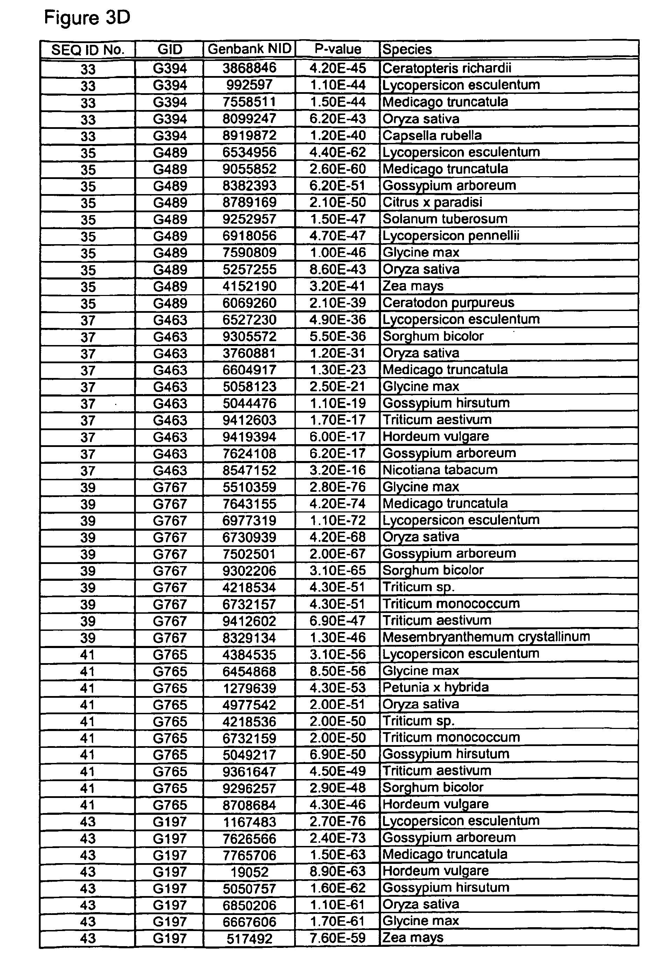

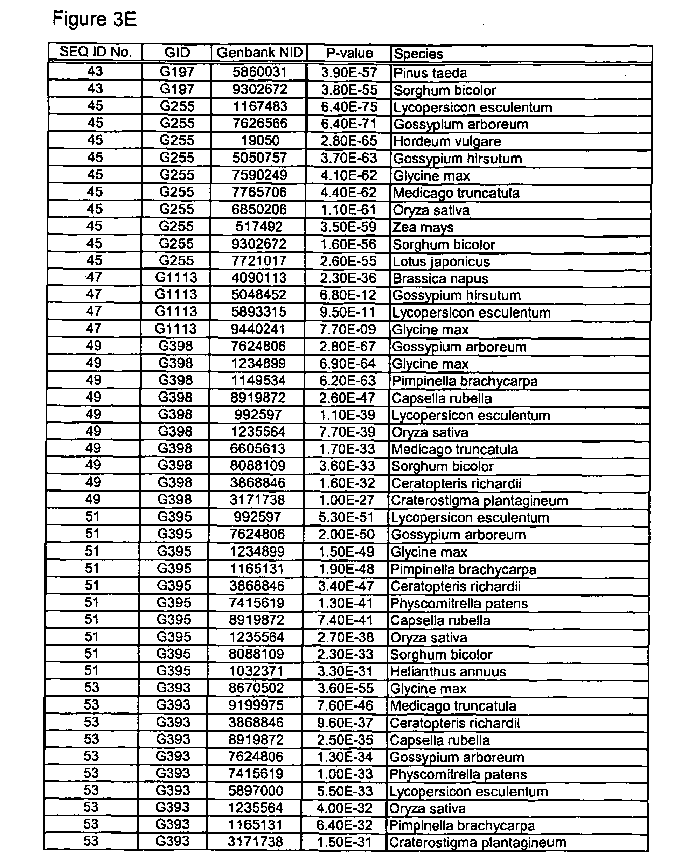

Figure 3 provides a table of exemplary sequences that are homologous to the sequences provided in Figures 1 and 2 and that are derived from plants other than *Arabidopsis thaliana.* The table includes from left to right: the SEQ ID No., the internal code reference number (GID), the unique GenBank sequence ID No. (NID), the probability that the comparison was generated by chance (P-value), and the species from which the homologous gene was identified.

### DETAILED DESCRIPTION

The present invention relates to polynucleotides and polypeptides, e.g. for modifying phenotypes of plants.

In particular, the polynucleotides or polypeptides are useful for modifying traits associated with a plant's environmental stress tolerance when the expression levels of the polynucleotides or expression levels or activity levels of the polypeptides are altered. Specifically, the polynucleotides and polypeptides are useful for modifying traits associated with a plant's environmental stress tolerance, such as freezing, chilling, heat, drought, water saturation, salt, photoconditions, radiation and ozone, or the like. Plants with altered expression of the polynucleotides or polypeptides of the invention are more tolerant to these environmental stresses compared with plants without altered expression levels.

The polynucleotides of the invention encode plant transcription factors. The plant transcription factors are derived, e.g., from *Arabidopsis thaliana* and can belong, e.g., to one or more of the following transcription factor families: the AP2 (APETALA2) domain transcription factor family (Riechmann and Meyerowitz (1998) J. Biol. Chem. 379:633-646); the MYB transcription factor family (Martin and Paz-Ares (1997) Trends Genet. 13:67-73); the MADS domain transcription factor family (Riechmann and Meyerowitz (1997) J. Biol. Chem. 378:1079-1101); the WRKY protein family (Ishiguro and Nakamura (1994) Mol. Gen. Genet. 244:563-571); the ankyrin-repeat protein family (Zhang et al. (1992) Plant Cell 4:1575-1588); the miscellaneous protein (MISC) family (Kim et al. (1997) Plant J. 11:1237-1251); the zinc finger protein (Z) family (Klug and Schwabe (1995) FASEB J. 9: 597-604); the homeobox (HB) protein family (Duboule (1994) Guidebook to the Homeobox Genes. Oxford University Press); the CAAT-element binding proteins (Forsburg and Guarente (1989) Genes Dev. 3:1166-1178); the squamosa promoter binding proteins (SPB) (Klein et al. (1996) Mol. Gen. Genet. 1996 250:7-16); the NAM protein family; the IAA/AUX proteins (Rouse et al. (1998) Science 279:1371-1373); the HLH/MYC protein family (Littlewood et al. (1994) Prot. Profile 1:639-709); the DNA-binding protein (DBP) family (Tucker et al. (1994) EMBO J. 13:2994-3002); the bZIP family of transcription factors (Foster et al. (1994) FASEB J. 8:192-200); the BPF-1 protein (Box P-binding factor) family (da Costa e Silva et al. (1993) Plant J. 4:125-135); and the golden protein (GLD) family (Hall et al. (1998) Plant Cell 10:925-936).

In addition to methods for modifying a plant phenotype by employing one or more polynucleotides and polypeptides of the invention described herein, the polynucleotides and polypeptides of the invention have a variety of additional uses. These uses include their use in the recombinant production (i.e, expression) of proteins; as regulators of plant gene expression, as diagnostic probes for the presence of complementary or partially complementary nucleic acids (including for detection of natural coding nucleic acids); as substrates for further reactions, e.g., mutation reactions, PCR reactions, or the like, of as substrates for cloning e.g., including digestion or ligation reactions, and for identifying exogenous or endogenous modulators of the transcription factors.

### DEFINITIONS

A "polynucleotide" is a nucleic acid sequence comprising a plurality of polymerized nucleotide residues, e.g., at least about 15 consecutive polymerized nucleotide residues, optionally at least about 30 consecutive nucleotides, at least about 50 consecutive nucleotides. In many instances, a polynucleotide comprises a nucleotide sequence encoding a polypeptide (or protein) or a domain or fragment thereof. Additionally, the polynucleotide may comprise a promoter, an intron, an enhancer region, a polyadenylation site, a translation initiation site, 5' or 3' untranslated regions, a reporter gene, a selectable marker, or the like. The polynucleotide can be single stranded or double stranded DNA or RNA. The polynucleotide optionally comprises modified bases or a modified backbone. The polynucleotide can be, e.g., genomic DNA or RNA, a transcript (such as an mRNA), a cDNA, a PCR product, a cloned DNA, a synthetic DNA or RNA, or the like. The polynucleotide can comprise a sequence in either sense or antisense orientations.

A "recombinant polynucleotide" is a polynucleotide that is not in its native state, e.g., the polynucleotide comprises a nucleotide sequence not found in nature, or the polynucleotide is in a context other than that in which it is naturally found, e.g., separated from nucleotide sequences with which it typically is in proximity in nature, or adjacent (or contiguous with) nucleotide sequences with which it typically is not in proximity. For example, the sequence at issue can be cloned into a vector, or otherwise recombined with one or more additional nucleic acid.

An "isolated polynucleotide" is a polynucleotide whether naturally occurring or recombinant, that is present outside the cell in which it is typically found in nature, whether purified or not. Optionally, an isolated polynucleotide is subject to one or more enrichment or purification procedures, e.g., cell lysis, extraction, centrifugation, precipitation, or the like.

A "recombinant polypeptide" is a polypeptide produced by translation of a recombinant polynucleotide. An "isolated polypeptide," whether a naturally occurring or a recombinant polypeptide, is more enriched in (or out of) a cell than the polypeptide in its natural state in a wild type cell, e.g., more than about 5% enriched, more than about 10% enriched, or more than about 20%, or more than about 50%, or more, enriched, i.e., alternatively denoted: 105%, 110%, 120%, 150% or more, enriched relative to wild type standardized at 100%. Such an enrichment is not the result of a natural response of a wild type plant. Alternatively, or additionally, the isolated polypeptide is separated from other cellular components with which it is typically associated, e.g., by any of the various protein purification methods herein.

The term "transgenic plant" refers to a plant that contains genetic material, not found in a wild type plant of the same species, variety or cultivar. The genetic material may include a transgene, an insertional mutagenesis event (such as by transposon or T-DNA insertional mutagenesis), an activation tagging sequence, a mutated sequence, a homologous recombination event or a sequence modified by chimeraplasty. Typically, the foreign genetic material has been introduced into the plant by human manipulation.

A transgenic plant may contain an expression vector or cassette. The expression cassette typically comprises a polypeptide-encoding sequence operably linked (i.e., under regulatory control of) to appropriate inducible or constitutive regulatory sequences that allow for the expression of polypeptide. The expression cassette can be introduced into a plant by transformation or by breeding after transformation of a parent plant. A plant refers to a whole plant as well as to a plant part, such as seed, fruit, leaf, or root, plant tissue, plant cells or any other plant material, e.g., a plant explant, as well as to progeny thereof, and to *in vitro* systems that mimic biochemical or cellular components or processes in a cell.

The phrase "ectopically expression or altered expression" in reference to a polynucleotide indicates that the pattern of expression in, e.g., a transgenic plant or plant tissue, is different from the expression pattern in a wild type plant or a reference plant of the same species. For example, the polynucleotide or polypeptide is expressed in a cell or tissue type other than a cell or tissue type in which the sequence is expressed in the wild type plant, or by expression at a time other than at the time the sequence is expressed in the wild type plant, or by a response to different inducible agents, such as hormones or environmental signals, or at different expression levels (either higher or lower) compared with those found in a wild type plant. The term also refers to altered expression patterns that are produced by lowering the levels of expression to below the detection level or completely abolishing expression. The resulting expression pattern can be transient or stable, constitutive or inducible. In reference to a polypeptide, the term "ectopic expression or altered expression" further may relate to altered activity levels resulting from the interactions of the polypeptides with exogenous or endogenous modulators or from interactions with factors or as a result of the chemical modification of the polypeptides.

The term "fragment" or "domain," with respect to a polypeptide, refers to a subsequence of the polypeptide. In some cases, the fragment or domain, is a subsequence of the polypeptide which performs at least one biological function of the intact polypeptide in substantially the same manner, or to a similar extent, as does the intact polypeptide. For example, a polypeptide fragment can comprise a recognizable structural motif or functional domain such as a DNA binding domain that binds to a DNA promoter region, an activation domain or a domain for protein-protein interactions. Fragments can vary in size from as few as 6 amino acids to the full length of the intact polypeptide, but are preferably at least about 30 amino acids in length and more preferably at least about 60 amino acids in length. In reference to a nucleotide sequence, "a fragment" refers to any subsequence of a polynucleotide, typically, of at least consecutive about 15 nucleotides, preferably at least about 30 nucleotides, more preferably at least about 50, of any of the sequences provided herein.

The term "trait" refers to a physiological, morphological, biochemical or physical characteristic of a plant or particular plant material or cell. In some instances, this characteristic is visible to the human eye, such as seed or plant size, or can be measured by available biochemical techniques, such as the protein, starch or oil content of seed or leaves or by the observation of the expression level of genes, e.g., by employing Northern analysis, RT-PCR, microarray gene expression assays or reporter gene expression systems, or by agricultural observations such as stress tolerance, yield or pathogen tolerance.

"Trait modification" refers to a detectable difference in a characteristic in a plant ectopically expressing a polynucleotide or polypeptide of the present invention relative to a plant not doing so, such as a wild type plant. In some cases, the trait modification can be evaluated quantitatively. For example, the trait modification can entail at least about a 2% increase or decrease in an observed trait (difference), at least a 5% difference, at least about a 10% difference, at least about a 20% difference, at least about a 30%, at least about a 50%, at least about a 70%, or at least about a 100%, or an even greater difference. It is known that there can be a natural variation in the modified trait. Therefore, the trait modification observed entails a change of the normal distribution of the trait in the plants compared with the distribution observed in wild type plant.

Trait modifications of particular interest include those to seed ( such as embryo or endosperm), fruit, root, flower, leaf, stem, shoot, seedling or the like, including: enhanced tolerance to environmental conditions including freezing, chilling, heat, drought, water saturation, radiation and ozone; improved tolerance to microbial, fungal or viral diseases; improved tolerance to pest infestations, including nematodes, mollicutes, parasitic higher plants or the like; decreased herbicide sensitivity; improved tolerance of heavy metals or enhanced ability to take up heavy metals; improved growth under poor photoconditions (e.g., low light and/or short day length), or changes in expression levels of genes of interest. Other phenotype that can be modified relate to the production of plant metabolites, such as variations in the production of taxol, tocopherol, tocotrienol, sterols, phytosterols, vitamins, wax monomers, anti-oxidants, amino acids, lignins, cellulose, tannins, prenyllipids (such as chlorophylls and carotenoids), glucosinolates, and terpenoids, enhanced or compositionally altered protein or oil production (especially in seeds), or modified sugar (insoluble or soluble) and/or starch composition. Physical plant characteristics that can be modified include cell development (such as the number of trichomes), fruit and seed size and number, yields of plant parts such as stems, leaves and roots, the stability of the seeds during storage, characteristics of the seed pod (e.g., susceptibility to shattering), root hair length and quantity, internode distances, or the quality of seed coat. Plant growth characteristics that can be modified include growth rate, germination rate of seeds, vigor of plants and seedlings, leaf and flower senescence, male sterility, apomixis, flowering time, flower abscission, rate of nitrogen uptake, biomass or transpiration characteristics, as well as plant architecture characteristics such as apical dominance, branching patterns, number of organs, organ identity, organ shape or size.

### POLYPEPTIDES AND POLYNUCLEOTIDES OF THE INVENTION

The present invention provides, among other things, transcription factors (TFs), and transcription factor homologue polypeptides, and isolated or recombinant polynucleotides encoding the polypeptides. These polypeptides and polynucleotides may be employed to modify a plant's environmental stress tolerance.

Exemplary polynucleotides encoding the polypeptides of the invention were identified in the *Arabidopsis thaliana* GenBank database using publicly available sequence analysis programs and parameters. Sequences initially identified were then further characterized to identify sequences comprising specified sequence strings corresponding to sequence motifs present in families of known transcription factors. Polynucleotide sequences meeting such criteria were confirmed as transcription factors.

Additional polynucleotides of the invention were identified by screening *Arabidopsis thaliana* and/or other plant cDNA libraries with probes corresponding to known transcription factors under low stringency hybridization conditions. Additional sequences, including full length coding sequences were subsequently recovered by the rapid amplification of cDNA ends (RACE) procedure, using a commercially available kit according to the manufacturer's instructions. Where necessary, multiple rounds of RACE are performed to isolate 5' and 3' ends. The full length cDNA was then recovered by a routine end-to-end polymerase chain reaction (PCR) using primers specific to the isolated 5' and 3' ends. Exemplary sequences are provided in the Sequence Listing.

The polynucleotides of the invention were ectopically expressed in overexpressor or knockout plants and changes in the environmental stress tolerance of the plants was observed. Therefore, the polynucleotides and polypeptides can be employed to improve the environmental stress resistance of plants.

### Making polynucleotides

The polynucleotides of the invention include sequences that encode transcription factors and transcription factor homologue polypeptides and sequences complementary thereto, as well as unique fragments of coding sequence, or sequence complementary thereto. Such polynucleotides can be, e.g., DNA or RNA, e.g., mRNA, cRNA, synthetic RNA, genomic DNA, cDNA synthetic DNA, oligonucleotides, etc. The polynucleotides are either double-stranded or single-stranded, and include either, or both sense (i.e., coding) sequences and antisense (i.e., non-coding, complementary) sequences. The polynucleotides include the coding sequence of a transcription factor, or transcription factor homologue polypeptide, in isolation, in combination with additional coding sequences (e.g., a purification tag, a localization signal, as a fusion-protein, as a pre-protein, or the like), in combination with non-coding sequences (e.g., introns or inteins, regulatory elements such as promoters, enhancers, terminators, and the like), and/or in a vector or host environment in which the polynucleotide encoding a transcription factor or transcription factor homologue polypeptide is an endogenous or exogenous gene.

A variety of methods exist for producing the polynucleotides of the invention. Procedures for identifying and isolating DNA clones are well known to those of skill in the art, and are described in, e.g., Berger and Kimmel, Guide to Molecular Cloning Techniques, Methods in Enzymology volume 152 Academic Press, Inc., San Diego, CA ("Berger"); Sambrook et al., Molecular Cloning - A Laboratory Manual (2nd Ed.), Vol. 1-3, Cold Spring Harbor Laboratory, Cold Spring Harbor, New York, 1989 ("Sambrook") and Current Protocols in Molecular Biology, F.M. Ausubel et al., eds., Current Protocols, a joint venture between Greene Publishing Associates, Inc. and John Wiley & Sons, Inc., (supplemented through 2000) ("Ausubel").

Alternatively, polynucleotides of the invention, can be produced by a variety of in vitro amplification methods adapted to the present invention by appropriate selection of specific or degenerate primers. Examples of protocols sufficient to direct persons of skill through in vitro amplification methods, including the polymerase chain reaction (PCR) the ligase chain reaction (LCR), Qbeta-replicase amplification and other RNA polymerase mediated techniques (e.g., NASBA), e.g., for the production of the homologous nucleic acids of the invention are found in Berger, Sambrook, and Ausubel, as well as Mullis et al., (1987) PCR Protocols A Guide to Methods and Applications (Innis et al. eds) Academic Press Inc. San Diego, CA (1990) (Innis). Improved methods for cloning in vitro amplified nucleic acids are described in Wallace et al., U.S. Pat. No. 5,426,039. Improved methods for amplifying large nucleic acids by PCR are summarized in Cheng et al. (1994) Nature 369: 684-685 and the references cited therein, in which PCR amplicons of up to 40kb are generated. One of skill will appreciate that essentially any RNA can be converted into a double stranded DNA suitable for restriction digestion, PCR expansion and sequencing using reverse transcriptase and a polymerase. *See*, e.g., Ausubel, Sambrook and Berger, *all supra*.

Alternatively, polynucleotides and oligonucleotides of the invention can be assembled from fragments produced by solid-phase synthesis methods. Typically, fragments of up to approximately 100 bases are individually synthesized and then enzymatically or chemically ligated to produce a desired sequence, e.g., a polynucletotide encoding all or part of a transcription factor. For example, chemical synthesis using the phosphoramidite method is described, e.g., by Beaucage et al. (1981) Tetrahedron Letters 22:1859-69; and Matthes et al. (1984) EMBO J. 3:801-5. According to such methods, oligonucleotides are synthesized, purified, annealed to their complementary strand, ligated and then optionally cloned into suitable vectors. And if so desired, the polynucleotides and polypeptides of the invention can be custom ordered from any of a number of commercial suppliers.

### HOMOLOGOUS SEQUENCES

Sequences homologous, i.e., that share significant sequence identity or similarity, to those provided in the Sequence Listing, derived from *Arabidopsis thaliana* or from other plants of choice are also an aspect of the invention. Homologous sequences can be derived from any plant including monocots and dicots and in particular agriculturally important plant species, including but not limited to, crops such as soybean, wheat, corn, potato, cotton, rice, oilseed rape (including canola), sunflower, alfalfa, sugarcane and turf; or fruits and vegetables, such as banana, blackberry, blueberry, strawberry, and raspberry, cantaloupe, carrot, cauliflower, coffee, cucumber, eggplant, grapes, honeydew, lettuce, mango, melon, onion, papaya, peas, peppers, pineapple, spinach, squash, sweet corn, tobacco, tomato, watermelon, rosaceous fruits (such as apple, peach, pear, cherry and plum) and vegetable brassicas (such as broccoli, cabbage, cauliflower, brussel sprouts and kohlrabi). Other crops, fruits and vegetables whose phenotype can be changed include barley, rye, millet, sorghum, currant, avocado, citrus fruits such as oranges, lemons, grapefruit and tangerines, artichoke, cherries, nuts such as the walnut and peanut, endive, leek, roots, such as arrowroot, beet, cassava, turnip, radish, yam, and sweet potato, and beans. The homologous sequences may also be derived from woody species, such pine, poplar and eucalyptus.

Transcription factors that are homologous to the listed sequences will typically share at least about 30% amino acid sequence identity. More closely related transcription factors can share at least about 50%, about 60%, about 65%, about 70%, about 75% or about 80% or about 90% or about 95% or about 98% or more sequence identity with the listed sequences. Factors that are most closely related to the listed sequences share, e.g., at least about 85%, about 90% or about 95% or more % sequence identity to the listed sequences. At the nucleotide level, the sequences will typically share at least about 40% nucleotide sequence identity, preferably at least about 50%, about 60%, about 70% or about 80% sequence identity, and more preferably about 85%, about 90%, about 95% or about 97% or more sequence identity to one or more of the listed sequences. The degeneracy of the genetic code enables major variations in the nucleotide sequence of a polynucleotide while maintaining the amino acid sequence of the encoded protein. Conserved domains within a transcription factor family may exhibit a higher degree of sequence homology, such as at least 65% sequence identity including conservative substitutions, and preferably at least 80% sequence identity.

### Identifying Nucleic Acids by Hybridization

Polynucleotides homologous to the sequences illustrated in the Sequence Listing can be identified, e.g., by hybridization to each other under stringent or under highly stringent conditions. Single stranded polynucleotides hybridize when they associate based on a variety of well characterized physico-chemical forces, such as hydrogen bonding, solvent exclusion, base stacking and the like. The stringency of a hybridization reflects the degree of sequence identity of the nucleic acids involved, such that the higher the stringency, the more similar are the two polynucleotide strands. Stringency is influenced by a variety of factors, including temperature, salt concentration and composition, organic and non-organic additives, solvents, etc. present in both the hybridization and wash solutions and incubations (and number), as described in more detail in the references cited above.

An example of stringent hybridization conditions for hybridization of complementary nucleic acids which have more than 100 complementary residues on a filter in a Southern or northern blot is about 5°C to 20°C lower than the thermal melting point (Tm) for the specific sequence at a defined ionic strength and pH. The Tₘ is the temperature (under defined ionic strength and pH) at which 50% of the target sequence hybridizes to a perfectly matched probe. Nucleic acid molecules that hybridize under stringent conditions will typically hybridize to a probe based on either the entire cDNA or selected portions, e.g., to a unique subsequence, of the cDNA under wash conditions of 0.2x SSC to 2.0 x SSC, 0.1% SDS at 50-65° C, for example 0.2 x SSC, 0.1% SDS at 65° C. For identification of less closely related homologues washes can be performed at a lower temperature, e.g., 50° C. In general, stringency is increased by raising the wash temperature and/or decreasing the concentration of SSC.

As another example, stringent conditions can be selected such that an oligonucleotide that is perfectly complementary to the coding oligonucleotide hybridizes to the coding oligonucleotide with at least about a 5-10x higher signal to noise ratio than the ratio for hybridization of the perfectly complementary oligonucleotide to a nucleic acid encoding a transcription factor known as of the filing date of the application. Conditions can be selected such that a higher signal to noise ratio is observed in the particular assay which is used, e.g., about 15x, 25x, 35x, 50x or more. Accordingly, the subject nucleic acid hybridizes to the unique coding oligonucleotide with at least a 2x higher signal to noise ratio as compared to hybridization of the coding oligonucleotide to a nucleic acid encoding known polypeptide. Again, higher signal to noise ratios can be selected, e.g., about 5x, 10x, 25x, 35x, 50x or more. The particular signal will depend on the label used in the relevant assay, e.g., a fluorescent label, a colorimetric label, a radio active label, or the like.

Alternatively, transcription factor homologue polypeptides can be obtained by screening an expression library using antibodies specific for one or more transcription factors. With the provision herein of the disclosed transcription factor, and transcription factor homologue nucleic acid sequences, the encoded polypeptide(s) can be expressed and purified in a heterologous expression system (e.g., *E. coli*) and used to raise antibodies (monoclonal or polyclonal) specific for the polypeptide(s) in question. Antibodies can also be raised against synthetic peptides derived from transcription factor, or transcription factor homologue, amino acid sequences. Methods of raising antibodies are well known in the art and are described in Harlow and Lane (1988) Antibodies: A Laboratory Manual, Cold Spring Harbor Laboratory, New York. Such antibodies can then be used to screen an expression library produced from the plant from which it is desired to clone additional transcription factor homologues, using the methods described above. The selected cDNAs can be confirmed by sequencing and enzymatic activity.

### SEQUENCE VARIATIONS

It will readily be appreciated by those of skill in the art, that any of a variety of polynucleotide sequences are capable of encoding the transcription factors and transcription factor homologue polypeptides of the invention. Due to the degeneracy of the genetic code, many different polynucleotides can encode identical and/or substantially similar polypeptides in addition to those sequences illustrated in the Sequence Listing.

For example, Table 1 illustrates, e.g., that the codons AGC, AGT, TCA, TCC, TCG, and TCT all encode the same amino acid: serine. Accordingly, at each position in the sequence where there is a codon encoding serine, any of the above trinucleotide sequences can be used without altering the encoded polypeptide.

**Table 1**

| Amino acids | | | Codon | | | | | |
|---|---|---|---|---|---|---|---|---|
| Alanine | Ala | A | GCA | GCC | GCG | GCU | | |
| Cysteine | Cys | C | TGC | TGT | | | | |
| Aspartic acid | Asp | D | GAC | GAT | | | | |
| Glutamic acid | Glu | E | GAA | GAG | | | | |
| Phenylalanine | Phe | F | TTC | TTT | | | | |
| Glycine | Gly | G | GGA | GGC | GGG | GGT | | |
| Histidine | His | H | CAC | CAT | | | | |
| Isoleucine | Ile | I | ATA | ATC | ATT | | | |
| Lysine | Lys | K | AAA | AAG | | | | |
| Leucine | Leu | L | TTA | TTG | CTA | CTC | CTG | CTT |
| Methionine | Met | M | ATG | | | | | |
| Asparagine | Asn | N | AAC | AAT | | | | |
| Proline | Pro | P | CCA | CCC | CCG | CCT | | |
| Glutamine | Gln | Q | CAA | CAG | | | | |
| Arginine | Arg | R | AGA | AGG | CGA | CGC | CGG | CGT |
| Serine | Ser | S | AGC | AGT | TCA | TCC | TCG | TCT |
| Threonine | Thr | T | ACA | ACC | ACG | ACT | | |
| Valine | Val | V | GTA | GTC | GTG | GTT | | |
| Tryptophan | Trp | W | TGG | | | | | |
| Tyrosine | Tyr | Y | TAC | TAT | | | | |

Sequence alterations that do not change the amino acid sequence encoded by the polynucleotide are termed "silent" variations. With the exception of the codons ATG and TGG, encoding methionine and tryptophan, respectively, any of the possible codons for the same amino acid can be substituted by a variety of techniques, e.g., site-directed mutagenesis, available in the art. Accordingly, any and all such variations of a sequence selected from the above table are a feature of the invention.

In addition to silent variations, other conservative variations that alter one, or a few amino acids in the encoded polypeptide, can be made without altering the function of the polypeptide, these conservative variants are, likewise, a feature of the invention.

For example, substitutions, deletions and insertions introduced into the sequences provided in the Sequence Listing are also envisioned by the invention. Such sequence modifications can be engineered into a sequence by site-directed mutagenesis (Wu (ed.) Meth. Enzymol. (1993) vol. 217, Academic Press) or the other methods noted below. Amino acid substitutions are typically of single residues; insertions usually will be on the order of about from 1 to 10 amino acid residues; and deletions will range about from 1 to 30 residues. In preferred embodiments, deletions or insertions are made in adjacent pairs, e.g., a deletion of two residues or insertion of two residues. Substitutions, deletions, insertions or any combination thereof can be combined to arrive at a sequence. The mutations that are made in the polynucleotide encoding the transcription factor should not place the sequence out of reading frame and should not create complementary regions that could produce secondary mRNA structure. Preferably, the polypeptide encoded by the DNA performs the desired function.

Conservative substitutions are those in which at least one residue in the amino acid sequence has been removed and a different residue inserted in its place. Such substitutions generally are made in accordance with the Table 2 when it is desired to maintain the activity of the protein. Table 2 shows amino acids which can be substituted for an amino acid in a protein and which are typically regarded as conservative substitutions.

**Table 2**

| **Residue** | **Conservative Substitutions** |
|---|---|
| Ala | Ser |
| Arg | Lys |
| Asn | Gln; His |
| Asp | Glu |
| Gln | Asn |
| Cys | Ser |
| Glu | Asp |
| Gly | Pro |
| His | Asn; Gln |
| Ile | Leu, Val |
| Leu | Ile; Val |
| Lys | Arg; Gln |
| Met | Leu; Ile |
| Phe | Met; Leu; Tyr |
| Ser | Thr; Gly |
| Thr | Ser; Val |
| Trp | Tyr |
| Tyr | Trp; Phe |
| Val | Ile; Leu |

Substitutions that are less conservative than those in Table 2 can be selected by picking residues that differ more significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. The substitutions which in general are expected to produce the greatest changes in protein properties will be those in which (a) a hydrophilic residue, e.g., seryl or threonyl, is substituted for (or by) a hydrophobic residue, e.g., leucyl, isoleucyl, phenylalanyl, valyl or alanyl; (b) a cysteine or proline is substituted for (or by) any other residue; (c) a residue having an electropositive side chain, e.g., lysyl, arginyl, or histidyl, is substituted for (or by) an electronegative residue, e.g., glutamyl or aspartyl; or (d) a residue having a bulky side chain, e.g., phenylalanine, is substituted for (or by) one not having a side chain, e.g., glycine.

### FURTHER MODIFYING SEQUENCES OF THE INVENTION-MUTATION/ FORCED EVOLUTION

In addition to generating silent or conservative substitutions as noted, above, the present invention optionally includes methods of modifying the sequences of the Sequence Listing. In the methods, nucleic acid or protein modification methods are used to alter the given sequences to produce new sequences and/or to chemically or enzymatically modify given sequences to change the properties of the nucleic acids or proteins.

Thus, in one embodiment, given nucleic acid sequences are modified, e.g., according to standard mutagenesis or artificial evolution methods to produce modified sequences. For example, Ausubel, *supra*, provides additional details on mutagenesis methods. Artificial forced evolution methods are described, e.g., by Stemmer (1994) Nature 370:389-391, and Stemmer (1994) Proc. Natl. Acad. Sci. USA 91:10747-10751. Many other mutation and evolution methods are also available and expected to be within the skill of the practitioner.

Similarly, chemical or enzymatic alteration of expressed nucleic acids and polypeptides can be performed by standard methods. For example, sequence can be modified by addition of lipids, sugars, peptides, organic or inorganic compounds, by the inclusion of modified nucleotides or amino acids, or the like. For example, protein modification techniques are illustrated in Ausubel, *supra*. Further details on chemical and enzymatic modifications can be found herein. These modification methods can be used to modify any given sequence, or to modify any sequence produced by the various mutation and artificial evolution modification methods noted herein.

Accordingly, the invention provides for modification of any given nucleic acid by mutation, evolution, chemical or enzymatic modification, or other available methods, as well as for the products produced by practicing such methods, e.g., using the sequences herein as a starting substrate for the various modification approaches.

For example, optimized coding sequence containing codons preferred by a particular prokaryotic or eukaryotic host can be used e.g., to increase the rate of translation or to produce recombinant RNA transcripts having desirable properties, such as a longer half-life, as compared with transcripts produced using a non-optimized sequence. Translation stop codons can also be modified to reflect host preference. For example, preferred stop codons for *S. cerevisiae* and mammals are TAA and TGA, respectively. The preferred stop codon for monocotyledonous plants is TGA, whereas insects and *E. coli* prefer to use TAA as the stop codon.

The polynucleotide sequences of the present invention can also be engineered in order to alter a coding sequence for a variety of reasons, including but not limited to, alterations which modify the sequence to facilitate cloning, processing and/or expression of the gene product. For example, alterations are optionally introduced using techniques which are well known in the art, e.g., site-directed mutagenesis, to insert new restriction sites, to alter glycosylation patterns, to change codon preference, to introduce splice sites, etc.

Furthermore, a fragment or domain derived from any of the polypeptides of the invention can be combined with domains derived from other transcription factors or synthetic domains to modify the biological activity of a transcription factor. For instance, a DNA binding domain derived from a transcription factor of the invention can be combined with the activation domain of another transcription factor or with a synthetic activation domain. A transcription activation domain assists in initiating transcription from a DNA binding site. Examples include the transcription activation region of VP16 or GAL4 (Moore et al. (1998) Proc. Natl. Acad. Sci. USA 95: 376-381; and Aoyama et al. (1995) Plant Cell 7:1773-1785), peptides derived from bacterial sequences (Ma and Ptashne (1987) Cell 51; 113-119) and synthetic peptides (Giniger and Ptashne, (1987) Nature 330:670-672).

### EXPRESSION AND MODIFICATION OF POLYPEPTIDES

Typically, polynucleotide sequences of the invention are incorporated into recombinant DNA (or RNA) molecules that direct expression of polypeptides of the invention in appropriate host cells, transgenic plants, in vitro translation systems, or the like. Due to the inherent degeneracy of the genetic code, nucleic acid sequences which encode substantially the same or a functionally equivalent amino acid sequence can be substituted for any listed sequence to provide for cloning and expressing the relevant homologue.

### Vectors, Promoters and Expression Systems

The present invention includes recombinant constructs comprising one or more of the nucleic acid sequences herein. The constructs typically comprise a vector, such as a plasmid, a cosmid, a phage, a virus (e.g., a plant virus), a bacterial artificial chromosome (BAC), a yeast artificial chromosome (YAC), or the like, into which a nucleic acid sequence of the invention has been inserted, in a forward or reverse orientation. In a preferred aspect of this embodiment, the construct further comprises regulatory sequences, including, for example, a promoter, operably linked to the sequence. Large numbers of suitable vectors and promoters are known to those of skill in the art, and are commercially available.

General texts which describe molecular biological techniques useful herein, including the use and production of vectors, promoters and many other relevant topics, include Berger, Sambrook and Ausubel, *supra*. Any of the identified sequences can be incorporated into a cassette or vector, e.g., for expression in plants. A number of expression vectors suitable for stable transformation of plant cells or for the establishment of transgenic plants have been described including those described in Weissbach and Weissbach, (1989) Methods for Plant Molecular Biology, Academic Press, and Gelvin et al., (1990) Plant Molecular Biology Manual, Kluwer Academic Publishers. Specific examples include those derived from a Ti plasmid of *Agrobacterium tumefaciens*, as well as those disclosed by Herrera-Estrella et al. (1983) Nature 303: 209, Bevan (1984) Nucl Acid Res. 12: 8711-8721, Klee (1985) Bio/Technology 3: 637-642, for dicotyledonous plants.

Alternatively, non-Ti vectors can be used to transfer the DNA into monocotyledonous plants and cells by using free DNA delivery techniques. Such methods can involve, for example, the use of liposomes, electroporation, microprojectile bombardment, silicon carbide whiskers, and viruses. By using these methods transgenic plants such as wheat, rice (Christou (1991) Bio/Technology 9: 957-962) and corn (Gordon-Kamm (1990) Plant Cell 2: 603-618) can be produced. An immature embryo can also be a good target tissue for monocots for direct DNA delivery techniques by using the particle gun (Weeks et al. (1993) Plant Physiol 102: 1077-1084; Vasil (1993) Bio/Technology 10: 667-674; Wan and Lemeaux (1994) Plant Physiol 104: 37-48, and for Agrobacterium-mediated DNA transfer (Ishida et al. (1996) Nature Biotech 14: 745-750).

Typically, plant transformation vectors include one or more cloned plant coding sequence (genomic or cDNA) under the transcriptional control of 5' and 3' regulatory sequences and a dominant selectable marker. Such plant transformation vectors typically also contain a promoter (e.g., a regulatory region controlling inducible or constitutive, environmentally-or developmentally-regulated, or cell- or tissue-specific expression), a transcription initiation start site, an RNA processing signal (such as intron splice sites), a transcription termination site, and/or a polyadenylation signal.

Examples of constitutive plant promoters which can be useful for expressing the TF sequence include: the cauliflower mosaic virus (CaMV) 35S promoter, which confers constitutive, high-level expression in most plant tissues (*see*, e.g., Odel et al. (1985) Nature 313:810); the nopaline synthase promoter (An et al. (1988) Plant Physiol 88:547); and the octopine synthase promoter (Fromm et al. (1989) Plant Cell 1: 977).

A variety of plant gene promoters that regulate gene expression in response to environmental, hormonal, chemical, developmental signals, and in a tissue-active manner can be used for expression of a TF sequence in plants. Choice of a promoter is based largely on the phenotype of interest and is determined by such factors as tissue (e.g., seed, fruit, root, pollen, vascular tissue, flower, carpel, etc.), inducibility (e.g., in response to wounding, heat, cold, drought, light, pathogens, etc.), timing, developmental stage, and the like. Numerous known promoters have been characterized and can favorable be employed to promote expression of a polynucleotide of the invention in a transgenic plant or cell of interest. For example, tissue specific promoters include: seed-specific promoters (such as the napin, phaseolin or DC3 promoter described in US Pat. No. 5,773,697), fruit-specific promoters that are active during fruit ripening (such as the dru 1 promoter (US Pat. No. 5,783,393), or the 2A11 promoter (US Pat. No. 4,943,674) and the tomato polygalacturonase promoter (Bird et al. (1988) Plant Mol Biol 11:651), root-specific promoters, such as those disclosed in US Patent Nos. 5,618,988, 5,837,848 and 5,905,186, pollen-active promoters such as PTA29, PTA26 and PTA13 (US Pat. No. 5,792,929), promoters active in vascular tissue (Ringli and Keller (1998) Plant Mol Biol 37:977-988), flower-specific (Kaiser et al, (1995) Plant Mol Biol 28:231-243), pollen (Baerson et al. (1994) Plant Mol Biol 26:1947-1959), carpels (Ohl et al. (1990) Plant Cell 2:837-848), pollen and ovules (Baerson et al. (1993) Plant Mol Biol 22:255-267), auxin-inducible promoters (such as that described in van der Kop et al. (1999) Plant Mol Biol 39:979-990 or Baumann et al. (1999) Plant Cell 11:323-334), cytokinin-inducible promoter (Guevara-Garcia (1998) Plant Mol Biol 38:743-753), promoters responsive to gibberellin (Shi et al. (1998) Plant Mol Biol 38:1053-1060, Willmott et al. (1998) 38:817-825) and the like. Additional promoters are those that elicit expression in response to heat (Ainley et al. (1993) Plant Mol Biol 22: 13-23), light (e.g., the pea rbcS-3A promoter, Kuhlemeier et al. (1989) Plant Cell 1:471, and the maize rbcS promoter, Schaffner and Sheen (1991) Plant Cell 3: 997); wounding (e.g., *wunI*, Siebertz et al. (1989) Plant Cell 1: 961); pathogens (such as the PR-1 promoter described in Buchel et al. (1999) Plant Mol. Biol. 40:387-396, and the PDF1.2 promoter described in Manners et al. (1998) Plant Mol. Biol. 38:1071-80), and chemicals such as methyl jasmonate or salicylic acid (Gatz et al. (1997) Plant Mol Biol 48: 89-108). In addition, the timing of the expression can be controlled by using promoters such as those acting at senescence (An and Amazon (1995) Science 270: 1986-1988); or late seed development (Odell et al. (1994) Plant Physiol 106:447-458).

Plant expression vectors can also include RNA processing signals that can be positioned within, upstream or downstream of the coding sequence. In addition, the expression vectors can include additional regulatory sequences from the 3'-untranslated region of plant genes, e.g., a 3' terminator region to increase mRNA stability of the mRNA, such as the PI-II terminator region of potato or the octopine or nopaline synthase 3' terminator regions.

### Additional Expression Elements

Specific initiation signals can aid in efficient translation of coding sequences. These signals can include, e.g., the ATG initiation codon and adjacent sequences. In cases where a coding sequence, its initiation codon and upstream sequences are inserted into the appropriate expression vector, no additional translational control signals may be needed. However, in cases where only coding sequence (e.g., a mature protein coding sequence), or a portion thereof, is inserted, exogenous transcriptional control signals including the ATG initiation codon can be separately provided. The initiation codon is provided in the correct reading frame to facilitate transcription. Exogenous transcriptional elements and initiation codons can be of various origins, both natural and synthetic. The efficiency of expression can be enhanced by the inclusion of enhancers appropriate to the cell system in use.

### Expression Hosts

The present invention also relates to host cells which are transduced with vectors of the invention, and the production of polypeptides of the invention (including fragments thereof) by recombinant techniques. Host cells are genetically engineered (i.e, nucleic acids are introduced, e.g., transduced, transformed or transfected) with the vectors of this invention, which may be, for example, a cloning vector or an expression vector comprising the relevant nucleic acids herein. The vector is optionally a plasmid, a viral particle, a phage, a naked nucleic acids, *etc*. The engineered host cells can be cultured in conventional nutrient media modified as appropriate for activating promoters, selecting transformants, or amplifying the relevant gene. The culture conditions, such as temperature, pH and the like, are those previously used with the host cell selected for expression, and will be apparent to those skilled in the art and in the references cited herein, including, Sambrook and Ausubel.

The host cell can be a eukaryotic cell, such as a yeast cell, or a plant cell, or the host cell can be a prokaryotic cell, such as a bacterial cell. Plant protoplasts are also suitable for some applications. For example, the DNA fragments are introduced into plant tissues, cultured plant cells or plant protoplasts by standard methods including electroporation (Fromm et al., (1985) Proc. Natl. Acad. Sci. USA 82, 5824, infection by viral vectors such as cauliflower mosaic virus (CaMV) (Hohn et al., (1982) Molecular Biology of Plant Tumors, (Academic Press, New York) pp. 549-560; US 4,407,956), high velocity ballistic penetration by small particles with the nucleic acid either within the matrix of small beads or particles, or on the surface (Klein et al., (1987) Nature 327, 70-73), use of pollen as vector (WO 85/01856), or use of *Agrobacterium tumefaciens* or *A. rhizogenes* carrying a T-DNA plasmid in which DNA fragments are cloned. The T-DNA plasmid is transmitted to plant cells upon infection by *Agrobacterium tumefaciens*, and a portion is stably integrated into the plant genome (Horsch et al. (1984) Science 233:496-498; Fraley et al. (1983) Proc. Natl. Acad. Sci. USA 80, 4803).

The cell can include a nucleic acid of the invention which encodes a polypeptide, wherein the cells expresses a polypeptide of the invention. The cell can also include vector sequences, or the like. Furthermore, cells and transgenic plants which include any polypeptide or nucleic acid above or throughout this specification, e.g., produced by transduction of a vector of the invention, are an additional feature of the invention.

For long-term, high-yield production of recombinant proteins, stable expression can be used. Host cells transformed with a nucleotide sequence encoding a polypeptide of the invention are optionally cultured under conditions suitable for the expression and recovery of the encoded protein from cell culture. The protein or fragment thereof produced by a recombinant cell may be secreted, membrane-bound, or contained intracellularly, depending on the sequence and/or the vector used. As will be understood by those of skill in the art, expression vectors containing polynucleotides encoding mature proteins of the invention can be designed with signal sequences which direct secretion of the mature polypeptides through a prokaryotic or eukaryotic cell membrane.

### Modified Amino Acids

Polypeptides of the invention may contain one or more modified amino acids. The presence of modified amino acids may be advantageous in, for example, increasing polypeptide half-life, reducing polypeptide antigenicity or toxicity, increasing polypeptide storage stability, or the like. Amino acid(s) are modified, for example, co-translationally or post-translationally during recombinant production or modified by synthetic or chemical means.

Non-limiting examples of a modified amino acid include incorporation or other use of acetylated amino acids, glycosylated amino acids, sulfated amino acids, prenylated (e.g., farnesylated, geranylgeranylated) amino acids, PEG modified (e.g., "PEGylated") amino acids, biotinylated amino acids, carboxylated amino acids, phosphorylated amino acids, etc. References adequate to guide one of skill in the modification of amino acids are replete throughout the literature.

### IDENTIFICATION OF ADDITIONAL FACTORS

A transcription factor provided by the present invention can also be used to identify additional endogenous or exogenous molecules that can affect a phentoype or trait of interest. On the one hand, such molecules include organic (small or large molecules) and/or inorganic compounds that affect expression of (i.e., regulate) a particular transcription factor. Alternatively, such molecules include endogenous molecules that are acted upon either at a transcriptional level by a transcription factor of the invention to modify a phenotype as desired. For example, the transcription factors can be employed to identify one or more downstream gene with which is subject to a regulatory effect of the transcription factor. In one approach, a transcription factor or transcription factor homologue of the invention is expressed in a host cell, e.g, a transgenic plant cell, tissue or explant, and expression products, either RNA or protein, of likely or random targets are monitored, e.g., by hybridization to a microarray of nucleic acid probes corresponding to genes expressed in a tissue or cell type of interest, by two-dimensional gel electrophoresis of protein products, or by any other method known in the art for assessing expression of gene products at the level of RNA or protein. Alternatively, a transcription factor of the invention can be used to identify promoter sequences (i.e., binding sites) involved in the regulation of a downstream target. After identifying a promoter sequence, interactions between the transcription factor and the promoter sequence can be modified by changing specific nucleotides in the promoter sequence or specific amino acids in the transcription factor that interact with the promoter sequence to alter a plant trait. Typically, transcription factor DNA binding sites are identified by gel shift assays. After identifying the promoter regions, the promoter region sequences can be employed in double-stranded DNA arrays to identify molecules that affect the interactions of the transcription factors with their promoters (Bulyk et al. (1999) Nature Biotechnology 17:573-577).

The identified transcription factors are also useful to identify proteins that modify the activity of the transcription factor. Such modification can occur by covalent modification, such as by phosphorylation, or by protein-protein (homo or-heteropolymer) interactions. Any method suitable for detecting protein-protein interactions can be employed. Among the methods that can be employed are co-immunoprecipitation, cross-linking and co-purification through gradients or chromatographic columns, and the two-hybrid yeast system.

The two-hybrid system detects protein interactions in vivo and is described in Chien, et al., (1991), Proc. Natl. Acad. Sci. USA 88, 9578-9582 and is commercially available from Clontech (Palo Alto, Calif.). In such a system, plasmids are constructed that encode two hybrid proteins: one consists of the DNA-binding domain of a transcription activator protein fused to the TF polypeptide and the other consists of the transcription activator protein's activation domain fused to an unknown protein that is encoded by a cDNA that has been recombined into the plasmid as part of a cDNA library. The DNA-binding domain fusion plasmid and the cDNA library are transformed into a strain of the yeast *Saccharomyces cerevisiae* that contains a reporter gene (e.g., lacZ) whose regulatory region contains the transcription activator's binding site. Either hybrid protein alone cannot activate transcription of the reporter gene. Interaction of the two hybrid proteins reconstitutes the functional activator protein and results in expression of the reporter gene, which is detected by an assay for the reporter gene product. Then, the library plasmids responsible for reporter gene expression are isolated and sequenced to identify the proteins encoded by the library plasmids. After identifying proteins that interact with the transcription factors, assays for compounds that interfere with the TF protein-protein interactions can be preformed.

### IDENTIFICATION OF MODULATORS

In addition to the intracellular molecules described above, extracellular molecules that alter activity or expression of a transcription factor, either directly or indirectly, can be identified. For example, the methods can entail first placing a candidate molecule in contact with a plant or plant cell. The molecule can be introduced by topical administration, such as spraying or soaking of a plant, and then the molecule's effect on the expression or activity of the TF polypeptide or the expression of the polynucleotide monitored. Changes in the expression of the TF polypeptide can be monitored by use of polyclonal or monoclonal antibodies, gel electrophoresis or the like. Changes in the expression of the corresponding polynucleotide sequence can be detected by use of microarrays, Northerns, quantitative PCR, or any other technique for monitoring changes in mRNA expression. These techniques are exemplified in Ausubel et al. (eds) Current Protocols in Molecular Biology, John Wiley & Sons (1998). Such changes in the expression levels can be correlated with modified plant traits and thus identified molecules can be useful for soaking or spraying on fruit, vegetable and grain crops to modify traits in plants.

Essentially any available composition can be tested for modulatory activity of expression or activity of any nucleic acid or polypeptide herein. Thus, available libraries of compounds such as chemicals, polypeptides, nucleic acids and the like can be tested for modulatory activity. Often, potential modulator compounds can be dissolved in aqueous or organic (e.g., DMSO-based) solutions for easy delivery to the cell or plant of interest in which the activity of the modulator is to be tested. Optionally, the assays are designed to screen large modulator composition libraries by automating the assay steps and providing compounds from any convenient source to assays, which are typically run in parallel (e.g., in microtiter formats on microtiter plates in robotic assays).

In one embodiment, high throughput screening methods involve providing a combinatorial library containing a large number of potential compounds (potential modulator compounds). Such "combinatorial chemical libraries" are then screened in one or more assays, as described herein, to identify those library members (particular chemical species or subclasses) that display a desired characteristic activity. The compounds thus identified can serve as target compounds.

A combinatorial chemical library can be, e.g., a collection of diverse chemical compounds generated by chemical synthesis or biological synthesis. For example, a combinatorial chemical library such as a polypeptide library is formed by combining a set of chemical building blocks (e.g., in one example, amino acids) in every possible way for a given compound length (i.e., the number of amino acids in a polypeptide compound of a set length). Exemplary libraries include peptide libraries, nucleic acid libraries, antibody libraries (see, e.g., Vaughn et al. (1996) Nature Biotechnology, 14(3):309-314 and PCT/US96/10287), carbohydrate libraries (see, e.g., Liang et al. Science (1996) 274:1520-1522 and U.S. Patent 5,593,853), peptide nucleic acid libraries (see, e.g., U.S. Patent 5,539,083), and small organic molecule libraries (see, e.g., benzodiazepines, Baum C&EN Jan 18, page 33 (1993); isoprenoids, U.S. Patent 5,569,588; thiazolidinones and metathiazanones, U.S. Patent 5,549,974; pyrrolidines, U.S. Patents 5,525,735 and 5,519,134; morpholino compounds, U.S. Patent 5,506,337) and the like.

Preparation and screening of combinatorial or other libraries is well known to those of skill in the art. Such combinatorial chemical libraries include, but are not limited to, peptide libraries (see, e.g., U.S. Patent 5,010,175, Furka, Int. J. Pept. Prot. Res. 37:487-493 (1991) and Houghton et al. Nature 354:84-88 (1991)). Other chemistries for generating chemical diversity libraries can also be used.

In addition, as noted, compound screening equipment for high-throughput screening is generally available, e.g., using any of a number of well known robotic systems that have also been developed for solution phase chemistries useful in assay systems. These systems include automated workstations including an automated synthesis apparatus and robotic systems utilizing robotic arms. Any of the above devices are suitable for use with the present invention, e.g., for high-throughput screening of potential modulators. The nature and implementation of modifications to these devices (if any) so that they can operate as discussed herein will be apparent to persons skilled in the relevant art.

Indeed, entire high throughput screening systems are commercially available. These systems typically automate entire procedures including all sample and reagent pipetting, liquid dispensing, timed incubations, and final readings of the microplate in detector(s) appropriate for the assay. These configurable systems provide high throughput and rapid start up as well as a high degree of flexibility and customization. Similarly, microfluidic implementations of screening are also commercially available.

The manufacturers of such systems provide detailed protocols the various high throughput. Thus, for example, Zymark Corp. provides technical bulletins describing screening systems for detecting the modulation of gene transcription, ligand binding, and the like. The integrated systems herein, in addition to providing for sequence alignment and, optionally, synthesis of relevant nucleic acids, can include such screening apparatus to identify modulators that have an effect on one or more polynucleotides or polypeptides according to the present invention.

In some assays it is desirable to have positive controls to ensure that the components of the assays are working properly. At least two types of positive controls are appropriate. That is, known transcriptional activators or inhibitors can be incubated with cells/plants/ etc. in one sample of the assay, and the resulting increase/decrease in transcription can be detected by measuring the resulting increase in RNA/ protein expression, etc., according to the methods herein. It will be appreciated that modulators can also be combined with transcriptional activators or inhibitors to find modulators which inhibit transcriptional activation or transcriptional repression. Either expression of the nucleic acids and proteins herein or any additional nucleic acids or proteins activated by the nucleic acids or proteins herein, or both, can be monitored.

In an embodiment, the invention provides a method for identifying compositions that modulate the activity or expression of a polynucleotide or polypeptide of the invention. For example, a test compound, whether a small or large molecule, is placed in contact with a cell, plant (or plant tissue or explant), or composition comprising the polynucleotide or polypeptide of interest and a resulting effect on the cell, plant, (or tissue or explant) or composition is evaluated by monitoring, either directly or indirectly, one or more of: expression level of the polynucleotide or polypeptide, activity (or modulation of the activity) of the polynucleotide or polypeptide. In some cases, an alteration in a plant phenotype can be detected following contact of a plant (or plant cell, or tissue or explant) with the putative modulator, e.g., by modulation of expression or activity of a polynucleotide or polypeptide of the invention.

### SUBSEQUENCES

Also contemplated are uses of polynucleotides, also referred to herein as oligonucleotides, typically having at least 12 bases, preferably at least 15, more preferably at least 20, 30, or 50 bases, which hybridize under at least highly stringent (or ultra-high stringent or ultra-ultra- high stringent conditions) conditions to a polynucleotide sequence described above. The polynucleotides may be used as probes, primers, sense and antisense agents, and the like, according to methods as noted *supra*.

Subsequences of the polynucleotides of the invention, including polynucleotide fragments and oligonucleotides are useful as nucleic acid probes and primers. An oligonucleotide suitable for use as a probe or primer is at least about 15 nucleotides in length, more often at least about 18 nucleotides, often at least about 21 nucleotides, frequently at least about 30 nucleotides, or about 40 nucleotides, or more in length. A nucleic acid probe is useful in hybridization protocols, e.g., to identify additional polypeptide homologues of the invention, including protocols for microarray experiments. Primers can be annealed to a complementary target DNA strand by nucleic acid hybridization to form a hybrid between the primer and the target DNA strand, and then extended along the target DNA strand by a DNA polymerase enzyme. Primer pairs can be used for amplification of a nucleic acid sequence, e.g., by the polymerase chain reaction (PCR) or other nucleic-acid amplification methods. See Sambrook and Ausubel, *supra*.

In addition, the invention includes an isolated or recombinant polypeptide including a subsequence of at least about 15 contiguous amino acids encoded by the recombinant or isolated polynucleotides of the invention. For example, such polypeptides, or domains or fragments thereof, can be used as immunogens, e.g., to produce antibodies specific for the polypeptide sequence, or as probes for detecting a sequence of interest. A subsequence can range in size from about 15 amino acids in length up to and including the full length of the polypeptide.

### PRODUCTION OF TRANSGENIC PLANTS

### Modification of Traits

The polynucleotides of the invention are favorably employed to produce transgenic plants with various traits, or characteristics, that have been modified in a desirable manner, e.g., to improve the environmental stress resistance of a plant. For example, alteration of expression levels or patterns (e.g., spatial or temporal expression patterns) of one or more of the transcription factors (or transcription factor homologues) of the invention, as compared with the levels of the same protein found in a wild type plant, can be used to modify a plant's traits. An illustrative example of trait modification, improved environmental stress tolerance, by altering expression levels of a particular transcription factor is described further in the Examples and the Sequence Listing.

### Antisense and Cosuppression Approaches

In addition to expression of the nucleic acids of the invention as gene replacement or plant phenotype modification nucleic acids, the nucleic acids are also useful for sense and anti-sense suppression of expression, e.g., to down-regulate expression of a nucleic acid of the invention, e.g., as a further mechanism for modulating plant phenotype. That is, the nucleic acids of the invention, or subsequences or anti-sense sequences thereof, can be used to block expression of naturally occurring homologous nucleic acids. A variety of sense and anti-sense technologies are known in the art, e.g., as set forth in Lichtenstein and Nellen (1997) Antisense Technology: A Practical Approach IRL Press at Oxford University, Oxford, England. In general, sense or anti-sense sequences are introduced into a cell, where they are optionally amplified, e.g., by transcription. Such sequences include both simple oligonucleotide sequences and catalytic sequences such as ribozymes.

For example, a reduction or elimination of expression (i.e., a "knock-out") of a transcription factor or transcription factor homologue polypeptide in a transgenic plant, e.g., to modify a plant trait, can be obtained by introducing an antisense construct corresponding to the polypeptide of interest as a cDNA. For antisense suppression, the transcription factor or homologue cDNA is arranged in reverse orientation (with respect to the coding sequence) relative to the promoter sequence in the expression vector. The introduced sequence need not be the full length cDNA or gene, and need not be identical to the cDNA or gene found in the plant type to be transformed. Typically, the antisense sequence need only be capable of hybridizing to the target gene or RNA of interest. Thus, where the introduced sequence is of shorter length, a higher degree of homology to the endogenous transcription factor sequence will be needed for effective antisense suppression. While antisense sequences of various lengths can be utilized, preferably, the introduced antisense sequence in the vector will be at least 30 nucleotides in length, and improved antisense suppression will typically be observed as the length of the antisense sequence increases. Preferably, the length of the antisense sequence in the vector will be greater than 100 nucleotides. Transcription of an antisense construct as described results in the production of RNA molecules that are the reverse complement of mRNA molecules transcribed from the endogenous transcription factor gene in the plant cell.

Suppression of endogenous transcription factor gene expression can also be achieved using a ribozyme. Ribozymes are RNA molecules that possess highly specific endoribonuclease activity. The production and use of ribozymes are disclosed in U.S. Patent No. 4,987,071 and U.S. Patent No. 5,543,508. Synthetic ribozyme sequences including antisense RNAs can be used to confer RNA cleaving activity on the antisense RNA, such that endogenous mRNA molecules that hybridize to the antisense RNA are cleaved, which in turn leads to an enhanced antisense inhibition of endogenous gene expression.

Vectors in which RNA encoded by a transcription factor or transcription factor homologue cDNA is over-expressed can also be used to obtain co-suppression of a corresponding endogenous gene, e.g., in the manner described in U.S. Patent No. 5,231,020 to Jorgensen. Such co-suppression (also termed sense suppression) does not require that the entire transcription factor cDNA be introduced into the plant cells, nor does it require that the introduced sequence be exactly identical to the endogenous transcription factor gene of interest. However, as with antisense suppression, the suppressive efficiency will be enhanced as specificity of hybridization is increased, e.g., as the introduced sequence is lengthened, and/or as the sequence similarity between the introduced sequence and the endogenous transcription factor gene is increased.

Vectors expressing an untranslatable form of the transcription factor mRNA, e.g., sequences comprising one or more stop codon, or nonsense mutation) can also be used to suppress expression of an endogenous transcription factor, thereby reducing or eliminating it's activity and modifying one or more traits. Methods for producing such constructs are described in U.S. Patent No. 5,583,021. Preferably, such constructs are made by introducing a premature stop codon into the transcription factor gene. Alternatively, a plant trait can be modified by gene silencing using double-strand RNA (Sharp (1999) Genes and Development 13: 139-141).

Another method for abolishing the expression of a gene is by insertion mutagenesis using the T-DNA of *Agrobacterium tumefaciens*. After generating the insertion mutants, the mutants can be screened to identify those containing the insertion in a transcription factor or transcription factor homologue gene. Plants containing a single transgene insertion event at the desired gene can be crossed to generate homozygous plants for the mutation (Koncz et al. (1992) Methods in Arabidopsis Research, World Scientific).

Alternatively, a plant phenotype can be altered by eliminating an endogenous gene, such as a transcription factor or transcription factor homologue, e.g., by homologous recombination (Kempin et al. (1997) Nature 389:802).

A plant trait can also be modified by using the cre-lox system (for example, as described in US Pat. No. 5,658,772). A plant genome can be modified to include first and second lox sites that are then contacted with a Cre recombinase. If the lox sites are in the same orientation, the intervening DNA sequence between the two sites is excised. If the lox sites are in the opposite orientation, the intervening sequence is inverted.

The polynucleotides and polypeptides of this invention can also be expressed in a plant in the absence of an expression cassette by manipulating the activity or expression level of the endogenous gene by other means. For example, by ectopically expressing a gene by T-DNA activation tagging (Ichikawa et al. (1997) Nature 390 698-701; Kakimoto et al. (1996) Science 274: 982-985). This method entails transforming a plant with a gene tag containing multiple transcriptional enhancers and once the tag has inserted into the genome, expression of a flanking gene coding sequence becomes deregulated. In another example, the transcriptional machinery in a plant can be modified so as to increase transcription levels of a polynucleotide of the invention (*See,* e.g., PCT Publications WO 96/06166 and WO 98/53057 which describe the modification of the DNA binding specificity of zinc finger proteins by changing particular amino acids in the DNA binding motif).

The transgenic plant can also include the machinery necessary for expressing or altering the activity of a polypeptide encoded by an endogenous gene, for example by altering the phosphorylation state of the polypeptide to maintain it in an activated state.

Transgenic plants (or plant cells, or plant explants, or plant tissues) incorporating the polynucleotides of the invention and/or expressing the polypeptides of the invention can be produced by a variety of well established techniques as described above. Following construction of a vector, most typically an expression cassette, including a polynucleotide, e.g., encoding a transcription factor or transcription factor homologue, of the invention, standard techniques can be used to introduce the polynucleotide into a plant, a plant cell, a plant explant or a plant tissue of interest. Optionally, the plant cell, explant or tissue can be regenerated to produce a transgenic plant.

The plant can be any higher plant, including gymnosperms, monocotyledonous and dicotyledenous plants. Suitable protocols are available for *Leguminosae* (alfalfa, soybean, clover, etc.), *Umbelliferae* (carrot, celery, parsnip), *Cruciferae* (cabbage, radish, rapeseed, broccoli, etc.), *Curcurbitaceae* (melons and cucumber), *Gramineae* (wheat, corn, rice, barley, millet, etc.), *Solanaceae* (potato, tomato, tobacco, peppers, etc.), and various other crops. See protocols described in Ammirato et al. (1984) Handbook of Plant Cell Culture -Crop Species. Macmillan Publ. Co. Shimamoto et al. (1989) Nature 338:274-276; Fromm et al. (1990) Bio/Technology 8:833-839; and Vasil et al. (1990) Bio/Technology 8:429-434.

Transformation and regeneration of both monocotyledonous and dicotyledonous plant cells is now routine, and the selection of the most appropriate transformation technique will be determined by the practitioner. The choice of method will vary with the type of plant to be transformed; those skilled in the art will recognize the suitability of particular methods for given plant types. Suitable methods can include, but are not limited to: electroporation of plant protoplasts; liposome-mediated transformation; polyethylene glycol (PEG) mediated transformation; transformation using viruses; micro-injection of plant cells; micro-projectile bombardment of plant cells; vacuum infiltration; and *Agrobacterium tumeficiens* mediated transformation. Transformation means introducing a nucleotide sequence in a plant in a manner to cause stable or transient expression of the sequence.

Successful examples of the modification of plant characteristics by transformation with cloned sequences which serve to illustrate the current knowledge in this field of technology, and which are herein incorporated by reference, include: U.S. Patent Nos. 5,571,706; 5,677,175; 5,510,471; 5,750,386; 5,597,945; 5,589,615; 5,750,871; 5,268,526; 5,780,708; 5,538,880; 5,773,269; 5,736,369 and 5,610,042.

Following transformation, plants are preferably selected using a dominant selectable marker incorporated into the transformation vector. Typically, such a marker will confer antibiotic or herbicide resistance on the transformed plants, and selection of transformants can be accomplished by exposing the plants to appropriate concentrations of the antibiotic or herbicide.

After transformed plants are selected and grown to maturity, those plants showing a modified trait are identified. The modifed trait can be any of those traits described above. Additionally, to confirm that the modified trait is due to changes in expression levels or activity of the polypeptide or polynucleotide of the invention can be determined by analyzing mRNA expression using Northern blots, RT-PCR or microarrays, or protein expression using immunoblots or Western blots or gel shift assays.

### INTEGRATED SYSTEMS-SEQUENCE IDENTITY

Additionally, the present invention may be an integrated system, computer or computer readable medium that comprises an instruction set for determining the identity of one or more sequences in a database. In addition, the instruction set can be used to generate or identify sequences that meet any specified criteria. Furthermore, the instruction set may be used to associate or link certain functional benefits, such improved environmental stress tolerance, with one or more identified sequence.

For example, the instruction set can include, e.g., a sequence comparison or other alignment program, e.g., an available program such as, for example, the Wisconsin Package Version 10.0, such as BLAST, FASTA, PILEUP, FINDPATTERNS or the like (GCG, Madision, WI). Public sequence databases such as GenBank, EMBL, Swiss-Prot and PIR or private sequence databases such as PhytoSeq (Incyte Pharmaceuticals, Palo Alto, CA) can be searched.

Alignment of sequences for comparison can be conducted by the local homology algorithm of Smith and Waterman (1981) Adv. Appl. Math. 2:482, by the homology alignment algorithm of Needleman and Wunsch (1970) J. Mol. Biol. 48:443, by the search for similarity method of Pearson and Lipman (1988) Proc. Natl. Acad. Sci. U.S.A. 85: 2444, by computerized implementations of these algorithms. After alignment, sequence comparisons between two (or more) polynucleotides or polypeptides are typically performed by comparing sequences of the two sequences over a comparison window to identify and compare local regions of sequence similarity. The comparison window can be a segment of at least about 20 contiguous positions, usually about 50 to about 200, more usually about 100 to about 150 contiguous positions. A description of the method is provided in Ausubel et al., *supra*.

A variety of methods of determining sequence relationships can be used, including manual alignment and computer assisted sequence alignment and analysis. This later approach is a preferred approach in the present invention, due to the increased throughput afforded by computer assisted methods. As noted above, a variety of computer programs for performing sequence alignment are available, or can be produced by one of skill.

One example algorithm that is suitable for determining percent sequence identity and sequence similarity is the BLAST algorithm, which is described in Altschul et al. J. Mol. Biol 215:403-410 (1990). Software for performing BLAST analyses is publicly available, e.g., through the National Center for Biotechnology Information (http://www.ncbi.nlm.nih.gov/). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul et al., *supra*). These initial neighborhood word hits act as seeds for initiating searches to find longer HSPs containing them. The word hits are then extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) of 10, a cutoff of 100, M=5, N=-4, and a comparison of both strands. For amino acid sequences, the BLASTP program uses as defaults a wordlength (W) of 3, an expectation (E) of 10, and the BLOSUM62 scoring matrix (*see* Henikoff & Henikoff (1989) Proc. Natl. Acad. Sci. USA 89:10915).

In addition to calculating percent sequence identity, the BLAST algorithm also performs a statistical analysis of the similarity between two sequences (*see,* e.g., Karlin & Altschul (1993) Proc. Natl. Acad. Sci. USA 90:5873-5787). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence (and, therefore, in this context, homologous) if the smallest sum probability in a comparison of the test nucleic acid to the reference nucleic acid is less than about 0.1, or less than about 0.01, and or even less than about 0.001. An additional example of a useful sequence alignment algorithm is PILEUP. PILEUP creates a multiple sequence alignment from a group of related sequences using progressive, pairwise alignments. The program can align, e.g., up to 300 sequences of a maximum length of 5,000 letters.

The integrated system, or computer typically includes a user input interface allowing a user to selectively view one or more sequence records corresponding to the one or more character strings, as well as an instruction set which aligns the one or more character strings with each other or with an additional character string to identify one or more region of sequence similarity. The system may include a link of one or more character strings with a particular phenotype or gene function. Typically, the system includes a user readable output element which displays an alignment produced by the alignment instruction set.

The methods of this invention can be implemented in a localized or distributed computing environment. In a distributed environment, the methods may implemented on a single computer comprising multiple processors or on a multiplicity of computers. The computers can be linked, e.g. through a common bus, but more preferably the computer(s) are nodes on a network. The network can be a generalized or a dedicated local or wide-area network and, in certain preferred embodiments, the computers may be components of an intra-net or an internet.

Thus, the invention provides methods for identifying a sequence similar or homologous to one or more polynucleotides as noted herein, or one or more target polypeptides encoded by the polynucleotides, or otherwise noted herein and may include linking or associating a given plant phenotype or gene function with a sequence. In the methods, a sequence database is provided (locally or across an inter or intra net) and a query is made against the sequence database using the relevant sequences herein and associated plant phenotypes or gene functions.

Any sequence herein can be entered into the database, before or after querying the database. This provides for both expansion of the database and, if done before the querying step, for insertion of control sequences into the database. The control sequences can be detected by the query to ensure the general integrity of both the database and the query. As noted, the query can be performed using a web browser based interface. For example, the database can be a centralized public database such as those noted herein, and the querying can be done from a remote terminal or computer across an internet or intranet.

### EXAMPLES

The following examples are intended to illustrate but not limit the present invention.

### EXAMPLE I. FULL LENGTH GENE IDENTIFICATION AND CLONING

Putative transcription factor sequences (genomic or ESTs) related to known transcription factors were identified in the *Arabidopsis thaliana* GenBank database using the tblastn sequence analysis program using default parameters and a P-value cutoff threshold of -4 or -5 or lower, depending on the length of the query sequence. Putative transcription factor sequence hits were then screened to identify those containing particular sequence strings. If the sequence hits contained such sequence strings, the sequences were confirmed as transcription factors.

Alternatively, Arabidopsis *thaliana* cDNA libraries derived from different tissues or treatments, or genomic libraries were screened to identify novel members of a transcription family using a low stringency hybridization approach. Probes were synthesized using gene specific primers in a standard PCR reaction (annealing temperature 60° C) and labeled with ³²P dCTP using the High Prime DNA Labeling Kit (Boehringer Mannheim). Purified radiolabelled probes were added to filters immersed in Church hybridization medium (0.5 M NaPO₄ pH 7.0, 7% SDS, 1 % w/v bovine serum albumin) and hybridized overnight at 60 °C with shaking. Filters were washed two times for 45 to 60 minutes with 1xSCC, 1% SDS at 60° C.

To identify additional sequence 5' or 3' of a partial cDNA sequence in a cDNA library, 5' and 3' rapid amplification of cDNA ends (RACE) was performed using the Marathon^{™} cDNA amplification kit (Clontech, Palo Alto, CA). Generally, the method entailed first isolating poly(A) mRNA, performing first and second strand cDNA synthesis to generate double stranded cDNA, blunting cDNA ends, followed by ligation of the Marathon^{™} Adaptor to the cDNA to form a library of adaptor-ligated ds cDNA.

Gene-specific primers were designed to be used along with adaptor specific primers for both 5' and 3' RACE reactions. Nested primers, rather than single primers, were used to increase PCR specificity. Using 5' and 3' RACE reactions, 5' and 3' RACE fragments were obtained, sequenced and cloned. The process can be repeated until 5' and 3' ends of the full-length gene were identified. Then the full-length cDNA was generated by PCR using primers specific to 5' and 3' ends of the gene by end-to-end PCR.

### EXAMPLE II. CONSTRUCTION OF EXPRESSION VECTORS

The sequence was amplified from a genomic or cDNA library using primers specific to sequences upstream and downstream of the coding region. The expression vector was pMEN20 or pMEN65, which are both derived from pMON316 (Sanders et al, (1987) Nucleic Acids Research 15:1543-58) and contain the CaMV 35S promoter to express transgenes. To clone the sequence into the vector, both pMEN20 and the amplified DNA fragment were digested separately with SalI and NotI restriction enzymes at 37° C for 2 hours. The digestion products were subject to electrophoresis in a 0.8% agarose gel and visualized by ethidium bromide staining. The DNA fragments containing the sequence and the linearized plasmid were excised and purified by using a Qiaquick gel extraction kit (Qiagen, CA). The fragments of interest were ligated at a ratio of 3:1 (vector to insert). Ligation reactions using T4 DNA ligase (New England Biolabs, MA) were carried out at 16° C for 16 hours. The ligated DNAs were transformed into competent cells of the *E. coli* strain DH5alpha by using the heat shock method. The transformations were plated on LB plates containing 50 mg/l kanamycin (Sigma).

Individual colonies were grown overnight in five milliliters of LB broth containing 50 mg/l kanamycin at 37° C. Plasmid DNA was purified by using Qiaquick Mini Prep kits (Qiagen, CA).

### EXAMPLE III. TRANSFORMATION OF AGROBACTERIUM WITH THE EXPRESSION VECTOR

After the plasmid vector containing the gene was constructed, the vector was used to transform *Agrobacterium tumefaciens* cells expressing the gene products. The stock of *Agrobacterium tumefaciens* cells for transformation were made as described by Nagel et al. (1990) FEMS Microbiol Letts. 67: 325-328. *Agrobacterium* strain ABI was grown in 250 ml LB medium (Sigma) overnight at 28°C with shaking until an absorbance (A₆₀₀) of 0.5 - 1.0 was reached. Cells were harvested by centrifugation at 4,000 x g for 15 min at 4°C. Cells were then resuspended in 250 µl chilled buffer (1 mM HEPES, pH adjusted to 7.0 with KOH). Cells were centrifuged again as described above and resuspended in 125 µl chilled buffer. Cells were then centrifuged and resuspended two more times in the same HEPES buffer as described above at a volume of 100 µl and 750 µl, respectively. Resuspended cells were then distributed into 40 µl aliquots, quickly frozen in liquid nitrogen, and stored at -80° C.

*Agrobacterium* cells were transformed with plasmids prepared as described above following the protocol described by Nagel et al. For each DNA construct to be transformed, 50 - 100 ng DNA (generally resuspended in 10 mM Tris-HCl, 1 mM EDTA, pH 8.0) was mixed with 40 µl of *Agrobacterium* cells. The DNA/cell mixture was then transferred to a chilled cuvette with a 2mm electrode gap and subject to a 2.5 kV charge dissipated at 25 µF and 200 µF using a Gene Pulser II apparatus (Bio-Rad). After electroporation, cells were immediately resuspended in 1.0 ml LB and allowed to recover without antibiotic selection for 2 - 4 hours at 28° C in a shaking incubator. After recovery, cells were plated onto selective medium of LB broth containing 100 µg/ml spectinomycin (Sigma) and incubated for 24-48 hours at 28° C. Single colonies were then picked and inoculated in fresh medium. The presence of the plasmid construct was verified by PCR amplification and sequence analysis.

### EXAMPLE IV. TRANSFORMATION OF ARABIDOPSIS PLANTS WITH AGROBACTERIUM TUMEFACIENS WITH EXPRESSION VECTOR

After transformation of *Agrobacterium tumefaciens* with plasmid vectors containing the gene, single *Agrobacterium* colonies were identified, propagated, and used to transform *Arabidopsis* plants. Briefly, 500 ml cultures of LB medium containing 50 mg/l kanamycin were inoculated with the colonies and grown at 28° C with shaking for 2 days until an absorbance (A₆₀₀) of > 2.0 is reached. Cells were then harvested by centrifugation at 4,000 x g for 10 min, and resuspended in infiltration medium (1/2 X Murashige and Skoog salts (Sigma), I X Gamborg's B-5 vitamins (Sigma), 5.0% (w/v) sucrose (Sigma), 0.044 µM benzylamino purine (Sigma), 200 µl/L Silwet L-77 (Lehle Seeds) until an absorbance (A₆₀₀) of 0.8 was reached.

Prior to transformation, *Arabidopsis thaliana* seeds (ecotype Columbia) were sown at a density of ∼10 plants per 4" pot onto Pro-Mix BX potting medium (Hummert International) covered with fiberglass mesh (18 mm X 16 mm). Plants were grown under continuous illumination (50-75 µE/m²/sec) at 22-23° C with 65-70% relative humidity. After about 4 weeks, primary inflorescence stems (bolts) are cut off to encourage growth of multiple secondary bolts. After flowering of the mature secondary bolts, plants were prepared for transformation by removal of all siliques and opened flowers.

The pots were then immersed upside down in the mixture of *Agrobacterium* infiltration medium as described above for 30 sec, and placed on their sides to allow draining into a 1' x 2' flat surface covered with plastic wrap. After 24 h, the plastic wrap was removed and pots are turned upright. The immersion procedure was repeated one week later, for a total of two immersions per pot. Seeds were then collected from each transformation pot and analyzed following the protocol described below.

### EXAMPLE V. IDENTIFICATION OF ARABIDOPSIS PRIMARY TRANSFORMANTS

Seeds collected from the transformation pots were sterilized essentially as follows. Seeds were dispersed into in a solution containing 0.1% (v/v) Triton X-100 (Sigma) and sterile H₂O and washed by shaking the suspension for 20 min. The wash solution was then drained and replaced with fresh wash solution to wash the seeds for 20 min with shaking. After removal of the second wash solution, a solution containing 0.1% (v/v) Triton X-100 and 70% ethanol (Equistar) was added to the seeds and the suspension was shaken for 5 min. After removal of the ethanol/detergent solution, a solution containing 0.1% (v/v) Triton X-100 and 30% (v/v) bleach (Clorox) was added to the seeds, and the suspension was shaken for 10 min. After removal of the bleach/detergent solution, seeds were then washed five times in sterile distilled H₂O. The seeds were stored in the last wash water at 4° C for 2 days in the dark before being plated onto antibiotic selection medium (1 X Murashige and Skoog salts (pH adjusted to 5.7 with 1M KOH), 1 X Gamborg's B-5 vitamins, 0.9% phytagar (Life Technologies), and 50 mg/l kanamycin). Seeds were germinated under continuous illumination (50-75 µE/m²/sec) at 22-23° C. After 7-10 days of growth under these conditions, kanamycin resistant primary transformants (T₁ generation) were visible and obtained. These seedlings were transferred first to fresh selection plates where the seedlings continued to grow for 3-5 more days, and then to soil (Pro-Mix BX potting medium).

Primary transformants were crossed and progeny seeds (T₂) collected; kanamycin resistant seedlings were selected and analyzed. The expression levels of the recombinant polynucleotides in the transformants varies from about a 5% expression level increase to a least a 100% expression level increase. Similar observations are made with respect to polypeptide level expression.

### EXAMPLE VI. IDENTIFICATION OF ARABIDOPSIS PLANTS WITH TRANSCRIPTION FACTOR GENE KNOCKOUTS

The screening of insertion mutagenized *Arabidopsis* collections for null mutants in a known target gene was essentially as described in Krysan et al (1999) Plant Cell 11:2283-2290. Briefly, gene-specific primers, nested by 5-250 base pairs to each other, were designed from the 5' and 3' regions of a known target gene. Similarly, nested sets of primers were also created specific to each of the T-DNA or transposon ends (the "right" and "left" borders). All possible combinations of gene specific and T-DNA/transposon primers were used to detect by PCR an insertion event within or close to the target gene. The amplified DNA fragments were then sequenced which allows the precise determination of the T-DNA/transposon insertion point relative to the target gene. Insertion events within the coding or intervening sequence of the genes were deconvoluted from a pool comprising a plurality of insertion events to a single unique mutant plant for functional characterization. The method is described in more detail in Yu and Adam, US Application Serial No. 09/177,733 filed October 23, 1998.

### EXAMPLE VII. IDENTIFICATION OF ENVIRONMENTAL STRESS TOLERANCE PHENOTYPE IN OVEREXPRESSOR OR GENE KNOCKOUT PLANTS

Experiments were performed to identify those transformants or knockouts that exhibited an improved environmental stress tolerance. For such studies, the transformants were exposed to a variety of environmental stresses. Plants were exposed to chilling stress (6 hour exposure to 4-8° C ), heat stress (6 hour exposure to 32-37° C), high salt stress (6 hour exposure to 200 mM NaCl), drought stress (168 hours after removing water from trays), osmotic stress (6 hour exposure to 3 M mannitol), or nutrient limitation (nitrogen, phosphate, and potassium) (Nitrogen: all components of MS medium remained constant except N was reduced to 20mg/L of NH 4 NO3, or Phosphate: All components of MS medium except KH 2 PO 4, which was replaced by K2SO4, Potassium: All components of MS medium except removal of KNO3 and KH2PO4, which were replaced by NaH4PO4).

Table 3 shows the phenotypes observed for particular overexpressor or knockout plants and provides the SEQ ID No., the internal reference code (GID), whether a knockout or overexpressor plant was analyzed and the observed phenotype.

**Table 3**

| SEQ ID No. | GID | Knockout (KO) or overexpressor (OX) | Phenotype observed |
|---|---|---|---|
| 1 | G22 | OE | Increased tolerance to high salt |
| 3 | G188 | KO | Better germination under osmotic stress |
| 5 | G225 | OE | Increased tolerance to nitrogen-limited medium |
| 7 | G226 | OE | Increased tolerance to nitrogen-limited medium |
| 9 | G256 | OE | Better germination and growth in cold |
| 11 | G419 | OE | Increased tolerance to potassium-free medium |
| 13 | G464 | OE | Better germination and growth in heat |
| 15 | G482 | OE | Increased tolerance to high salt |
| 17 | G502 | KO | Increased sensitivity to osmotic stress |
| 19 | G526 | OE | Increased sensitivity to osmotic stress |
| 21 | G545 | OE | Susceptible to high salt |
| 23 | G561 | OE | Increased tolerance to potassium-free medium |
| 25 | G664 | OE | Better germination and growth in cold |
| 27 | G682 | OE | Better germination and growth in heat |
| 29 | G911 | OE | Increased growth on potassium-free medium |
| 31 | G964 | OE | Better germination and growth in heat |
| 33 | G394 | OE | More sensitive to chilling |
| 35 | G489 | OE | Increased tolerance to osmotic stress |

For a particular overexpressor that shows a decreased tolerance to an environmental stress, it may be more useful to select a plant with a decreased expression of the particular transcription factor. For a particular knockout that shows a decreased tolerance to an environmental stress, it may be more useful to select a plant with an increased expression of the particular transcription factor.

### EXAMPLE VIII. IDENTIFICATION OF HOMOLOGOUS SEQUENCES

Homologous sequences from *Arabidopsis* and plant species other than *Arabidopsis* were identified using database sequence search tools, such as the Basic Local Alignment Search Tool (BLAST) (Altschul et al. (1990) J. Mol. Biol. 215:403-410; and Altschul et al. (1997) Nucl. Acid Res. 25: 3389-3402). The tblastx sequence analysis programs were employed using the BLOSUM-62 scoring matrix (Henikoff, S. and Henikoff, J. G. (1992) Proc. Natl. Acad. Sci. USA 89: 10915-10919).

Identified *Arabidopsis* homologous sequences are provided in Figure 2 and included in the Sequence Listing. The percent sequence identity among these sequences is as low as 47% sequence identity. Additionally, the entire NCBI GenBank database was filtered for sequences from all plants except *Arabidopsis thaliana* by selecting all entries in the NCBI GenBank database associated with NCBI taxonomic ID 33090 (Viridiplantae; all plants) and excluding entries associated with taxonomic ID 3701 (*Arabidopsis thaliana*). These sequences were compared to sequences representing genes of SEQ IDs Nos. 1-54 on 9/26/2000 using the Washington University TBLASTX algorithm (version 2.0a19MP). For each gene of SEQ IDs Nos. 1-54, individual comparisons were ordered by probability score (P-value), where the score reflects the probability that a particular alignment occurred by chance. For example, a score of 3.6e-40 is 3.6 x 10⁻⁴⁰. For up to ten species, the gene with the lowest P-value (and therefore the most likely homolog) is listed in Figure 3.

In addition to P-values, comparisons were also scored by percentage identity. Percentage identity reflects the degree to which two segments of DNA or protein are identical over a particular length. The ranges of percent identity between the non-Arabidopsis genes shown in Figure 3 and the Arabidopsis genes in the sequence listing are: SEQ ID No. 1: 53%-67%; SEQ ID No. 3: 38%-76%; SEQ ID No. 5: 34%-67%; SEQ ID No. 7: 50%-69%; SEQ ID No. 9: 32%-91%; SEQ ID No. 11: 48%-66%; SEQ ID No. 13: 34%-60%; SEQ ID No. 15: 58%-81%; SEQ ID No. 17: 65%-94%; SEQ ID No. 19: 72%-83%; SEQ ID No. 21: 52%-64%; SEQ ID No. 23: 40%-89%; SEQ ID No. 25: 86%-97%; SEQ ID No. 27: 41%-75%; SEQ ID No. 29: 29%-72%; SEQ ID No. 31: 49%-70%; SEQ ID No. 33: 56%-86%; SEQ ID No. 35: 61%-84%; SEQ ID No. 37: 40%-58%; SEQ ID No. 39: 63%-87%; SEQ ID No. 41: 51%-88%; SEQ ID No. 43: 80%-90%; SEQ ID No. 45: 79%-90%; SEQ ID No. 47: 30%-58%; SEQ ID No. 49: 52%-62%; SEQ ID No. 51: 55%-73% and SEQ ID No. 53: 44%-80%.

The polynucleotides and polypeptides in the Sequence Listing and the identified homologous sequences may be stored in a computer system and have associated or linked with the sequences a function, such as that the polynucleotides and polypeptides are useful for modifying the environmental stress tolerance of a plant.

All references, publications, patents and other documents herein are incorporated by reference in their entirety for all purposes. Although the invention has been described with reference to the embodiments and examples above, it should be understood that various modifications can be made without departing from the spirit of the invention.

The invention is further defined by the following numbered clauses:
Clause 1: A transgenic plant with modified environmental stress tolerance, which plant comprises a recombinant polynucleotide comprising a nucleotide sequence selected from the group consisting of:
   (a) a nucleotide sequence encoding a polypeptide comprising a sequence selected from SEQ ID Nos. 2N, where N=1-27, or a complementary nucleotide sequence thereof;
   (b) a nucleotide sequence encoding a polypeptide comprising a conservatively substituted variant of a polypeptide of (a);
   (c) a nucleotide sequence comprising a sequence selected from those of SEQ ID Nos. 2N-1, where N=1-27, or a complementary nucleotide sequence thereof;
   (d) a nucleotide sequence comprising silent substitutions in a nucleotide sequence of (c);
   (e) a nucleotide sequence which hybridizes under stringent conditions to a nucleotide sequence of one or more of: (a), (b), (c), or (d);
   (f) a nucleotide sequence comprising at least 15 consecutive nucleotides of a sequence of any of (a)-(e);
   (g) a nucleotide sequence comprising a subsequence or fragment of any of (a)-(f), which subsequence or fragment encodes a polypeptide that modifies a plant's environmental stress tolerance;
   (h) a nucleotide sequence having at least 30% sequence identity to a nucleotide sequence of any of (a)-(g);
   (i) a nucleotide sequence having at least 60% identity sequence identity to a nucleotide sequence of any of (a)-(g);
   (j) a nucleotide sequence which encodes a polypeptide having at least 30% identity sequence identity to a polypeptide of SEQ ID Nos. 2N, where N=1-27;
   (k) a nucleotide sequence which encodes a polypeptide having at least 60% identity sequence identity to a polypeptide of SEQ ID Nos. 2N, where N=1-27; and
   (l) a nucleotide sequence which encodes a polypeptide having at least 65% sequence identity to a conserved domain of a polypeptide of SEQ ID Nos. 2N, where N=1-27.
Clause 2: The transgenic plant of clause 1, further comprising a constitutive, inducible, or tissue-active promoter operably linked to said nucleotide sequence.
Clause 3: The transgenic plant of clause 1, wherein the plant is selected from the group consisting of: soybean, wheat, corn, potato, cotton, rice, oilseed rape, sunflower, alfalfa, sugarcane, turf, banana, blackberry, blueberry, strawberry, raspberry, cantaloupe, carrot, cauliflower, coffee, cucumber, eggplant, grapes, honeydew, lettuce, mango, melon, onion, papaya, peas, peppers, pineapple, spinach, squash, sweet corn, tobacco, tomato, watermelon, rosaceous fruits, and vegetable brassicas.
Clause 4: An isolated or recombinant polynucleotide comprising a nucleotide sequence selected from the group consisting of:
   (a) a nucleotide sequence encoding a polypeptide comprising a sequence selected from SEQ ID Nos. 2N, where N=1-27, or a complementary nucleotide sequence thereof;
   (b) a nucleotide sequence encoding a polypeptide comprising a conservatively substituted variant of a polypeptide of (a);
   (c) a nucleotide sequence comprising a sequence selected from those of SEQ ID Nos. 2N-1, where N=1-27, or a complementary nucleotide sequence thereof;
   (d) a nucleotide sequence comprising silent substitutions in a nucleotide sequence of (c);
   (e) a nucleotide sequence which hybridizes under stringent conditions to a nucleotide sequence of one or more of: (a), (b), (c), or (d);
   (f) a nucleotide sequence comprising at least 15 consecutive nucleotides of a sequence of any of (a)-(e);
   (g) a nucleotide sequence comprising a subsequence or fragment of any of (a)-(f), which subsequence or fragment encodes a polypeptide that modifies a plant's environmental stress tolerance;
   (h) a nucleotide sequence having at least 30% sequence identity to a nucleotide sequence of any of (a)-(g);
   (i) a nucleotide sequence having at least 60% identity sequence identity to a nucleotide sequence of any of (a)-(g);
   (j) a nucleotide sequence which encodes a polypeptide having at least 30% identity sequence identity to a polypeptide of SEQ ID Nos. 2N, where N=1-27;
   (k) a nucleotide sequence which encodes a polypeptide having at least 60% identity sequence identity to a polypeptide of SEQ ID Nos. 2N, where N=1-27; and
   (l) a nucleotide sequence which encodes a conserved domain of a polypeptide having at least 65% sequence identity to a conserved domain of a polypeptide of SEQ ID Nos. 2N, where N=1-27.
Clause 5: The isolated or recombinant polynucleotide of clause 4, further comprising a constitutive, inducible, or tissue-active promoter operably linked to the nucleotide sequence.
Clause 6: A cloning or expression vector comprising the isolated or recombinant polynucleotide of clause 4.
Clause 7: A cell comprising the cloning or expression vector of clause 6.
Clause 8: A transgenic plant comprising the isolated or recombinant polynucleotide of clause 4.
Clause 9: A composition produced by one or more of:
   (a) incubating one or more polynucleotide of clause 4 with a nuclease;
   (b) incubating one or more polynucleotide of clause 4 with a restriction enzyme;
   (c) incubating one or more polynucleotide of clause 4 with a polymerase;
   (d) incubating one or more polynucleotide of clause 4 with a polymerase and a primer;
   (e) incubating one or more polynucleotide of clause 4 with a cloning vector, or
   (f) incubating one or more polynucleotide of clause 4 with a cell.
Clause 10: A composition comprising two or more different polynucleotides of clause 4.
Clause 11: An isolated or recombinant polypeptide comprising a subsequence of at least about 15 contiguous amino acids encoded by the recombinant or isolated polynucleotide of clause 4.
Clause 12: A plant ectopically expressing an isolated polypeptide of clause 11.
Clause 13: A method for producing a plant having a modifed environmental stress tolerance, the method comprising altering the expression of the isolated or recombinant polynucleotide of clause 4 or the expression levels or activity of a polypeptide of clause 11 in a plant, thereby producing a modified plant, and selecting the modified plant for improved environmental stress tolerance thereby providing the modified plant with a modified environmental stress tolerance.
Clause 14: The method of clause 13, wherein the polynucleotide is a polynucleotide of clause 4.
Clause 15: A method of identifying a factor that is modulated by or interacts with a polypeptide encoded by a polynucleotide of clause 4, the method comprising:
   (a) expressing a polypeptide encoded by the polynucleotide in a plant; and
   (b) identifying at least one factor that is modulated by or interacts with the polypeptide.
Clause 16: The method of clause 15, wherein the identifying is performed by detecting binding by the polypeptide to a promoter sequence, or detecting interactions between an additional protein and the polypeptide in a yeast two hybrid system.
Clause 17: The method of clause 15, wherein the identifying is performed by detecting expression of a factor by hybridization to a microarray, subtractive hybridization or differential display.
Clause 18: A method of identifying a molecule that modulates activity or expression of a polynucleotide or polypeptide of interest, the method comprising:
   (a) placing the molecule in contact with a plant comprising the polynucleotide or polypeptide encoded by the polynucleotide of clause 4; and,
   (b) monitoring one or more of:
      (i) expression level of the polynucleotide in the plant;
      (ii) expression level of the polypeptide in the plant;
      (iii) modulation of an activity of the polypeptide in the plant; or
      (iv) modulation of an activity of the polynucleotide in the plant.
Clause 19: An integrated system, computer or computer readable medium comprising one or more character strings corresponding to a polynucleotide of clause 4, or to a polypeptide encoded by the polynucleotide.
Clause 20: The integrated system, computer or computer readable medium of clause 19, further comprising a link between said one or more sequence strings to a modified plant environmental stress tolerance phenotype.
Clause 21: A method of identifying a sequence similar or homologous to one or more polynucleotides of clause 4, or one or more polypeptides encoded by the polynucleotides, the method comprising:
   (a) providing a sequence database; and,
   (b) querying the sequence database with one or more target sequences corresponding to the one or more polynucleotides or to the one or more polypeptides to identify one or more sequence members of the database that display sequence similarity or homology to one or more of the one or more target sequences.
Clause 22: The method of clause 21, wherein the querying comprises aligning one or more of the target sequences with one or more of the one or more sequence members in the sequence database.
Clause 23: The method of clause 21, wherein the querying comprises identifying one or more of the one or more sequence members of the database that meet a user-selected identity criteria with one or more of the target sequences.
Clause 24: The method of clause 21, further comprising linking the one or more of the polynucleotides of clause 4, or encoded polypeptides, to a modified plant environmental stress tolerance phenotype.
Clause 25: A plant comprising altered expression levels of the isolated or recombinant polynucleotide of clause 4.
Clause 26: A plant comprising altered expression levels or the activity of the isolated or recombinant polypeptide of clause 11.
Clause 27: A plant lacking a nucleotide sequence encoding a polypeptide of clause 11.

## Claims

1. A transgenic plant having greater tolerance to heat as compared to a wild-type plant of the same species, wherein said plant is transformed with a recombinant polynucleotide that encodes a polypeptide having at least 85% amino acid sequence identity to SEQ ID NO:28, or to SEQ ID NO:14, or to SEQ ID NO:32;
wherein the polypeptide regulates expression of at least one gene; and
wherein when the polypeptide is expressed in the transgenic plant, said expression results in the transgenic plant having greater tolerance to heat as compared to a wild-type plant of the same species.

2. The transgenic plant of claim 1, wherein the transgenic plant has greater tolerance to a six hour exposure to 32° C to 37° C than the wild-type plant.

3. The transgenic plant of claim 1, wherein the transgenic plant is a germinating seedling.

4. A seed produced by the transgenic plant of claim 1, wherein the seed comprises the recombinant polynucleotide encoding a polypeptide having at least 85% amino acid sequence identity to SEQ ID NO: 28.

5. The transgenic plant of claim 1, wherein the polypeptide has at least 95% amino acid sequence identity to SEQ ID NO: 28.

6. The transgenic plant of claim 1, wherein the polypeptide comprises SEQ ID NO: 28.

7. A method for producing a transgenic plant that is more heat-tolerant than a wild-type plant of the same species; the method steps comprising:
introducing a recombinant polynucleotide into a target plant to produce a transgenic plant;
wherein the recombinant polynucleotide encodes a polypeptide having at least 85% amino acid sequence identity to SEQ ID NO: 28, or to SEQ ID NO:14, or to SEQ ID NO:32;
wherein the polypeptide regulates expression of the polypeptide; and
wherein when the polypeptide is expressed in the transgenic plant, said expression results in the transgenic plant having greater tolerance to heat as compared to the wild-type plant.

8. The method of claim 7, wherein the polynucleotide comprises a constitutive, inducible, or tissue-specific promoter operably linked to the nucleotide sequence.

9. The method of claim 7, wherein the transgenic plant has greater tolerance to a six hour exposure to 32° C to 37°C than the wild-type plant.

10. The method of claim 7, wherein the transgenic plant is a germinating seedling.

11. The method of claim 7, wherein the polypeptide has at least 95% amino acid sequence identity to SEQ ID NO: 28.

12. The method of claim 7, wherein the polypeptide comprises SEQ ID NO: 28.
